# EUROPÄISCHE PATENTSCHRIFT

(11) **EP 0 612 731 B1**
(45) Veröffentlichungstag und Bekanntmachung des Hinweises auf die Patenterteilung: **20.08.1997**
(21) Anmeldenummer: 94101223.9
(22) Anmeldetag: 27.01.1994
(51) Int. Cl.: C07D 233/54, C07D 249/04, C07D 249/08, C07D 401/12, C07D 401/14, C07D 403/14, A61K 31/47

(54) **Neue Chinolon- und Naphthyridoncarbonsäurederivate**
Novel guinolone and naphthyridone derivatives
Nouveaux dérivés de quinoléinone et naphtyridone

(30) Priorität: 09.02.1993 DE 4303657
(43) Veröffentlichungstag der Anmeldung: 31.08.1994
(73) Patentinhaber: BAYER AG, 51368 Leverkusen (DE)
(72) Erfinder: Bartel, Stephan, Dr., D-51465 Bergisch Gladbach (DE); Kleefeld, Gerd, Dr., D-41468 Neuss (DE); Schulze, Thomas-J., Dr., D-51065 Köln (DE); Paessens, Arnold, Dr., D-42781 Haan (DE); Neumann, Rainer, Dr., D-50937 Köln (DE); Reefschläger, Jürgen, Dr., D-42111 Wuppertal (DE); Streissle, Gert, Dr., D-42115 Wuppertal (DE)

(56) Entgegenhaltungen:
- EP-A- 0 131 839
- EP-A- 0 422 485
- EP-A- 0 523 512
- WO-A-90/13542

## Beschreibung

Die vorliegende Erfindung betrifft neue Chinolon- und Naphthyridoncarbonsäurederivate, Verfahren zu ihrer Herstellung, sowie ihre Verwendung als Arzneimittel, insbesondere als antivirale Mittel.

Die antibakterielle Wirkung bestimmter Chinoloncarbonsäuren ist seit längerer Zeit bekannt. Über die antivirale Wirksamkeit derartiger Substanzen ist wenig bekannt [vgl. JP 022 64 724 A2, US 4 959 363; EP 431 991 A1].
Chinolone zur Behandlung von mit AIDS infizierten Zellen werden in: AIDS 4(12), 1283 (1990); Cell Struc. Funct.15(5), 295 (1990); EP 394 553 A2; WO 90 135 42 A1; beschrieben. Hierbei handelt es sich um eine Schutzwirkung für bestimmte Zellinien, ohne daß das Virus abgetötet wird. Die beschriebenen Substanzen sind auch antibakteriell wirksam.

Die vorliegende Erfindung betrifft jetzt neue Chinolon- und Naphthyridoncarbonsäurederivate der allgemeinen Formel (I) 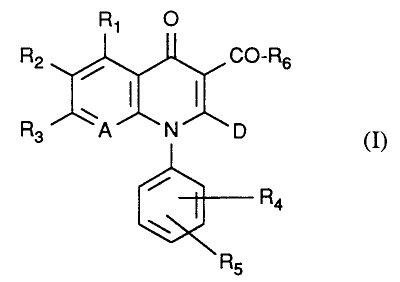 in welcher
- R¹: für Wasserstoff, Hydroxy, Mercapto, Halogen, geradkettiges oder verzweigtes Alkyl mit bis zu 3 Kohlenstoffatomen, Perfluoralkyl mit bis zu 3 Kohlenstoffatomen, geradkettiges oder verzweigtes Alkoxy oder Alkylthio mit jeweils bis zu 6 Kohlenstoffatomen, Arylthio mit 6 bis 10 Kohlenstoffatomen oder
für die Gruppe der Formel -NR⁷R⁸ steht,
worin
- R⁷ und R⁸: gleich oder verschieden sind und Wasserstoff oder geradkettiges oder verzweigtes Alkyl mit bis zu 6 Kohlenstoffatomen bedeuten,
- R²: für Wasserstoff, Nitro oder Halogen steht,
- R³: für einen Rest der Formel 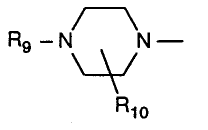 steht,
worin
- R⁹ und R¹⁰: gleich oder verschieden sind und Wasserstoff, geradkettiges oder verzweigtes Alkyl mit bis zu 6 Kohlenstoffatomen oder einen Rest der Formel 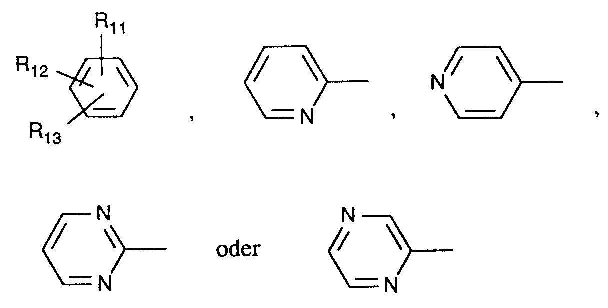 bedeuten,
worin
- R¹¹, R¹² und R¹³: gleich oder verschieden sind und Wasserstoff, Halogen, Nitro, Hydroxy, Cyano, Mercapto, Arylthio mit 6 bis 10 Kohlenstoffatomen, geradkettiges oder verzweigtes Alkyl, Alkoxy, Acyl oder Alkylthio mit jeweils bis zu 6 Kohlenstoffatomen oder Perfluoralkyl mit bis zu 4 Kohlenstoffatomen bedeuten,
- A: für ein Stickstoffatom oder für die Gruppe der Formel - CR¹⁴ steht,
worin
- R¹⁴: Wasserstoff, Halogen, Methyl, Hydroxy, Methoxy, Vinyl oder Alkinyl bedeutet,
- R⁴: für einen Rest der Formel 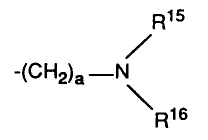 steht,
worin
- a: eine Zahl 1, 2, 3 oder 4 bedeutet,
- R¹⁵ und R¹⁶: gemeinsam mit dem Stickstoffatom einen Imidazolyl-, Pyrrolyl-, 1,2,3-Triazol-1-yl-, 1,2,3-Triazol-2-yl, 1,2,4-Triazol-1-yl- bzw. 1,2,4-Triazol-4-yl- oder Tetrazolylring bilden,
- R⁵: für Wasserstoff, Hydroxy, Halogen oder für geradkettiges oder verzweigtes Alkyl oder Alkoxy mit jeweils bis zu 6 Kohlenstoffatomen steht,
- R⁶: für Hydroxy, Benzyloxy, Morpholino oder geradketttiges oder verzweigtes Alkoxy mit bis zu 6 Kohlenstoffatomen steht, wobei letzteres bis zu 3-fach gleich oder verschieden durch Halogen, Hydroxy oder durch einen heterocyclischen Rest der Formel 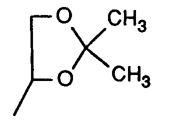 substituiert sein kann, oder
für eine Gruppe der Formel -NR¹⁷R¹⁸ steht,
worin
- R¹⁷ und R¹⁸: gleich oder verschieden sind und Wasserstoff oder geradkettiges oder verzweigtes Alkyl mit bis zu 8 Kohlenstoffatomen bedeuten, das gegebenenfalls bis zu 3-fach gleich oder verschieden durch Hydroxy oder durch geradkettiges oder verzweigtes Alkoxy mit bis zu 6 Kohlenstoffatomen substituiert ist,
oder
- R⁶: für einen Rest der Formel 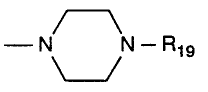 steht,
worin
- R¹⁹: Wasserstoff oder geradkettiges oder verzweigtes Alkyl mit bis zu 4 Kohlenstoffatomen bedeutet,
- D: für Wasserstoff, Amino, Phenyl, Cyano, Hydroxy oder für geradkettiges oder verzweigtes Alkenyl, Alkylthio oder Alkoxycarbonyl mit jeweils bis zu 6 Kohlenstoffatomen steht, oder
für geradkettiges oder verzweigtes Alkyl mit bis zu 6 Kohlenstoffatomen steht, das gegebenenfalls durch geradkettiges oder verzweigtes Alkoxycarbonyl mit bis zu 4 Kohlenstoffatomen substituiert ist
und deren Hydrate und Salze, gegebenenfalls in einer isomeren Form.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Alkali-, Erdalkali, Silber-und Guanidiniumsalze der erfindungsgemäßen Verbindungen sein.

Bevorzugt sind Verbindungen der allgemeinen Formel (I),
in welcher
- R¹: für Wasserstoff, Hydroxy, Mercapto, Fluor, Chlor, Brom, geradkettiges oder verzweigtes Alkyl mit bis zu 3 Kohlenstoffatomen, Trifluormethyl, geradkettiges oder verzweigtes Alkoxy oder Alkylthio mit jeweils bis zu 4 Kohlenstoffatomen, Phenylthio oder für die Gruppe der Formel - NR⁷R⁸ steht,
worin
- R⁷ und R⁸: gleich oder verschieden sind und Wasserstoff oder geradkettiges oder verzweigtes Alkyl mit bis zu 4 Kohlenstoffatomen bedeuten,
- R²: für Wasserstoff, Nitro, Fluor, Chlor oder Brom steht,
- R³: für einen Rest der Formel 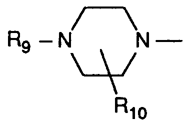 steht,
worin
- R⁹ und R¹⁰: gleich oder verschieden sind und Wasserstoff, geradkettiges oder verzweigtes Alkyl mit bis zu 4 Kohlenstoffatomen bedeuten, oder
einen Rest der Formel 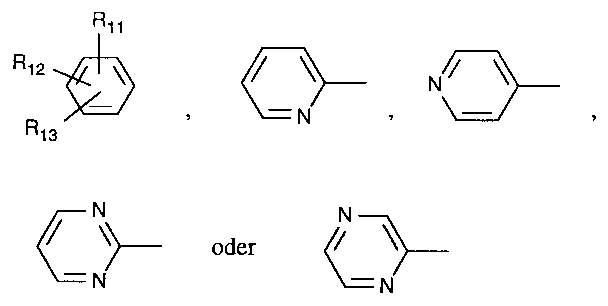 bedeuten,
worin
- R¹¹, R¹² und R¹³: gleich oder verschieden sind und Wasserstoff, Fluor, Chlor, Brom, Nitro, Hydroxy, Cyano, Phenylthio, geradkettiges oder verzweigtes Alkyl, Alkoxy, Acyl oder Alkylthio mit jeweils bis zu 4 Kohlenstoffatomen oder Trifluormethyl bedeuten,
- A: für ein Stickstoffatom oder für die Gruppe der Formel - CR¹⁴ steht,
worin
- R¹⁴: Wasserstoff, Fluor, Chlor, Brom, Methyl, Hydroxy, Methoxy, Vinyl oder Alkinyl bedeutet,
- R⁴: für einen Rest der Formel 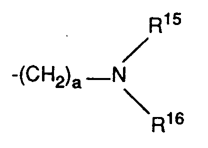 steht,
worin
- a: eine Zahl 1, 2 oder 3 bedeutet,
- R¹⁵ und R¹⁶: gemeinsam mit dem Stickstoffatom einen Imidazolyl-, Pyrrolyl-, 1,2,3-Triazol-1-yl-, 1,2,3-Triazol-2-yl, 1,2,4-Triazol-1-yl- bzw. 1,2,4-Triazol-4-yl- oder Tetrazolylring bilden,
- R⁵: für Wasserstoff, Hydroxy, Fluor, Chlor, Brom oder
für geradkettiges oder verzweigtes Alkyl oder Alkoxy mit jeweils bis zu 4 Kohlenstoffatomen steht,
- R⁶: für Hydroxy, Benzyloxy, Morpholino oder geradketttiges oder verzweigtes Alkoxy mit bis zu 5 Kohlenstoffatomen steht, wobei letzteres bis zu 2-fach gleich oder verschieden durch Halogen, Hydroxy oder durch einen heterocyclischen Rest der Formel 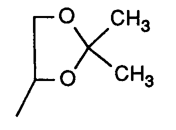 substituiert sein kann, oder
für eine Gruppe der Formel -NR¹⁷R¹⁸ steht,
worin
- R¹⁷ und R¹⁸: gleich oder verschieden sind und Wasserstoff oder geradkettiges oder verzweigtes Alkyl mit bis zu 6 Kohlenstoffatomen bedeuten, das gegebenenfalls bis zu 2-fach gleich oder verschieden durch Hydroxy oder durch geradkettiges oder verzweigtes Alkoxy mit bis zu 6 Kohlenstoffatomen substituiert ist,
oder
- R⁶: für einen Rest der Formel 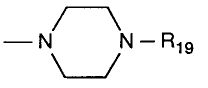 steht,
worin
- R¹⁹: Wasserstoff oder geradkettiges oder verzweigtes Alkyl mit bis zu 4 Kohlenstoffatomen bedeutet,
und
- D: für Methyl, Vinyl, Phenyl oder Wasserstoff steht,
und deren Hydrate und Salze, gegebenenfalls in einer isomeren Form.

Besonders bevorzugt sind Verbindungen der allgemeinen Formel (I),
in welcher
- R¹: für Wasserstoff, Fluor, Chlor, Brom oder Methyl steht,
- R²: für Wasserstoff, Fluor oder Chlor steht,
- R³: für einen Rest der Formel 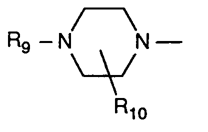 steht,
worin
- R⁹ und R¹⁰: gleich oder verschieden sind und Wasserstoff, geradkettiges oder verzweigtes Alkyl mit bis zu 4 Kohlenstoffatomen bedeuten, oder einen Rest der Formel 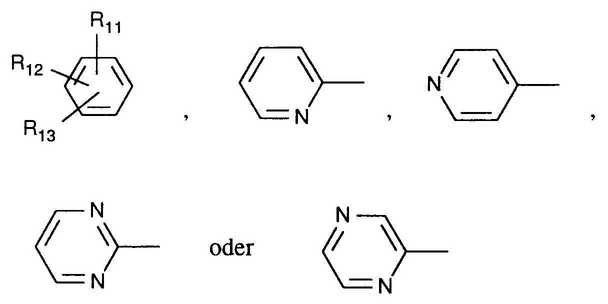 bedeuten,
worin
- R¹¹, R¹² und R¹³: gleich oder verschieden sind und Wasserstoff, Fluor, Chlor, Brom, Nitro, Hydroxy, Cyano, Phenylthio, geradkettiges oder verzweigtes Alkyl, Alkoxy, Acyl oder Alkylthio mit jeweils bis zu 3 Kohlenstoffatomen oder Trifluormethyl bedeuten,
- A: für ein Stickstoffatom oder für die Gruppe der Formel - CR¹⁴ steht,
worin
- R¹⁴: Wasserstoff, Fluor oder Chlor bedeutet,
- R⁴: für einen Rest der Formel 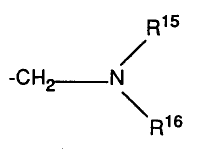 steht,
worin
- R¹⁵ und R¹⁶: gemeinsam mit dem Stickstoffatom einen Imidazolyl-, Pyrrolyl-, 1,2,3-Triazol-1-yl-, 1,2,3-Triazol-2-yl, 1,2,4-Triazol-1-yl- bzw. 1,2,4-Triazol-4-yl-Ring bilden,
- R⁵: für Wasserstoff steht,
- R⁶: für Hydroxy, Benzyloxy, Morpholino oder geradkettiges oder verzweigtes Alkoxy mit bis zu 4 Kohlenstoffatomen steht, wobei letzteres gegebenenfalls durch Fluor, Chlor, Brom, Hydroxy oder durch einen Rest der Formel 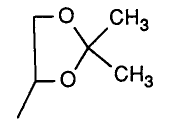 substituiert sein kann, oder
für eine Gruppe der Formel -NR¹⁷R¹⁸ steht,
worin
- R¹⁷ und R¹⁸: gleich oder verschieden sind und Wasserstoff oder geradkettiges oder verzweigtes Alkyl mit bis zu 4 Kohlenstoffatomen bedeuten, das gegebenenfalls bis zu 2-fach gleich oder verschieden durch Hydroxy oder durch geradkettiges oder verzweigtes Alkoxy mit bis zu 6 Kohlenstoffatomen substituiert ist,
oder
- R⁶: für einen Rest der Formel 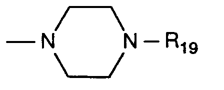 steht,
worin
- R¹⁹: Wasserstoff oder geradkettiges oder verzweigtes Alkyl mit bis zu 3 Kohlenstoffatomen bedeutet,
und
- D: für Wasserstoff steht,
und deren Hydrate und Salze, gegebenenfalls in einer isomeren Form.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II) 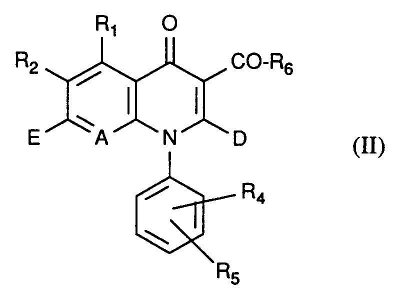 in welcher
- R¹, R², R⁴, R⁵, R⁶, A und D: die oben angegebene Bedeutung haben
und
- E: für Halogen, vorzugsweise für Chlor und Fluor steht,
mit Verbindungen der allgemeinen Formel (III)

R³-H (III)

in welcher
- R³: die oben angegebene Bedeutung hat,
in inerten Lösemitteln, gegebenenfalls in Anwesenheit von Säurefängern umsetzt,
und im Fall der Säuren die Ester verseift,
und im Fall der Ester die Säuren nach üblichen Methoden mit den entsprechenden Alkoholen umsetzt,
und im Fall der Amide die Säuren, gegebenenfalls nach vorgeschalteter Aktivierung und in Anwesenheit einer Base und/oder Hilfsstoffes, amidiert.

Das erfindungsgemäße Verfahren kann durch folgendes Formelschema beispielhaft erläutert werden:

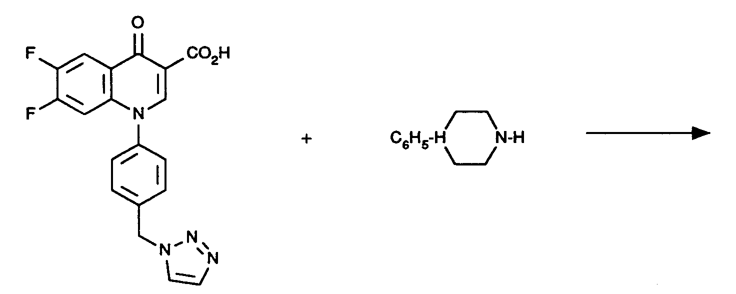

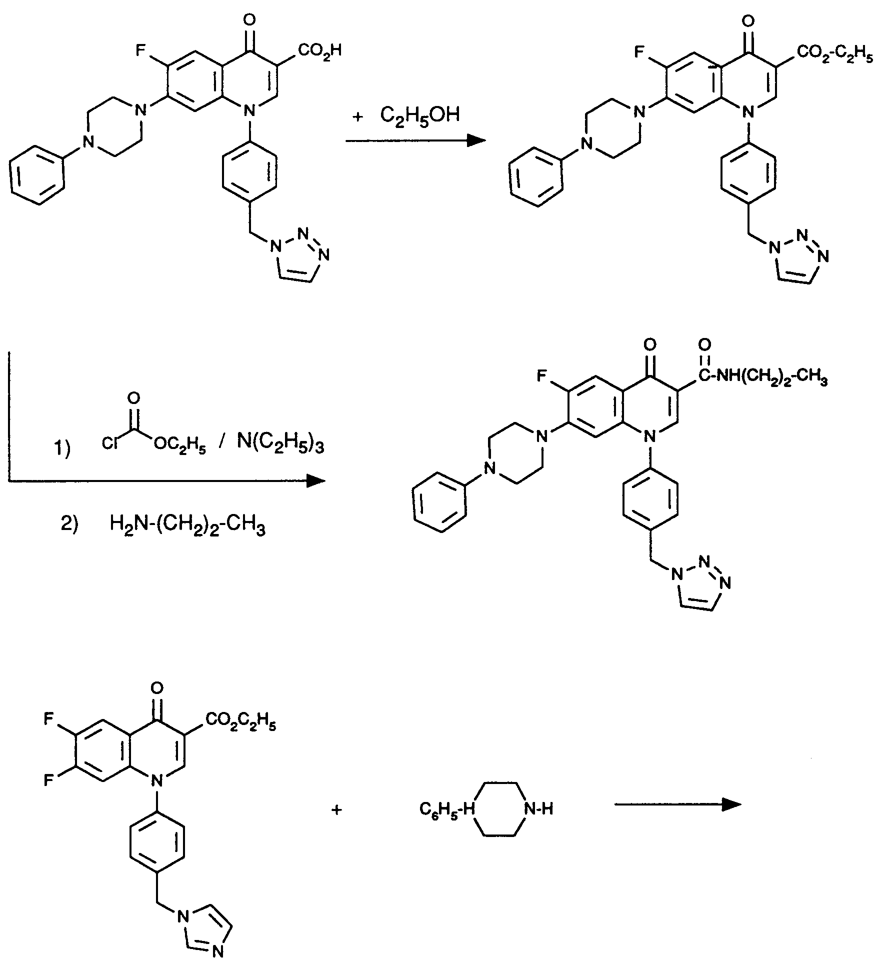

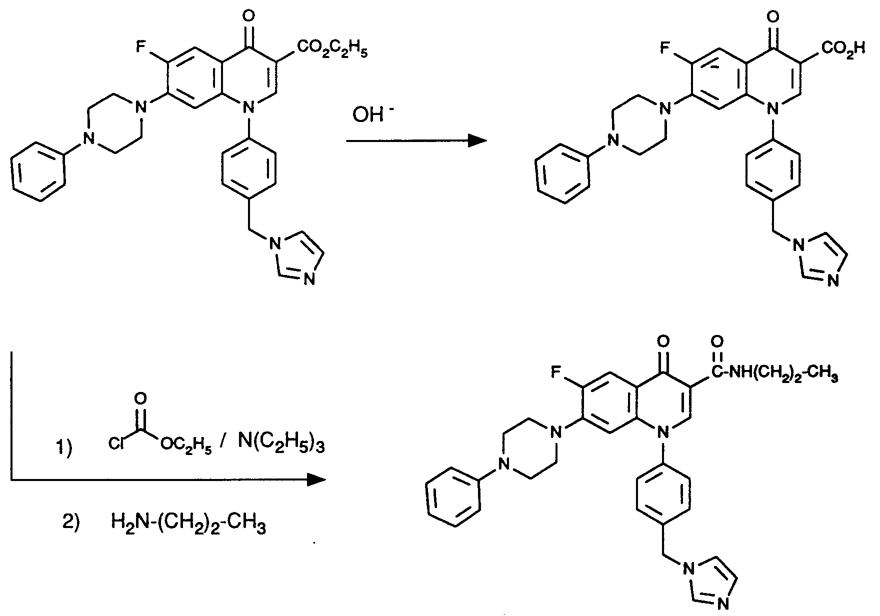

Als Lösemittel eignen sich für alle Verfahrensschritte die üblichen inerten Losemittel, die sich unter den Reaktionsbedingungen nicht verändern. Hierzu gehören bevorzugt organische Lösemittel wie Ether z.B. Diethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, N,N-Dimethylformamid, Hexamethylphosphorsäuretriamid, Sulfolan, Essigester, Pyridin, Triethylamin, N-Methylpyrrolidon, Anisol oder Picolin. Ebenso ist es möglich, Gemische der genannten Lösemittel zu verwenden. Bevorzugt sind Dimethylsulfoxid, N,N-Dimethylformamid und N-Methylpyrrolidon.

Als Basen für einzelne Reaktionsschritte eignen sich die üblichen basischen Verbindungen. Hierzu gehören beispielsweise Alkali- oder Erdalkalihydroxide, Pyridin, Triethylamin, Diisopropylethylamin oder N-Methylpiperidin, oder bicyclische Amidine wie 1,4-Diazabicyclo[2,2,2]octan (DABCO), 1,5-Diazabicyclo[4,3,0]-non-5-ene (DBN) oder 1,8-Diazabicyclo[5.4.0]undec-5-ene (DBU). Bevorzugt sind Diisopropylethylamin und DABCO.

Die Basen werden im allgemeinen in einer Menge von 1 bis 3 mol, bevorzugt von 1 bis 1,5 mol, bezogen auf 1 mol der entsprechenden Carbonsäure, eingesetzt.

Das Verfahren wird im allgemeinen in einem Temperaturbereich von +0°C bis +160°C, bevorzugt von +0°C bis +140°C, durchgeführt.

Im allgemeinen wird bei Normaldruck gearbeitet. Es ist aber auch möglich, das Verfahren bei Unterdruck oder bei Überdruck durchzuführen (z.B. in einem Bereich von 0,5 bis 5 bar).

Als Lösemittel eignen sich für die Verseifung Wasser oder Wasser in Kombination mit einem üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet.

Die Verseifung erfolgt mit Basen, beispielsweise Alkali- und Erdalkalihydroxiden, vorzugsweise Lithiumhydroxid oder Natriumhydroxid, in einem der oben aufgeführten Lösemittel, vorzugsweise Dimethylformamid, Tetrahydrofuran oder Ethanol in Kombination mit Wasser.

Die Verseifung kann auch mit Säuren wie beispielsweise Essigsäure, Chlorwasserstoffsäure, Bromwasserstoffsäure, Methansulfonsäure, Schwefelsäure oder Perchlorsäure erfolgen.

Die Verseifung wird im allgemeinen in einem Temperaturbereich von 0°C bis +130°C, bevorzugt von +20°C bis +110°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Säure im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Die Verseifung von tert.Butylestern erfolgt im allgemeinen mit Säuren, wie beispielsweise Salzsäure oder Trifluoressigsäure, in Anwesenheit eines der oben angegebenen Lösemittel und/oder Wasser oder deren Gemische, vorzugsweise mit Dioxan oder Tetrahydrofuran.

Die Veresterung der Säuren erfolgt
a) nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dann der entsprechende Alkohol auch als Lösemittel eingesetzt.
b) Alternativ wird die Säure auch durch allgemein bekannte Methoden der organischen Chemie aktiviert und zum Beispiel mit Cl-CO₂C₂H₅ in das gemischte Anhydrid überführt, das nach a) in den Ester überführt wird.

Als Katalysatoren für a) können anorganische Säuren, wie bespielsweise Schwefelsäure oder anorganische Säurechloride, wie beispielsweise Thionylchlorid, eingesetzt werden. Im Fall b) kann gegebenenfalls die Zugabe von einer der oben aufgeführten und unter den Reaktionsbedingungen inerten Basen, wie z.B. Triethylamin, Pyridin oder bicyclischen Amidinen erfolgen.

Im allgemeinen setzt man im Fall a) 0,01 bis 1, bevorzugt 0,05 bis 0,5 mol Katalysator, im Fall b) 0,5 - 2, bevorzugt 1 - 1,5 mol Katalysator, jeweils bezogen auf 1 mol Reaktionspartner, ein.

Die Amidierung erfolgt im allgemeinen in inerten Lösemitteln und gegebenenfalls in Anwesenheit einer der oben aufgeführten Basen und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Die Amidierung wird im allgemeinen in einem Temperaturbereich von 0°C bis 150°C, bevorzugt bei 25°C bis 40°C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung wird die Base im allgemeinen in einer Menge von 1 bis 3 Mol, bevorzugt von 1 bis 1,5 Mol, bezogen auf 1 Mol der Carbonsäure, eingesetzt.

Die Verbindungen der allgemeinen Formel (II) sind neu und können hergestellt werden, indem man zunächst Verbindungen der allgemeinen Formel (IV) 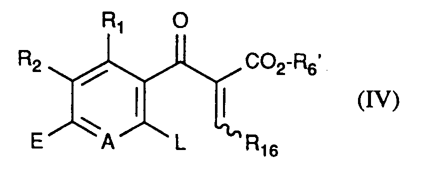 in welcher
- R¹, R², A und E: die oben angegebene Bedeutung haben,
- L: für Halogen, vorzugsweise für Chlor oder Fluor steht,
- R^{6'}: für C₁-C₄-Alkyl steht
und
- R¹⁶: für C₁-C₄-Alkoxy oder C₁-C₄-Dialkylamino steht,
durch Umsetzung mit Aminen der allgemeinen Formel (V) 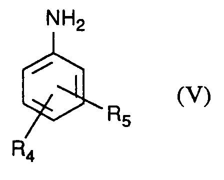 in welcher
- R⁴ und R⁵: die oben angegebene Bedeutung haben,
in einem der oben aufgeführten Lösemittel, vorzugsweise Ethanol,
in die Verbindungen der allgemeinen Formel (VI) 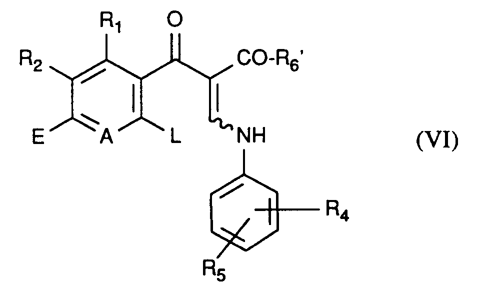 in welcher
- R¹, R², R⁴, R⁵, A, E, L und R^{6'}: die oben angegebene Bedeutung haben,
überführt, und in einem letzten Schritt in einem der oben aufgeführten Lösemittel und einer dort genannten Base, vorzugsweise DMF und K₂CO₃/KF cyclisiert,
und im Fall D ≠ H, diese Substituenten nach üblichen Methoden variiert.

Das Verfahren wird im allgemeinen in einem Temperaturbereich von +0°C bis +150°C, bevorzugt von +0°C bis +120°C, durchgeführt.

Im allgemeinen wird bei Normaldruck gearbeitet. Es ist aber auch möglich, das Verfahren bei Unterdruck oder bei Überdruck durchzuführen (z.B. in einem Bereich von 0,5 bis 5 bar).

Die Verbindungen der allgemeinen Formel (IV) und (V) sind an sich bekannt oder können nach publizierten Methoden hergestellt werden.

Die Verbindungen der allgemeinen Formel (VI) sind neu und können dann beispielsweise wie oben beschrieben hergestellt werden.

Überraschenderweise zeigten die erfindungsgemäßen Verbindungen Wirkung in Lentivirus infizierten Zellkulturen. Dies konnte am Beispiel des HIV-Virus gezeigt werden.

### HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, während die unbehandelte Zellkontrolle keine Syncytien aufwies.

Die IC₅₀-Werte wurden als die Konzentration der erfindungsgemäßen Verbindungen ermittelt, bei der 50% (ca. 10 Syncytien) der virusinduzierten Syncytien durch die Behandlung mit der erfindungsgemäßen Verbindung unterdruckt waren.

Es wurde nun gefunden, daß die erfindungsgemäßen Verbindungen HIV infizierte Zellen vor der virusinduzierten Zellzerstörung schützen.

**Tabelle A**

| Bsp.-Nr. | IC₅₀ (uM) |
|---|---|
| 8 | 3 |
| 14 | 0,7 |
| 20 | 0,2 |
| 23 | 0,4 |
| 31 | 0,7 |
| 39 | 0,25 |
| 50 | 0,5 |
| 51 | 0,2 |
| 69 | 0,7 |
| 92 | 0,6 |
| 113 | 2 |
| 146 | 0,7 |

Die erfindungsgemäßen Verbindungen stellen wertvolle Wirkstoffe zur Behandlung und Prophylaxe von Erkrankungen, hervorgerufen durch Retroviren, in der Human-und Tiermedizin dar.

Als Indikationsgebiete in der Humanmedizin können beispielsweise genannt werden:
1.) Die Behandlung und Prophylaxe von menschlichen Retrovirusinfektionen.
2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Für die Behandlung oder die Prophylaxe einer HTLV-I oder HTLV-II Infektion.
4.) Für die Behandlung oder die Prophylaxe des AIDS-carrier Zustandes (AIDS-Überträger-Zustand).

Als Indikationen in der Tiermedizin können beispielsweise angeführt werden:
Infektionen mit
a) Maedvisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegersiekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz (FIV)
h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

### Ausgangsverbindungen

### Beispiel I

### 2-(4-Aminobenzyl)-2H-1,2,3-triazol

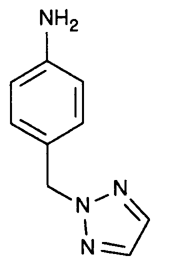

a. 2-(4-Nitrobenzyl)-2H-1,2,3-triazol
   15,6 g (0,072 mol) 4-Nitrobenzylbromid werden mit 5,0 g (0,072 mol) 1,2,3-Triazol und 20,2 g (0,15 mol) Kaliumcarbonat in 70 ml Aceton sechs Stunden zum Rückfluß erhitzt. Nach dem Abkühlen wird filtriert und das Lösungsmittel im Vakuum entfernt. Das Rohprodukt wird durch Säulenchromatographie (Kieselgel, Methylenchlorid/Methanol 98/2) gereinigt.
   Ausbeute: 3,87 g (26% der Theorie)
   Schmelzpunkt: 96-100°C
b. 2-(4-Aminobenzyl)-2-H-1,2,3-triazol
   3,5 g (0,017 mol) 2-(4-Nitrobenzyl)-2H-1,2,3-triazol werden in 50 ml Essigester und 1 g Pd/C (10%) bei Normaldruck hydriert. Der Katalysator wird abfiltriert und gut mit Dimethylformamid nachgewaschen. Die Lösungsmittel werden im Vakuum entfernt.
   Ausbeute: 2,1 g ( 72% der Theorie)
   ¹H-NMR (d₆-DMSO): s 5,4 (2H); d 6,5 (2H); d 7,0 (2H);s 7,7 (2H).

### Beispiel II und Beispiel III

### 1-(3-Aminobenzyl)-1H-1,2,3-triazol (II) und 2-(3-Aminobenzyl)-2H-1,2,3-triazol (III)

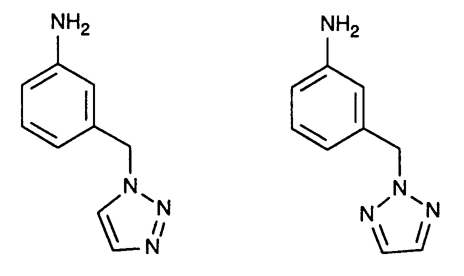

a. 1-(3-Nitrobenzyl)-1H-1,2,3-triazol und 2-(3-Nitrobenzyl)-2H-1,2,3-triazol
   12,3 g (0,072 mol) 3-Nitrobenzylchlorid und 5,0 g 1,2,3-Triazol werden mit 20,2 g (0,15 mol) Kaliumcarbonat in 70 ml Aceton sechs Stunden zum Rückfluß erhitzt. Der erkaltete Ansatz wird filtriert und das Lösungsmittel im Vakuum entfernt. Das Rohprodukt wird durch Säulenchromatographie (Kieselgel, Methylenchlorid/Methanol 96/4) getrennt.
   Ausbeute: 4,1 g 1-(3-Nitrobenzyl)-1H-1,2,3-triazol (28% der Theorie)
   Schmelzpunkt: 96-97°C
      und
      4,6 g 2-(3-Nitrobenzyl)-2H-1,2,3-triazol (31% der Theorie)
   Schmelzpunkt: 91-93°C

   4,1 g (0,02 mol) 1-(3-Nitrobenzyl)-1H-1,2,3-triazol werden in 100 ml Essigester mit 1 g Pd/C (10%) bei Normaldruck hydriert. Der Katalysator wird abfiltriert und mit Dimethylformamid gründlich nachgewaschen. Die Lösungsmittel werden im Vakuum entfernt.
   Ausbeute: 3,2 g (94% der Theorie) (Beispiel II)
   ¹H-NMR (CDCl₃): s 5,4 (2H); m 6,5-6,7 (3H); t 7,15 (1H); s 7,50 (1H); s 7,70 (1H).

   4,6 g (0,023 mol) 2-(3-Nitrobenzyl)-2H-1,2,3-triazol werden analog zu Beispiel II hydriert.
   Ausbeute: 3,9 g (97% der Theorie) (Beispiel III)

### Beispiel IV und Beispiel V

### 1-(2-Aminobenzyl)-1H-1,2,3-triazol (IV) und 2-(2-Aminobenzyl)-2H-1,2,3-triazol (V)

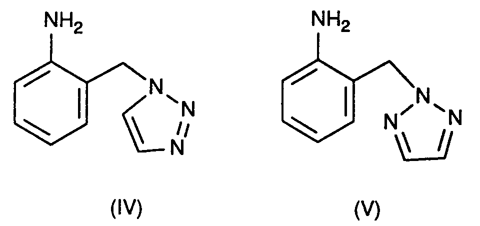

a. 1-(2-Nitrobenzyl)-1H-1,2,3-triazol und 2-(2-Nitrobenzyl)-2H-1,2,3-triazol
   15,5 g (0,072 mol) 2-Nitrobenzylbromid und 5,0 g (0,072 mol) 1,2,3-Triazol werden mit 20,2 g (0,15 mol) Kaliumcarbonat in 70 ml Aceton vier Stunden zum Rückfluß erhitzt. Der erkaltete Ansatz wird filtriert und das Lösungsmittel im Vakuum entfernt. Das Rohprodukt wird durch Säulenchromatographie (Kieselgel, Methylenchlorid/Methanol 96/4) getrennt.
   Ausbeute: 3,0 g 2-(2-Nitrobenzyl)-2H-1,2,3-triazol
      (20% der Theorie)
   Schmelzpunkt: 77-80°C
      und
      6,8 g 1-(2-Nitrobenzyl)-1H-1,2,3-triazol
      (46% der Theorie)
   Schmelzpunkt: 110-115°C
b. 1-(2-Aminobenzyl)-1H-1,2,3-triazol
   6,8 g (0,033 mol) 1-(2-Nitrobenzyl)-1H-1,2,3-triazol werden in 100 ml Essigester mit 1 g Pd/C (10%) bei Normaldruck hydriert Der Katalysator wird abfiltriert und mit Dimethylformamid gründlich nachgewaschen. Die Lösungsmittel werden im Vakuum entfernt.
   Ausbeute: 5,6 g (97% der Theorie)
   ¹H-NMR (CDCl₃): s 5,45 (2H); m 6,65-6,85 (2H); m 7,10-7,25 (2H); s 7,50 (1H); s 7,70 (1H).

   3,0 g (0,015 mol) 2-(2-Nitrobenzyl)-2H-1,2,3-triazol werden analog zu Beispiel b. hydriert.
   Ausbeute: 2,4 g (91% der Theorie)
   ¹H-NMR (CDCl₃): s 5,50 (2H); m 6,60-6,85 (2H); m 7,05-7,35 (2H); s 7,55 (2H)

### Beispiel VI

### 6,7-Difluor-1,4-dihydro-4-oxo-1-[4-(1H-1,2,3-triazol-1-yl-methyl)-phenyl]-3-chinolincarbonsäure

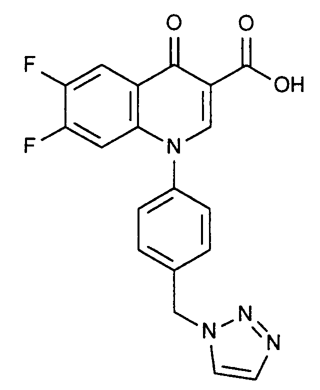

a. 2-(2,4,5-Trifluorbenzoyl)-3-[4-(1H-1,2,3-triazol-1-yl-methyl)-phenylamino]-acrylsäureethylester
   11,5 g (0,038 mol) 3-Ethoxy-2-(2,4,5-trifluorbenzoyl)-acrylsäureethylester werden in 75 ml Ethanol vorgelegt und 6,7 g (0,038 mol) 1-(4-Aminobenzyl)-1H-1,2,3-triazol zugetropft. Man rührt 12 Stunden bei Raumtemperatur nach und entfernt das Lösungsmittel im Vakuum.
   Rohausbeute: 15,8 g
b. 6,7-Difluor-1,4-dihydro-4-oxo-1-[4-(1H-1,2,3-triazol-1-yl-methyl)-phenyl]-3-chinolincarbonsäureethylester
   15 g (0,035 mol) des unter a. erhaltenen Produktes werden mit 5,7 g (0,041 mol) Kaliumcarbonat in 70 ml Dimethylformamid fünf Stunden auf 100°C erwärmt. Das Lösungsmittel wird im Vakuum entfernt und der Rückstand mit Wasser verrührt. Es wird bei ca. 100°C getrocknet.
   Ausbeute: 13,2 g (92% der Theorie)
   Schmelzpunkt: 183-185°C

   10 g (24 mmol) des unter b. erhaltenen Esters werden mit 0,6 g (24 mmol) Lithiumhydroxid in einer Mischung aus 100 ml Tetrahydrofuran, 5 ml Dimethylformamid und 5 ml Wasser drei Tage bei Raumtemperatur gerührt. Anschließend wird abgesaugt, der Rückstand gründlich mit iso-Propanol verrührt und bei ca. 100°C getrocknet.
   Ausbeute: 8,2 g (89% der Theorie)
   Schmelzpunkt: >280°C

### Beispiel VII

### 7-Chlor-6-fluor-1,4-dihydro-4-oxo-1-[4-(1H-1,2,3-triazol-1-yl-methyl)-phenyl]-1,8-naphthyridin-3-carbonsäureethylester

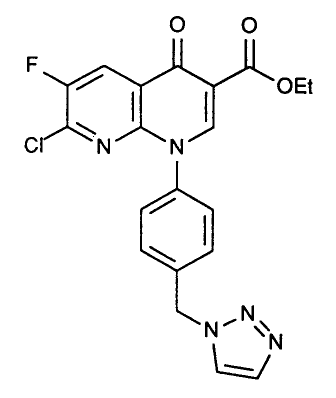

a. 2-(2,5-Dichlor-4-fluor-nicotinoyl)-3-[4-(1H-1,2,3-triazol-1-yl-methyl)-phenylamino]-acrylsäureethylester
   2,9 g (8,6 mmol) 3-Ethoxy-2-(2,5-dichlor-4-fluor-nicotinoyl)-acrylsäureethylester und 1,5 g (8,6 mmol) 1-(4-Aminobenzyl)-1H-1,2,3-triazol werden in 20 ml Ethanol 12 Stunden bei Raumtemperatur gerührt. Das Lösungsmittel wird im Vakuum entfernt.
   Ausbeute: 3,65 g (91% der Theorie)
   Schmelzpunkt: 153-158°C

   3,5 g (7,5 mmol) des unter a. erhaltenen Produktes werden mit 1,3 g (9,4 mmol) Kaliumcarbonat in 20 ml Dimethylformamid fünf Stunden auf 120°C erwärmt. Der abgekühlte Ansatz wird auf Eiswasser gegeben und das ausgefallene Produkt isoliert. Es wird bei ca 100°C getrocknet. Ausbeute: 3,05 g (94% der Theorie) Schmelzpunkt: 216-220°C

### Beispiel VIII

### 6,7-Difluor-1,4-dihydro-4-oxo-1-[4-(2H-1,2,3-triazol-2-yl-methyl)-phenyl]-3-chinolincarbonsäureethylester

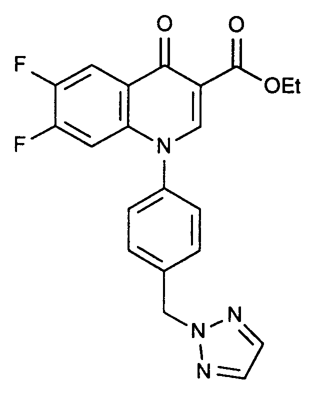

a. 2-(2,4,5-Trifluorbenzoyl)-3-[4-(2H-1,2,3-triazol-2-yl-methyl)-phenylamino]-acrylsäureethylester
   3,6 g (0,012 mol) 3-Ethoxy-2-(2,4,5-trifluorbenzoyl)-acrylsäureethylester und 2,1 g (0,038 mol) 2-(4-Aminobenzyl)-2H-1,2,3-triazol werden in 25 ml Ethanol über Nacht bei Raumtemperatur gerührt. Alle flüchtigen Komponenten werden im Vakuum entfernt.
   Ausbeute: 5,0 g (97% der Theorie)
   Schmelzpunkt: 108-110°C

   5,0 g (0,012 mol) des unter a. erhaltenen Produktes werden mit 1,9 g (0,014 mol) Kaliumcarbonat in 25 ml Dimethylformamid fünf Stunden auf 100°C erwärmt. Der abgekühlte Ansatz wird auf Eiswasser gegeben und das ausgefallene Produkt isoliert. Es wird bei ca 100°C getrocknet.
   Ausbeute: 4,0 g (85% der Theorie)
   Schmelzpunkt: 203-206°C

### Beispiel IX

### 6,7-Difluor-1,4-dihydro-4-oxo-1-[4-(1H-1,2,4-triazol-1-yl-methyl)-phenyl]-3-chinolincarbonsäureethylester

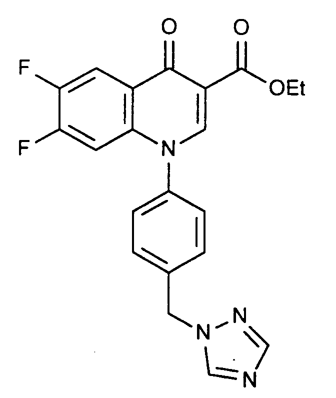

a. 2-(2,4,5-Trifluorbenzoyl)-3-[4-(1H-1,2,4-triazol-1-yl-methyl)-phenylamino]-acrylsäureethylester
   17,2 g (0,052 mol) 3-Ethoxy-2-(2,4,5-trifluorbenzoyl)-acrylsäureethylester und 10,0 g (0,057 mol) 1-(4-Aminobenzyl)-1H-1,2,4-triazol werden in 100 ml Ethanol über Nacht bei Raumtemperatur gerührt. Das ausgefallene Produkt wird isoliert und mit Ethanol gewaschen.
   Ausbeute: 20,2 g (82% der Theorie)
   Schmelzpunkt: 137-139°C

   4,5 g (0,011 mol) des unter a. erhaltenen Produktes werden mit 1,9 g (0,014 mol) Kaliumcarbonat in 25 ml Dimethylformamid sieben Stunden auf 100°C erwärmt. Der abgekühlte Ansatz wird auf Eiswasser gegeben und das ausgefallene Produkt isoliert. Es wird bei ca 100°C getrocknet.
   Ausbeute: 2,94 g (68% der Theorie)
   Schmelzpunkt: 226-228°C

### Beispiel X

### 7-Chlor-6-fluor-1,4-dihydro-4-oxo-1-[4-(1H-1,2,4-triazol-1-yl-methyl)-phenyl]-1,8-naphthyridin-3-carbonsäureethylester

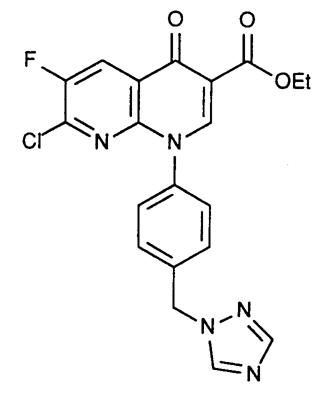

a. 2-(2,5-Dichlor-4-fluor-nicotinoyl)-3-[4-(1H-1,2,4-triazol-1-yl-methyl)-phenylamino]-acrylsäureethylester
   9,7 g (0,029 mol) 3-Ethoxy-2-(2,5-dichlor-4-fluor-nicotinoyl)-acrylsäureethylester und 5,0 g (0,029 mol) 1-(4-Aminobenzyl)-1H-1,2,4-triazol werden in 60 ml Ethanol drei Stunden bei Raumtemperatur gerührt. Das Lösungsmittel wird im Vakuum entfernt.
   Ausbeute: 13,5 g Rohprodukt

   13,3 g (28 mmol) des unter a. erhaltenen Produktes werden mit 5,0 g (36 mmol) Kaliumcarbonat in 70 ml Dimethylformamid vier Stunden auf 100°C erwärmt. Der abgekühlte Ansatz wird auf Eiswasser gegeben und das ausgefallene Produkt isoliert. Es wird bei ca 100°C getrocknet.
   Ausbeute: 10,8 g (85% der Theorie)
   Schmelzpunkt: 225-228°C

### Beispiel XI

### 6,7-Difluor-1,4-dihydro-1-[4-(N-imidazolyl-methyl)-phenyl]-4-oxo-3-chinolincarbonsäure

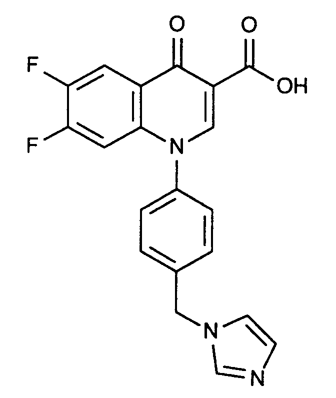

a. 2-(2,4,5-Trifiuorbenzoyl)-3-[4-(N-imidazolyl-methyl)-phenylamino]-acrylsäureethylester
   17,2 g (0,057 mol) 3-Ethoxy-2-(2,4,5-trifluorbenzoyl)-acrylsäureethylester und 10,0 g (0,057 mol) N-(4-Aminobenzyl)-imidazol werden in 100 ml Ethanol über Nacht bei Raumtemperatur gerührt. Alle flüchtigen Komponenten werden im Vakuum entfernt.
   Ausbeute: 24,0 g Rohprodukt
b. 6,7-Difluor-1,4-dihydro-1-[4-(N-imidazolyl-methyl)-phenyl]-4-oxo-3-chinolincarbonsäureethylester
   24,0 g des unter a. erhaltenen Produktes werden mit 14,3 g (0,1 mol) Kaliumcarbonat in 200 ml Dimethylformamid sechs Stunden auf 100°C erwärmt. Alle flüchtigen Komponenten werden im Vakuum entfernt und der Rückstand durch Säulenchromatographie (Kieselgel, Methylenchlorid/Methanol 96/4) gereinigt
   Ausbeute: 15,3 g (65% der Theorie über zwei Stufen)
   Schmelzpunkt: 251-254°C

   200 mg (0,49 mmol) des unter b. erhaltenen Esters werden in einer Mischung aus 1 ml Essigsäure, 1 ml Wasser und 0,1 ml konzentrierter Schwefelsäure zwei Stunden auf 80°C erwärmt. Nach dem Abkühlen wird auf wenig Eiswasser gegeben und das Produkt isoliert.
   Ausbeute: 180 mg (96% der Theorie)
   Schmelzpunkt: >280°C

### Beispiel XII

### 6,7-Difluor-1,4-dihydro-4-oxo-1-[3-(1H-1,2,3-triazol-1-yl-methyl)-phenyl]-3-chinolincarbonsäureethylester

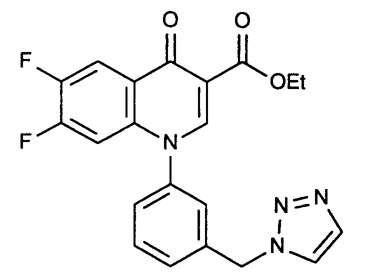

a. 2-(2,4,5-Trifluorbenzoyl)-3-[3-(1H-1,2,3-triazol-1-yl-methyl)-phenylamino]-acrylsäureethylester
   5,9 g (0,019 mol) 3-Ethoxy-2-(2,4,5-trifluorbenzoyl)-acrylsäureethylester und 3,4 g (0,019 mol) 1-(3-Aminobenzyl)-1H-1,2,3-triazol werden in 40 ml Ethanol über Nacht gerührt. Alle flüchtigen Komponenten werden im Vakuum entfernt.
   Ausbeute: 7,8 g (92% der Theorie)
   Schmelzpunkt: 126-128°C

   7,7 g (0,018 mol) des unter a. erhaltenen Produktes werden mit 3,1 g (0,022 mol) Kaliumcarbonat in 45 ml Dimethylformamid vier Stunden auf 100°C erwärmt. Der abgekühlte Ansatz wird auf 180 ml Eiswasser gegeben und das ausgefallene Produkt isoliert. Es wird bei ca 100°C getrocknet.
   Ausbeute: 6,2 g (85% der Theorie)
   Schmelzpunkt: 187-189°C

### Beispiel XIII

### 6,7-Difluor-1,4-dihydro-1-[3-(2H-1,2,3-triazol-2-yl-methyl)-phenyl]-4-oxo-3-chinolincarbonsäureethylester

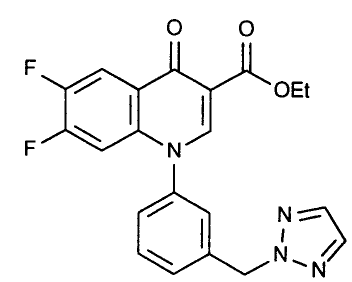

a. 2-(2,4,5-Trifluorbenzoyl)-3-[3-(2H-1,2,3-triazol-2-yl-methyl)-phenylamino]-acrylsäureethylester
   4,0 g (0,013 mol) 3-Ethoxy-2-(2,4,5-trifluorbenzoyl)-acrylsäureethylester und 2,3 g (0,013 mol) 2-(3-Aminobenzyl)-2H-1,2,3-triazol werden in 25 ml Ethanol über Nacht gerührt. Alle flüchtigen Komponenten werden im Vakuum entfernt.
   Ausbeute: 5,5 g Rohausbeute

   5,5 g (0,013 mol) des unter a. erhaltenen Produktes werden mit 2,3 g (0,017 mol) Kaliumcarbonat in 30 ml Dimethylformamid vier Stunden auf 100°C erwärmt. Der abgekühlte Ansatz wird auf 180 ml Eiswasser gegeben und das ausgefallene Produkt isoliert. Es wird bei ca 100°C getrocknet.
   Ausbeute: 4,5 g (86% der Theorie)
   Schmelzpunkt: 144-146°C

### Beispiel XIV

### 6,7-Difluor-1,4-dihydro-1-[3-(1H-1,2,4-triazol-1-yl-methyl)-phenyl]-4-oxo-3-chinolincarbonsäureethylester

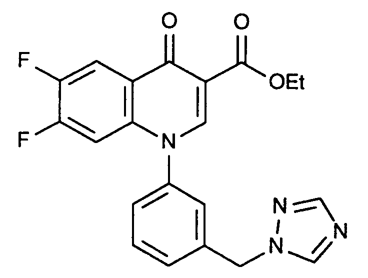

a. 2-(2,4,5-Trifluorbenzoyl)-3-[3-(1H-1,2,4-triazol-1-yl-methyl)-phenylamino]-acrylsäureethylester
   8,7 g (0,029 mol) 3-Ethoxy-2-(2,4,5-trifluorbenzoyl)-acrylsäureethylester und 5,0 g (0,029 mol) 1-(3-Aminobenzyl)-1H-1,2,4-triazol werden in 60 ml Ethanol über Nacht gerührt. Alle flüchtigen Komponenten werden im Vakuum entfernt.
   Ausbeute: 12,4 g Rohausbeute

   12,4 g (0,013 mol) des unter a. erhaltenen Produktes werden mit 5,4 g (0,039 mol) Kaliumcarbonat in 80 ml Dimethylformamid zwei Stunden auf 100°C erwärmt. Der abgekühlte Ansatz wird auf Eiswasser gegeben, mit Methylenchlorid extrahiert und das Lösungsmittel einrotiert. Es wird bei ca. 100°C getrocknet.
   Ausbeute: 10,0 g (84% der Theorie)
   Schmelzpunkt: 175-180°C

### Beispiel XV

### 7-Chlor-6-fluor-1,4-dihydro-4-oxo-1-[3-(1H-1,2,4-triazol-1-yl-methyl)-phenyl]-1,8-naphthyridin-3-carbonsäureethylester

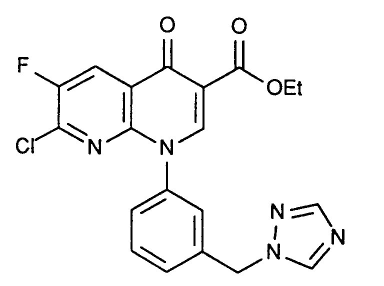

a. 2-(2,5-Dichlor-4-fluor-nicotinoyl)-3-[3-(1H-1,2,4-triazol-1-yl-methyl)-phenylamino]-acrylsäureethylester
   5,7 g (0,017 mol) 3-Ethoxy-2-(2,5-dichlor-4-fluor-nicotinoyl)-acrylsäureethylester und 3,0 g (0,017 mol) 1-(3-Aminobenzyl)-1H-1,2,4-triazol werden in 35 ml Ethanol über Nacht bei Raumtemperatur gerührt. Das Lösungsmittel wird im Vakuum entfernt.
   Ausbeute: 7,7 g Rohprodukt

   7,7 g (16 mmol) des unter a. erhaltenen Produktes werden mit 3,4 g (24 mmol) Kaliumcarbonat in 50 ml Dimethylformamid zwei Stunden auf 100°C erwärmt. Der abgekühlte Ansatz wird auf Eiswasser gegeben, es wird mit Methylenchlorid extrahiert, die organische Phase wird über Natriumsulfat getrocknet und einrotiert. Es wird bei ca 100°C getrocknet.
   Ausbeute: 4,9 g (67% der Theorie über zwei Stufen)
   Schmelzpunkt: 186-189°C

### Beispiel XVI

### 6,7-Difluor-1,4-dihydro-1-[3-(N-imidazolyl-methyl)-phenyl]-4-oxo-3-chinolincarbonsäure

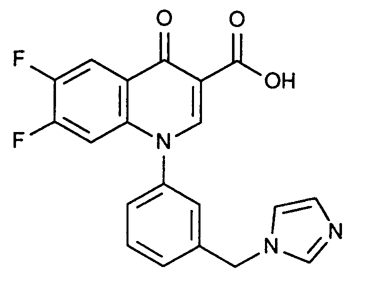

a. 2-(2,4,5-Trifluorbenzoyl)-3-[3-(N-imidazolyl-methyl)-phenylamino]-acrylsäureethylester
   8,7 g (0,029 mol) 3-Ethoxy-2-(2,4,5-trifluorbenzoyl)-acrylsäureethylester und 5,0 g (0,029 mol) N-(3-Aminobenzyl)-imidazol werden in 60 ml Ethanol über Nacht bei Raumtemperatur gerührt. Alle flüchtigen Komponenten werden im Vakuum entfernt.
   Ausbeute: 12,3 g Rohprodukt
b. 6,7-Difluor-1,4-dihydro-1-[3-(N-imidazolyl-methyl)-phenyl]-4-oxo-3-chinolincarbonsäureethylester
   12,3 g des unter a. erhaltenen Produktes werden mit 5 g (0,036 mol) Kaliumcarbonat in 70 ml Dimethylformamid zwei Stunden auf 100°C erwärmt. Der abgekühlte Ansatz wird auf Eiswasser gegeben, mit Methylenchlorid extrahiert und einrotiert. Das Rohprodukt wird durch Säulenchromatographie (Kieselgel, Methylenchlorid/Methanol 96/4) gereinigt.
   Ausbeute: 3,3 g (28% der Theorie über zwei Stufen)
   Schmelzpunkt: 193-194°C

   200 mg (0,49 mmol) des unter b. erhaltenen Esters werden in einer Mischung aus 1 ml Essigsäure, 1 ml Wasser und 0,1 ml konzentrierter Schwefelsäure zwei Stunden auf 80°C erwärmt. Nach dem Abkühlen wird auf wenig Eiswasser gegeben und das Produkt isoliert.
   Ausbeute: 130 mg (68% der Theorie)
   Schmelzpunkt: >280°C

### Beispiel XVII

### 6,7-Difluor-1,4-dihydro-4-oxo-1-[2-(1H-1,2,3-triazol-1-yl-methyl)-phenyl]-3-chinolincarbonsäureethylester

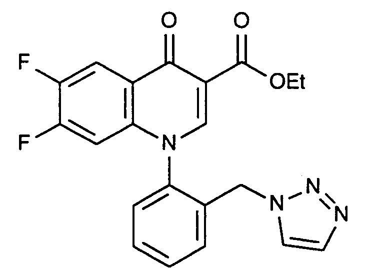

a. 2-(2,4,5-Trifluorbenzoyl)-3-[2-(1H-1,2,3-triazol-1-yl-methyl)-phenylamino]-acrylsäureethylester
   5,0 g (0,016 mol) 3-Ethoxy-2-(2,4,5-trifluorbenzoyl)-acrylsäureethylester und 2,8 g (0,016 mol) 1-(2-Aminobenzyl)-1H-1,2,3-triazol werden in 30 ml Ethanol über Nacht gerührt. Alle flüchtigen Komponenten werden im Vakuum entfernt.
   Ausbeute: 6,4 g Rohprodukt
   Schmelzpunkt: 124-126°C

   6,0 g (0,014 mol) des unter a. erhaltenen Produktes werden mit 2,4 g (0,017 mol) Kaliumcarbonat in 35 ml Dimethylformamid vier Stunden auf 100°C erwärmt. Der abgekühlte Ansatz wird auf 180 ml Eiswasser gegeben und das ausgefallene Produkt isoliert. Es wird bei ca 100°C getrocknet.
   Ausbeute: 3,9 g (68% der Theorie)
   Schmelzpunkt: 224-226°C

### Beispiel XVIII

### 6,7-Difluor-1,4-dihydro-4-oxo-1-[2-(2H-1,2,3-triazol-2-yl-methyl)-phenyl]-3-chinolincarbonsäureethylester

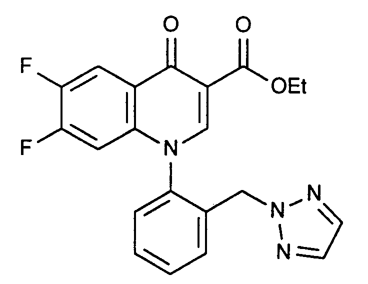

a. 2-(2,4,5-Trifluorbenzoyl)-3-[2-(2H-1,2,3-triazol-2-yl-methyl)-phenylamino]-acrylsäureethylester
   3,9 g (0,013 mol) 3-Ethoxy-2-(2,4,5-trifluorbenzoyl)-acrylsäureethylester und 2,3 g (0,013 mol) 2-(3-Aminobenzyl)-2H-1,2,3-triazol werden in 25 ml Ethanol über Nacht gerührt. Alle flüchtigen Komponenten werden im Vakuum entfernt. Ausbeute: 4,9 g (89% der Theorie)
   Schmelzpunkt: 98-99°C

   4,5 g (0,011 mol) des unter a. erhaltenen Produktes werden mit 1,7 g (0,012 mol) Kaliumcarbonat in 25 ml Dimethylformamid vier Stunden auf 100°C erwärmt. Der abgekühlte Ansatz wird auf 180 ml Eiswasser gegeben und das ausgefallene Produkt isoliert. Es wird bei ca 100°C getrocknet.
   Ausbeute: 3,7 g (88% der Theorie)
   Schmelzpunkt: 144-145°C

### Beispiel XIX

### 6,7-Difluor-1,4-dihydro-4-oxo-1-[2-(1H-1,2,4-triazol-1-yl-methyl)-phenyl]-3-chinolincarbonsäureethylester

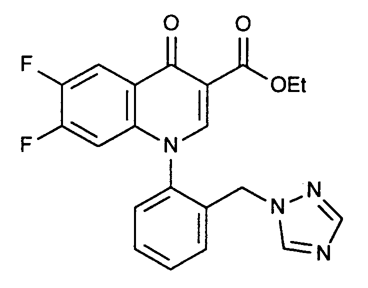

a. 2-(2,4,5-Trifluorbenzoyl)-3-[2-(1H-1,2,4-triazol-1-yl-methyl)-phenylamino]-acrylsäureethylester
   8,7 g (0,029 mol) 3-Ethoxy-2-(2,4,5-trifluorbenzoyl)-acrylsäureethylester und 5,0 g (0,029 mol) 1-(3-Aminobenzyl)-1H-1,2,4-triazol werden in 60 ml Ethanol über Nacht gerührt. Alle flüchtigen Komponenten werden im Vakuum entfernt.
   Ausbeute: 14,3 g Rohausbeute

   12,4 g des unter a. erhaltenen Produktes werden mit 5,7 g (0,04 mol) Kaliumcarbonat in 80 ml Dimethylformamid 3,5 Stunden auf 100°C erwärmt. Der abgekühlte Ansatz wird auf Eiswasser gegeben, mit Methylenchlorid extrahiert und das Lösungsmittel einrotiert. Das Rohprodukt wird durch Säulenchromatographie (Kieselgel, Methylenchlorid/Methanol 96/4) gereinigt.
   Ausbeute: 6,5 g (54% der Theorie)
   Schmelzpunkt: 214-216°C

### Beispiel XX

### 6,7-Difluor-1,4-dihydro-1-[2-(N-imidazolyl-methyl)-phenyl]-4-oxo-3-chinolincarbonsäure

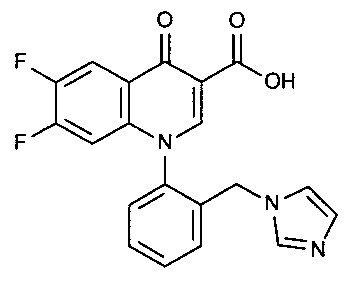

a. 2-(2,4,5-Trifluorbenzoyl)-3-[2-(N-imidazolyl-methyl)-phenylamino]-acrylsäureethylester
   7,85 g (0,026 mol) 3-Ethoxy-2-(2,4,5-trifluorbenzoyl)-acrylsäureethylester und 4,5 g (0,026 mol) N-(2-Aminobenzyl)-imidazol werden in 50 ml Ethanol über Nacht bei Raumtemperatur gerührt. Alle flüchtigen Kmponenten werden im Vakuum entfernt und der Rückstand durch Chromatographie gereinigt.
   Ausbeute: 7,87 g (70% der Theorie)
b. 6,7-Difluor-1,4-dihydro-1-[2-(N-imidazolyl-methyl)-phenyl]-4-oxo-3-chinolincarbonsäureethylester
   7,8 g (0,018 mol) des unter a. erhaltenen Produktes werden mit 3,1 g (0,022 mol) Kaliumcarbonat in 45 ml Dimethylformamid 3,5 Stunden auf 100°C erwärmt. Der abgekühlte Ansatz wird auf Eiswasser gegeben, mit Methylenchlorid extrahiert und einrotiert. Das Rohprodukt wird durch Säulenchromatographie (Kieselgel, Methylenchlorid/Methanol 96/4) gereinigt.
   Ausbeute: 4,7 g (64% der Theorie)
   Schmelzpunkt: 225-227°C

   200 mg (0,49 mmol) des unter b. erhaltenen Esters werden in einer Mischung aus 1 ml Essigsäure, 1 ml Wasser und 0,1 ml konzentrierter Schwefelsäure zwei Stunden auf 80°C erwärmt. Nach dem Abkühlen wird mit verdünnter Natronlauge neutralisiert, mit Methylenchlorid extrahiert und einrotiert.
   Ausbeute: 67 mg (37% der Theorie)
   Schmelzpunkt: 210-214°C (unter Zersetzung)

### Beispiel XXI

### 6,7,8-Trifluor-1,4-dihydro-4-oxo-1-[4-(1H-1,2,3-triazol-1-yl-methyl)-phenyl]-3-chinolincarbonsäure

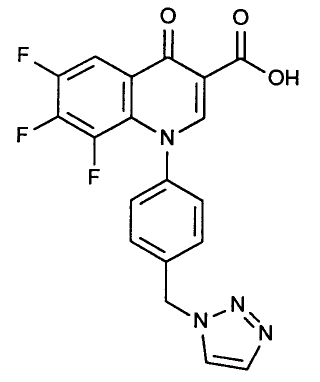

a. 2-(2,3,4,5-Tetrafluorbenzoyl)-3-[4-(1H-1,2,3-triazol-1-yl-methyl)-phenylamino]-acrylsäureethylester
   12 g (0,069 mol) 1-(4-Aminobenzyl)-1H-1,2,3-triazol werden in 120 ml Ethanol vorgelegt und mit 22,0 g (0,069 mol) 3-Ethoxy-2-(2,3,4,5-tetrafluorbenzoyl)-acrylsäureethylester in 30 ml Methylenchlorid bei Raumtemperatur versetzt. Man rührt 12 Stunden bei Raumtemperatur nach und entfernt das
   Lösungsmittel im Vakuum.
   Rohausbeute: 30,8 g
b. 6,7,8-Trifluor-1,4-dihydro-4-oxo-1-[4-(1H-1,2,3-triazol-1-yl-methyl)-phenyl]-3-chinolincarbonsäureethylester
   30,8 g (0,069 mol) des unter a. erhaltenen Produktes werden mit 4,4 g (0,076 mol) Kaliumfluorid in 130 ml Dimethylformamid drei Stunden auf 90°C erwärmt. Das Lösungsmittel wird im Vakuum entfernt, der Rückstand in Methylenchlorid/Wasser aufgenommen. Nach Rückextraktion werden die vereinigten organischen Phasen mit Wasser neutral gewaschen und über Natriumsulfat getrocknet. Nach dem Entfernen des Lösungsmittels im Vakuum wird der Rückstand mit Essigester/Isooctan 1:1 verrührt.
   Ausbeute: 23,2 g (79% der Theorie)
   Schmelzpunkt: 170-172°C

   23,2 g (54,2 mmol) des unter b. erhaltenen Esters werden mit 1,3 g (54,2 mmol) Lithiumhydroxid in einer Mischung aus 240 ml Tetrahydrofuran, 100 ml Dimethylformamid und 12 ml Wasser über Nacht bei Raumtemperatur gerührt. Nach Entfernen der Lösungsmittel im Vakuum wird das Rohprodukt durch Säulenchromatographie (Kieselgel, Methylenchlorid/Methanol 95/5) gereinigt.
   Ausbeute: 10,7 g (89% der Theorie)
   Schmelzpunkt: 170-172°C

### Beispiel XXII

### 8-Chlor-6,7-difluor-1,4-dihydro-4-oxo-1-[4-(1H-1,2,3-triazol-1-yl-methyl)-phenyl]-3-chinolincarbonsäure

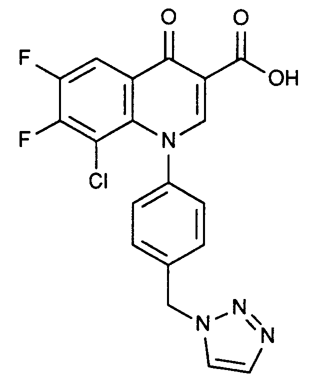

a. 2-(3-Chlor-2,4,5-trifluorbenzoyl)-3-[4-(1H-1,2,3-triazol-1-yl-methyl)-phenylamino]-acrylsäureethylester
   8 g (0,046 mol) 1-(4-Aminobenzyl)-1H-1,2,3-triazol werden in 80 ml Ethanol vorgelegt und mit 14,7 g (0,046 mol) 3-Ethoxy-2-(3-chlor-2,4,5-trifluorbenzoyl)-acrylsäureethylester in 4 ml Methylenchlorid bei Raumtemperatur versetzt. Man rührt 12 Stunden bei Raumtemperatur nach und entfernt das Losungsmittel im Vakuum.
   Rohausbeute: 21,3 g
b. 8-Chlor-6,7-difluor-1,4-dihydro-4-oxo-1-[4-(1H-1,2,3-triazol-1-yl-methyl)-phenyl]-3-chinolincarbonsäureethylester
   21,3 g (0,069 mol) des unter a. erhaltenen Produktes werden mit 4,9 g (0,050 mol) Kaliumfluorid in 90 ml Dimethylformamid drei Stunden auf 90°C erwärmt. Das Lösungsmittel wird im Vakuum entfernt, der Rückstand in Methylenchlorid/Wasser aufgenommen. Nach Rückextraktion werden die vereinigten organischen Phasen mit Wasser neutralgewaschen und über Natriumsulfat getrocknet. Das Lösungsmittel wird im Vakuum entfernt.
   Ausbeute: 12,2 g (60% der Theorie)
   Schmelzpunkt: 120-122°C

   12,0 g (27 mmol) des unter b. erhaltenen Esters werden mit 700 mg (27 mmol) Lithiumhydroxid in einer Mischung aus 120 ml Tetrahydrofuran, 50 ml Dimethylformamid und 6 ml Wasser drei Tage bei Raumtemperatur gerührt. Nach Entfernen der Lösungsmittel im Vakuum wird das Rohprodukt durch Säulenchromatographie (Kieselgel, Methylenchlorid/Methanol/Essigsäure 10/1/0,5) gereinigt.
   Ausbeute: 9,4 g (84% der Theorie)
   Schmelzpunkt: 201-203°C

### Beispiel XXIII

### 8-Chlor-6,7-difluor-1,4-dihydro-4-oxo-1-[4-(2H-1,2,3-triazol-2-yl-methyl)-phenyl]-3-chinolincarbonsäure

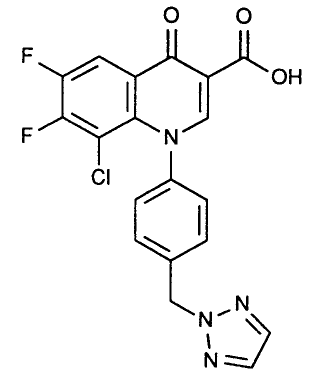

Analog zum Beispiel XXII wurde die Titelverbindung erhalten.
Schmelzpunkt: >250°C

### Beispiel XXIV

### 6,7,8-Trifluor-1,4-dihydro-4-oxo-1-[4-(1H-1,2,4-triazol-1-yl-methyl)-phenyl]-3-chinolincarbonsäure

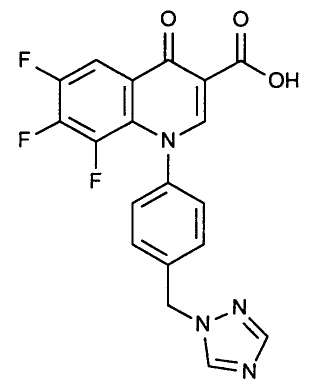

a. 2-(2,3,4,5-Tetrafluorbenzoyl)-3-[4-(1H-1,2,4-triazol-1-yl-methyl)-phenylamino]-acrylsäureethylester
   3,9 g (0,022 mol) 1-(4-Aminobenzyl)-1H-1,2,4-triazol werden in 40 ml Ethanol vorgelegt und mit 7,2 g (0,022 mol) 3-Ethoxy-2-(2,3,4,5-tetrafluorbenzoyl)-acrylsäureethylester in 2 ml Methylenchlorid bei Raumtemperatur versetzt. Man rührt 12 Stunden bei Raumtemperatur nach und entfernt das Lösungsmittel im Vakuum.
   Rohausbeute: 10,0g
b. 6,7,8-Trifluor-1,4-dihydro-4-oxo-1-[4-(1H-1,2,4-triazol-1-yl-methyl)-phenyl]-3-chinolincarbonsäureethylester
   10,0 g (0,022 mol) des unter a. erhaltenen Produktes werden mit 1,4 g (0,025 mol) Kaliumfluorid in 50 ml Dimethylformamid drei Stunden auf 90°C erwärmt. Das Lösungsmittel wird im Vakuum entfernt, der Rückstand in Methylenchlorid/Wasser aufgenommen. Nach Rückextraktion werden die vereinigten organischen Phasen mit Wasser neutralgewaschen und über Natriumsulfat getrocknet. Das Rohprodukt wird durch Säulenchromatographie (Kieselgel, Methylenchlorid/Methanol 95:5) gereinigt.
   Ausbeute: 8,9 g (94% der Theorie)
   Schmelzpunkt: 160-162°C

   8,9 g (20,8 mmol) des unter b. erhaltenen Esters werden mit 500 mg (20,8 mmol) Lithiumhydroxid in einer Mischung aus 90 ml Tetrahydrofuran, 4,5 ml Dimethylformamid und 4,5 ml Wasser über Nacht bei Raumtemperatur gerührt. Nach Entfernen der Lösungsmittel im Vakuum wird das Rohprodukt durch Säulenchromatographie (Kieselgel, Methylenchlorid/Methanol 95/5) gereinigt.
   Ausbeute: 6,2 g (75% der Theorie)
   Schmelzpunkt: 256-258°C

### Beispiel XXV

### 8-Chlor-6,7-difluor-1,4-dihydro-4-oxo-1-[4-(1H-1,2,4-triazol-1-yl-methyl)-phenyl]-3-chinolincarbonsäure

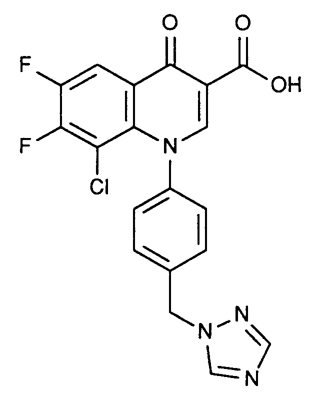

Analog zum Beispiel XXIV wurde die Titelverbindung erhalten.
Schmelzpunkt: 232-234°C

### Beispiel XXVI

### N-Isopropyl-[6,7-difluor-1,4-dihydro-4-oxo-1-[4-(1H-1,2,3-triazol-1-yl-methyl)-phenyl]-3-chinolincarbonsäureamid]

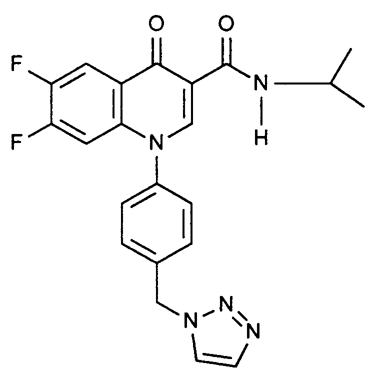

2,67 g (7,0 mmol) 6,7-Difluor-1,4-dihydro-4-oxo-1-[4-(1H-1,2,3-triazol-1-yl - methyl)-phenyl]-3-chinolincarbonsäure werden in 25 ml trockenen Toluol suspendiert und zur Trocknung am Wasserabscheider gekocht. Anschließend gibt man zur abgekühlten Suspension 0,55 ml (7,5 mmol) Thionylchlorid, kocht bis zum Ende der Gasentwicklung, gibt dann bei Raumtemperatur 1,3 ml (15 mmol) Isopropylamin zu und kocht zwei weitere Stunden. Zur Aufarbeitung rührt man die abgekühlte Lösung in 1N Salzsäure und extrahiert mit Methylenchlorid. Die vereinigten organischen Phasen werden mit gesättigter Natriumhydrogencarbonatlösung und gesättigter Natriumchloridlösung gewaschen. Die über Natriumsulfat getrocknete Lösung wird einrotiert und durch Säulenchromatographie (Kieselgel, Methylenchlorid/Methanol 9/1) gereinigt.
Ausbeute: 0,7 g (24% der Theorie)
Schmelzpunkt: 223-225°C

### Beispiel XXVII

### N,N-Diisopropyl-[6,7-difluor-1,4-dihydro-4-oxo-1-[4-(1H-1,2,3-triazol-1-yl-methyl)-phenyl]-3-chinolincarbonsäureamid]

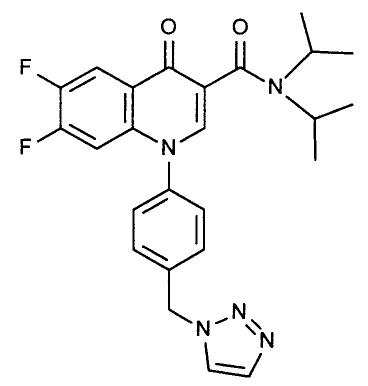

0,95 g (2,5 mmol) 6,7-Difluor-1,4-dihydro-4-oxo-1-[4-(1H-1,2,3-triazol-1-yl-methyl)-phenyl]-3-chinolincarbonsäure werden in 10 ml Aceton bei 0°C mit 0,28 ml (2,7 mmol) Triethylamin versetzt. Anschließend tropft man bei dieser Temperatur innerhalb von 30 Minuten 0,38 ml (2,7 mmol) Chlorameisensäureethylester zu. Nach Zugabe von 0,38 ml (2,7 mmol) Diisopropylamin wird eine Stunde unter Rückfluß gekocht. Zur Aufarbeitung gibt man auf gesättigte Natriumhydrogencarbonatlösung und extrahiert mit Essigester. Die vereinigten organischen Phasen werden mit gesättigter Natriumchloridlösung gewaschen und über Natriumsulfat getrocknet.
Ausbeute: 1,1 g (95% der Theorie)
Schmelzpunkt: 85-87°C

Analog zum Beispiel XXVII werden die in Tabelle I aufgeführten Verbindungen erhalten:

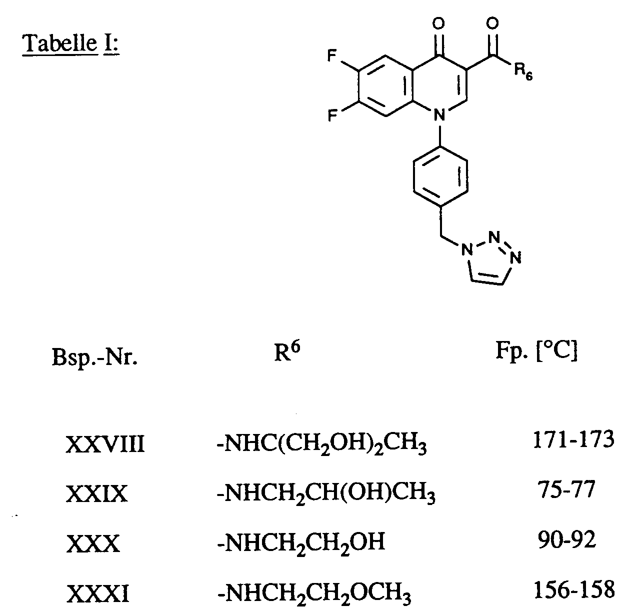

### Beispiel XXXII

### 7-Chlor-1,4-dihydro-4-oxo-1-[4-(1H-1,2,4-triazol-1-yl-methyl)phenyl]-1,8-naphthyridin-3-carbonsäureethylester

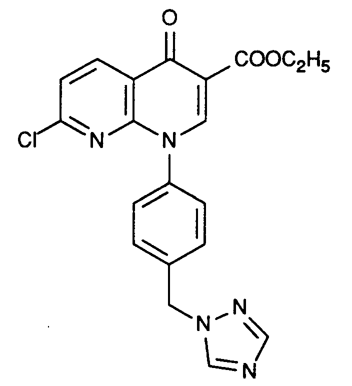

a. (2,6-Dichlornicotinoyl)malonsäurediethylester
   Man legt 7,21 g (0,075 mol) Magnesiumchlorid bei 0°C in 75 ml absolutem Acetonitril vor und tropft unter Eisbadkühlung 12,12 g (0,075 mol) Malonsäurediethylester zu. Anschließend werden 15,34 g (0,150 mol) Triethylamin bei 0°C zugetropft, nach 60 minütigem Nachrühren werden bei 0°C 17,0 g (0,075 mol) 2,6-Dichlornicotinsäurechlorid (Helvetica Chimica Acta 59, 222 (1976)) zugetropft und unter Erwärmung auf Raumtemperatur über Nacht nachgerührt. Es wird mit 80 ml 18%iger Salzsäure versetzt und mit Methyl-tert.burylether extrahiert, über Natriumsulfat getrocknet und im Vakuum eingeengt.
b. (2,6-Dichlornicotinoyl)essigsäureethylester
   Der rohe (2,6-Dichlornicotinoyl)-malonsäurediethylester wird in 45 ml Wasser mit 90 mg p-Toluolsulfonsäure 2 Stunden zum Rückfluß erhitzt. Es wird mit Methylenchlorid extrahiert, mit Wasser gewaschen, über Natriumsulfat getrocknet und im Vakuum eingeengt. Das Rohprodukt wird an Kieselgel (Laufmittel Dichlormethan) gereinigt.
   Ausbeute: 14,2 g (72% d.Th. über zwei Schritte).
c. 2-(2,6-Dichlornicotinoyl)-3-ethoxyacrylsäureethylester
   43 g (0,162 mol) des Produktes von b. werden in 38,1 g (0,26 mol) Orthoameisensäureethylester und 42,4 g (0,42 mol) Essigsäureanhydrid zwei Stunden auf 150-160°C erwärmt. Alle leicht flüchtigen Bestandteile werden im Hochvakuum bei einer Badtemperatur bis 100°C abdestilliert und das Rohprodukt direkt weiter umgesetzt.
   Rohausbeute: 50,5 g
d. 2-(2,5-Dichlornicotinoyl)-3-[4-(1H-1,2,4-triazol-1-yl-methyl)phenylamino]-acrylsäureethylester
   7 g (0,022 mol) des unter c. erhaltenen Produktes und 3,8 g (0,022 mol) 1-(4-Aminobenzyl)-1H-1,2,4-triazol werden in 40 ml Ethanol über Nacht bei Raumtemperatur gerührt. Das Lösemittel wird im Vakuum entfernt.
   Ausbeute: 9,7 g Rohprodukt

   26,0 g (0,058 mol) des bei d. erhaltenen Produktes werden mit 9,5 g (0,066 mol) Kaliumcarbonat in 140 ml Dimethylformamid vier Stunden auf 80°C erwärmt. Der abgekühlte Ansatz wird auf Eiswasser gegeben, das ausgefallene Produkt isoliert und bei ca. 100°C getrocknet.
   Ausbeute: 18,7 g (78% d.Th.)
   Schmp.: 253-256°C

### Herstellungsbeispiele:

### Beispiel 1:

### 6-Fluor-7-[4-(4-fluorphenyl)-piperazin-1-yl]-1,4-dihydro-4-oxo-1-[4-(1H-1,2,3-triazol-1-yl-methyl)-phenyl]-3-chinolincarbonsäure-ethylester

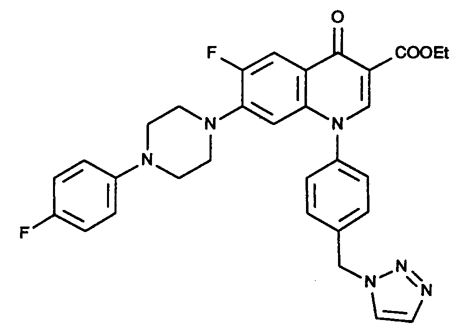

490 mg (1,2 mmol) 6,7-Difluor-1,4-dihydro-4-oxo-1-[4-(1H-1,2,3-triazol-1-yl-methyl)-phenyl]-3-chinolincarbonsäure-ethylester werden mit 650 mg (3,6 mmol) N-(4-Fluorphenyl)-piperazin in 12 ml N-Methylpyrrolidon acht Stunden auf 140°C erwärmt. Alle flüchtigen Komponenten werden im Hochvakuum entfernt, der Rückstand mit iso-Propanol verrührt und bei ca 100°C getrocknet.
Ausbeute: 580 mg (84% der Theorie)
Schmelzpunkt: 128-130°C

### Beispiel 2:

### 6-Fluor-1,4-dihydro-4-oxo-7-(3-phenylpiperazin-1-yl)-1-[4-(1H-1,2,3-triazol-1-yl-methyl)-phenyl]-3-chinolincarbonsäure-ethylester

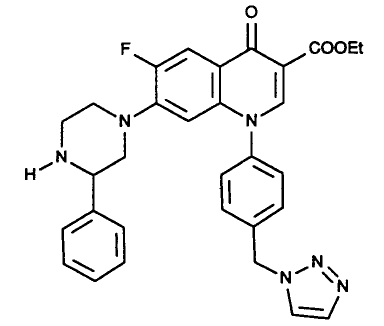

Analog zum Beispiel 1 erhält man bei der Umsetzung mit 3-Phenylpiperazin nach Verrühren in Acetonitril die Titelverbindung.
Schmelzpunkt: 219-221°C

### Beispiel 3:

### 6-Fluor-1,4-dihydro-4-oxo-7-(4-phenylpiperazin-1-yl)-1-[4-(1H-1,2,3-triazol-1-yl-methyl)-phenyl]-3-chinolincarbonsäure

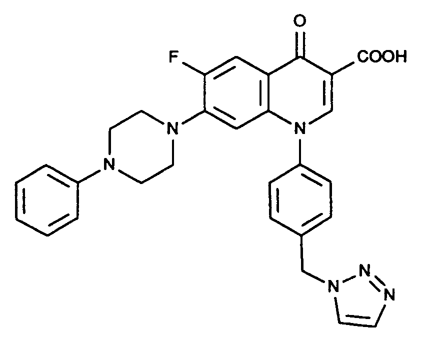

286 mg (0,75 mmol) 6,7-Difluor-1,4-dihydro-4-oxo-1-[4-(1H-1,2,3-triazol-1-yl-methyl)-phenyl]-3-chinolincarbonsäure und 364 mg (2,25 mmol) N-Phenylpiperazin werden in 7,5 ml N-Methylpyrrolidon eine Stunde auf 120°C erwärmt. Alle flüchtigen Bestandteile werden im Hochvakuum entfernt, der Rückstand mit Acetonitril verrührt und bei ca.100°C getrocknet.
Ausbeute: 150 mg (38% der Theorie)
Schmelzpunkt: >290°C

In Analogie zur Vorschrift des Beispiels 3 werden die in Tabelle 1 aufgeführten Verbindungen hergestellt:

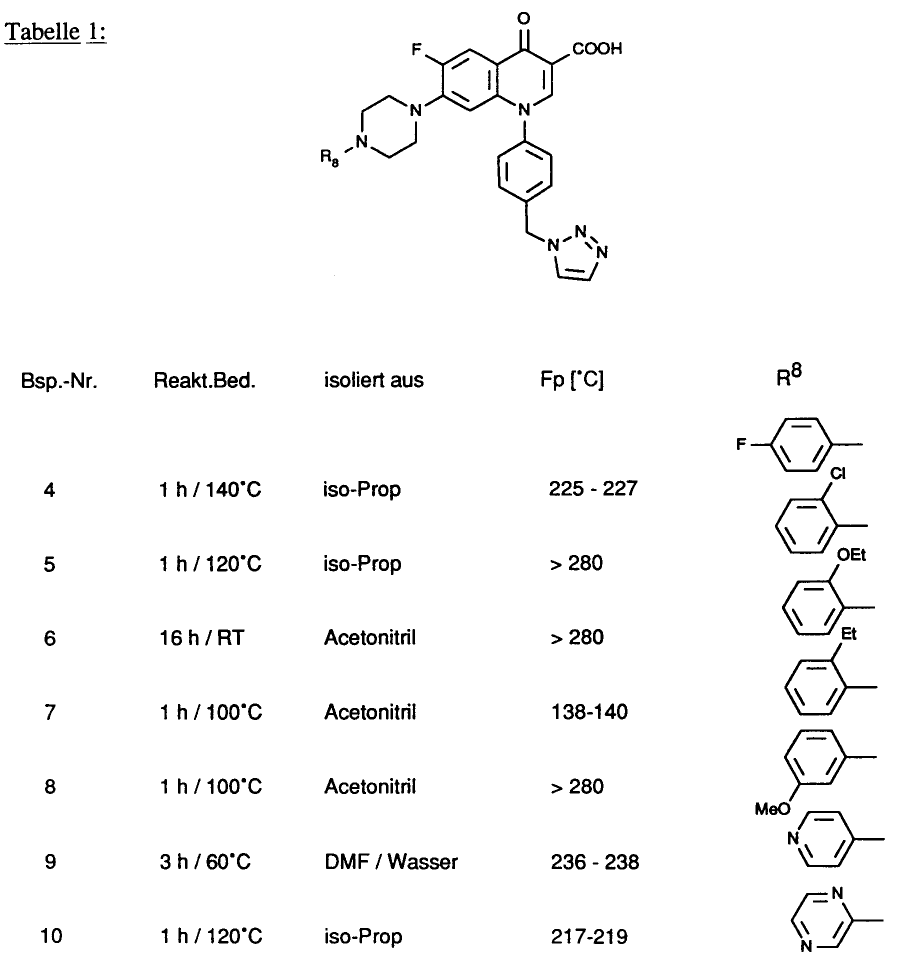

### Beispiel 11

### 6-Fluor-1,4-dihydro-4-oxo-7-(3-phenylpiperazin-1-yl)-1-[4-(1H-1,2,3-triazol-1-yl-methyl)-phenyl]-3-chinolincarbonsäure

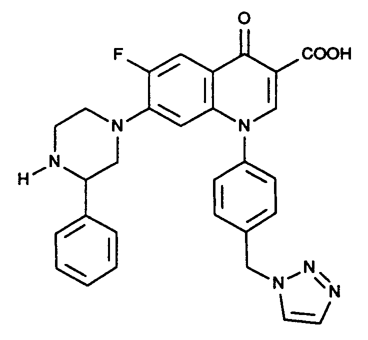

286 mg (0,75 mmol) 6,7-Difluor-1,4-dihydro-4-oxo-1-[4-(1H-1,2,3-triazol-1-yl-methyl)-phenyl]-3-chinolincarbonsäure und 365 mg (2,25 mmol) 3-Phenylpiperazin werden in 7,5 ml N-Methylpyrrolidon eine Stunde auf 120°C erwärmt. Alle flüchtigen Komponenten werden im Vakuum entfernt, der Rückstand mit iso-Propanol verrührt und bei ca. 100°C getrocknet.
Ausbeute: 210 mg (53% der Theorie)
Schmelzpunkt: 163-165°C

### Beispiel 12

### 6-Fluor-1,4-dihydro-4-oxo-7-[4-(4-hydroxyphenyl)-piperazin-1-yl]-1-[4-(1H-1,2,3-triazol-1-yl-methyl)-phenyl]-3-chinolincarbonsäure

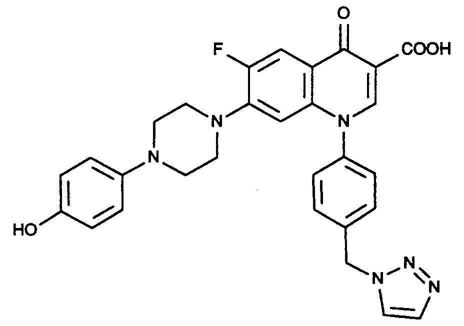

286 mg (0,75 mmol) 6,7-Difluor-1,4-dihydro-4-oxo-1-[4-(1H-1,2,3-triazol-1-yl-methyl)- phenyl]-3-chinolincarbonsäure werden mit 290 mg (0,86 mmol) N-(4-Hydroxyphenyl)- piperazin und 385 mg (3,4 mmol) Diazabicyclo[2.2.2]octan in 7,5 ml N-Methylpyrrolidon zwei Stunden auf 120°C erwärmt. Alle flüchtigen Komponenten werden im Hochvakuum entfernt, der Rückstand mit Wasser verrührt und bei ca. 100°C getrocknet.
Ausbeute: 390 mg (96% der Theorie)
Schmelzpunkt: 269-271°C

In Analogie zur Vorschrift des Beispiels 12 werden die in Tabelle 2 aufgeführten Verbindungen hergestellt:

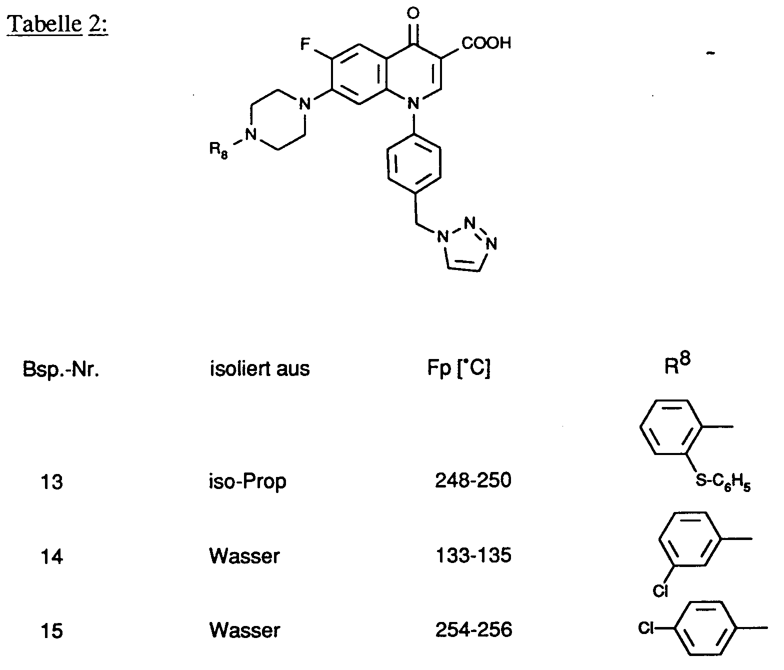

### Beispiel 16

### 6-Fluor-1,4-dihydro-4-oxo-7-(4-phenylpiperazin-1-yl)-1-[4-(1H-1,2,3-triazol-1-yl-methyl)-phenyl]-1,8-naphthyridin-3-carbonsäureethylester

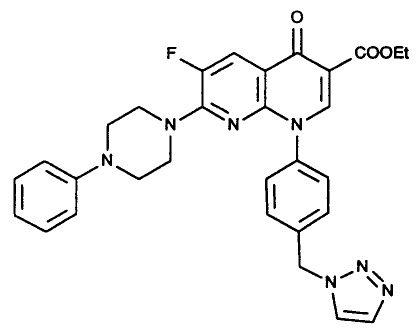

0,5 g (1,2 mmol) 7-Chlor-6-fluor-1,4-dihydro-4-oxo-1-[4-(1H-1,2,3-triazol-1-yl-methyl)- phenyl]-1,8-naphthyridin-3-carbonsäureethylester werden mit 190 mg (1,2 mmol) N-Phenylpiperazin und 460 mg (4,1 mmol) Diazabicyclo[2.2.2]octan in 12 ml Dimethylsulfoxid vier Stunden auf 120°C erwärmt. Alle flüchtigen Komponenten werden im Hochvakuum entfernt, der Rückstand mit Wasser verrührt und bei ca 100°C getrocknet.
Ausbeute: 430 mg (66 % der Theorie)
Schmelzpunkt: 201-203°C

In Analogie zur Vorschrift des Beispiels 16 werden die in Tabelle 3 aufgeführten Verbindungen hergestellt:

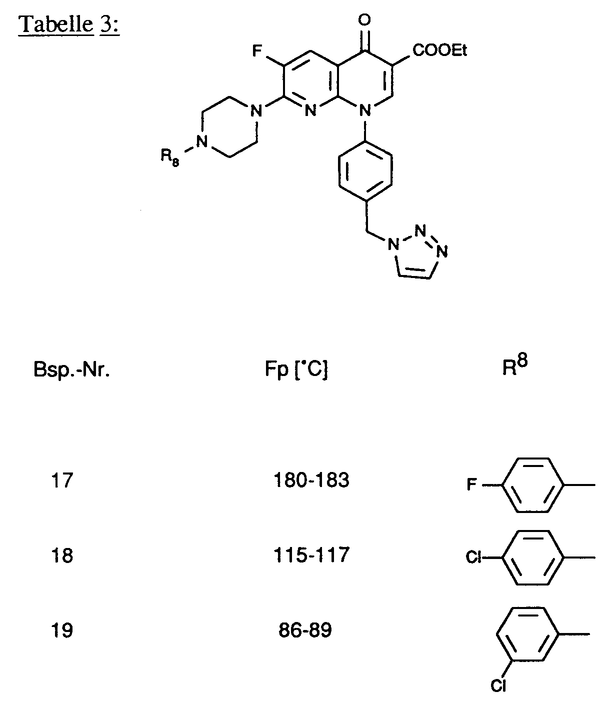

### Beispiel 20

### 6-Fluor-1,4-dihydro-4-oxo-7-(4-phenylpiperazin-1-yl)-1-[4-(1H-1,2,3-triazol-1-yl-methyl)- phenyl]-1,8-naphthyridin-3-carbonsäure

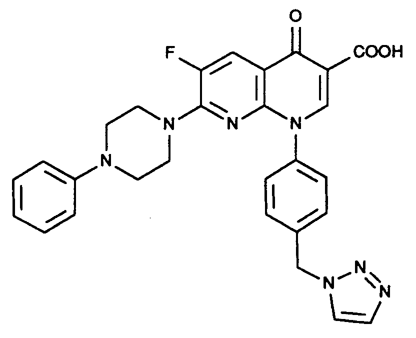

300 mg (0,5 mmol) des Esters aus Beispiel 16 werden mit 1 ml 1 N Natronlauge in einem Gemisch aus 5 ml Ethanol und 5 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 240 mg (85% der Theorie)
Schmelzpunkt: 279-281°C (unter Zersetzung)

In Analogie zur Vorschrift des Beispiels 20 werden die in Tabelle 4 aufgeführten Verbindungen hergestellt:

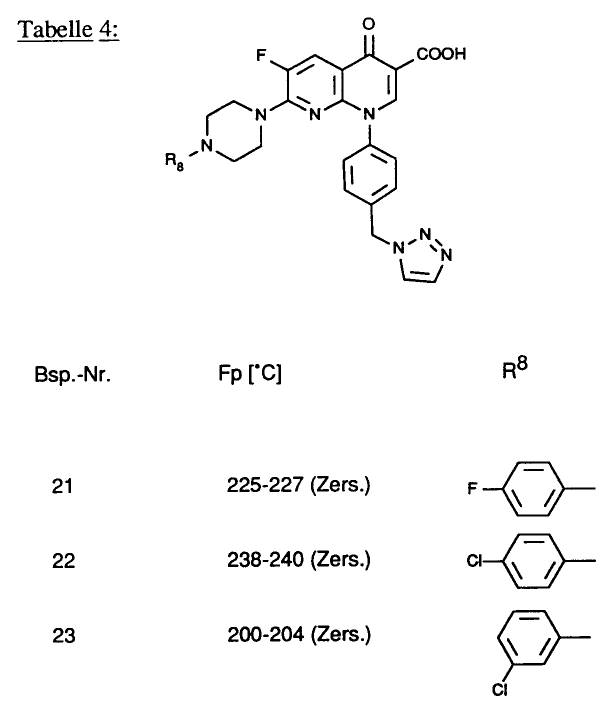

### Beispiel 24

### 6-Fluor-1,4-dihydro-4-oxo-7-(4-phenylpiperazin-1-yl)-1-[4-(2H-1,2,3-triazol-2-yl-methyl)phenyl]-3-chinolin-carbonsäureethylester

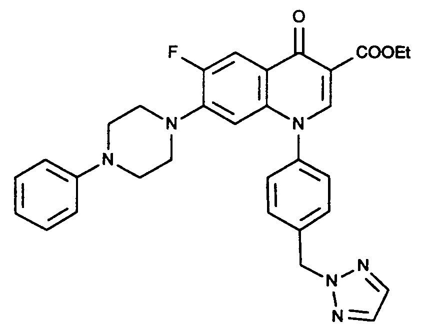

0,5 g (1,2 mmol) 6,7-Difluor-1,4-dihydro-4-oxo-1-[4-(2H-1,2,3-triazol-2-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid drei Stunden auf 120°C erwärmt. Alle flüchtigen Komponenten werden im Hochvakuum entfernt, der Rückstand mit Wasser verrührt und bei ca 100°C getrocknet.
Ausbeute: 580 mg (80 % der Theorie)
Schmelzpunkt: 230-232°C

In Analogie zur Vorschrift des Beispiels 24 werden die in Tabelle 5 aufgeführten Verbindungen hergestellt:

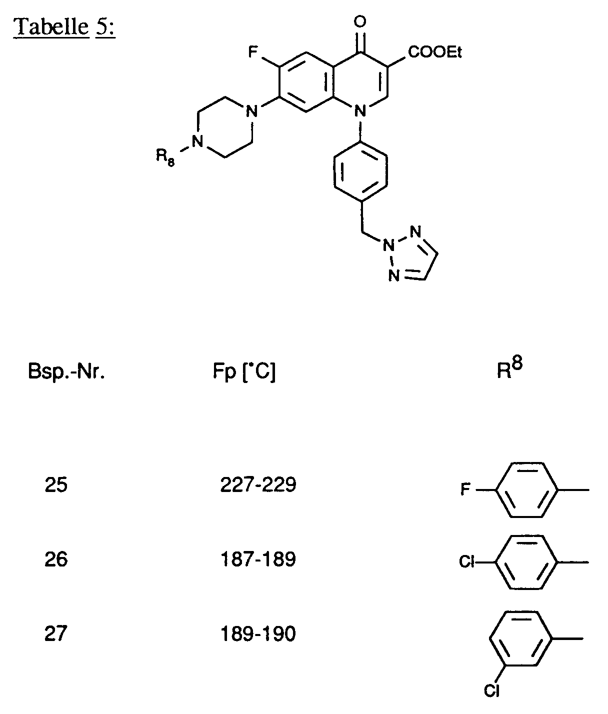

### Beispiel 28

### 6-Fluor-1,4-dihydro-4-oxo-7-(4-phenylpiperazin-1-yl)-1-[4-(2H-1,2,3-triazol-2-yl-methyl)phenyl]-3-chinolin-carbonsäure

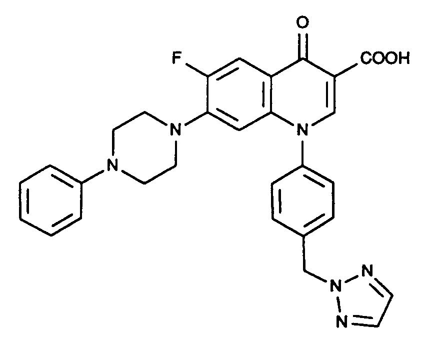

0,5 g (0,9 mmol) des im Beispiel 20 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,39 g (82% der Theorie)
Schmelzpunkt: 208-210°C

In Analogie zur Vorschrift des Beispiels 28 werden die in Tabelle 6 aufgeführten Verbindungen hergestellt:

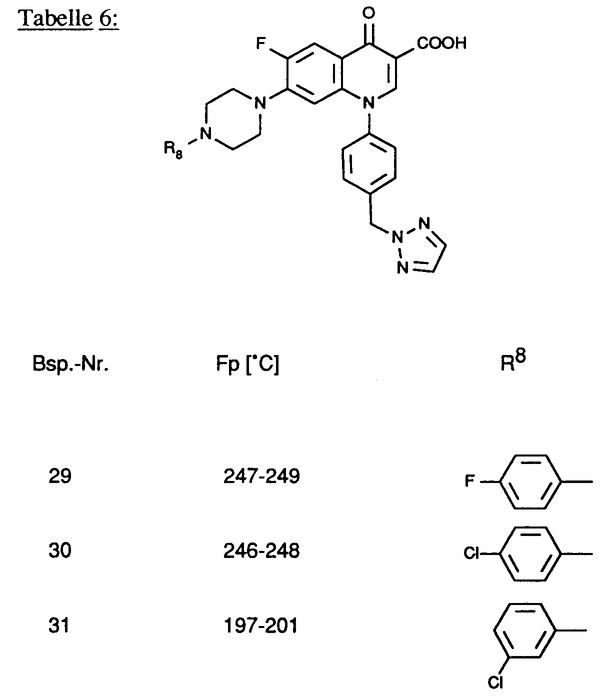

### Beispiel 32

### 6-Fluor-1,4-dihydro-4-oxo-7-(4-phenylpiperazin-1-yl)-1-[4-(1H-1,2,4-triazol-1-yl-methyl)phenyl]-3-chinolin-carbonsäureethylester

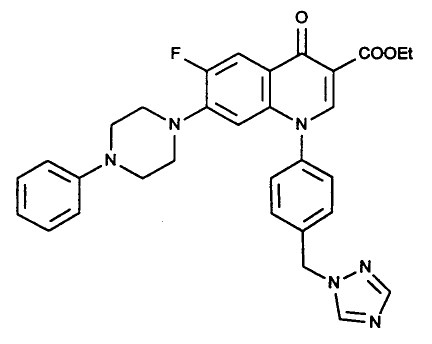

0,5 g (1,2 mmol) 6,7-Difluor-1,4-dihydro-4-oxo-1-[4-(1H-1,2,4-triazol-1-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid drei Stunden auf 120°C erwärmt. Alle flüchtigen Komponenten werden im Hochvakuum entfernt, der Rückstand mit Wasser verrührt und bei ca 100°C getrocknet.
Ausbeute: 600 mg (89 % der Theorie)
Schmelzpunkt: 217-218°C

In Analogie zur Vorschrift des Beispiels 32 werden die in Tabelle 7 aufgeführten Verbindungen hergestellt:

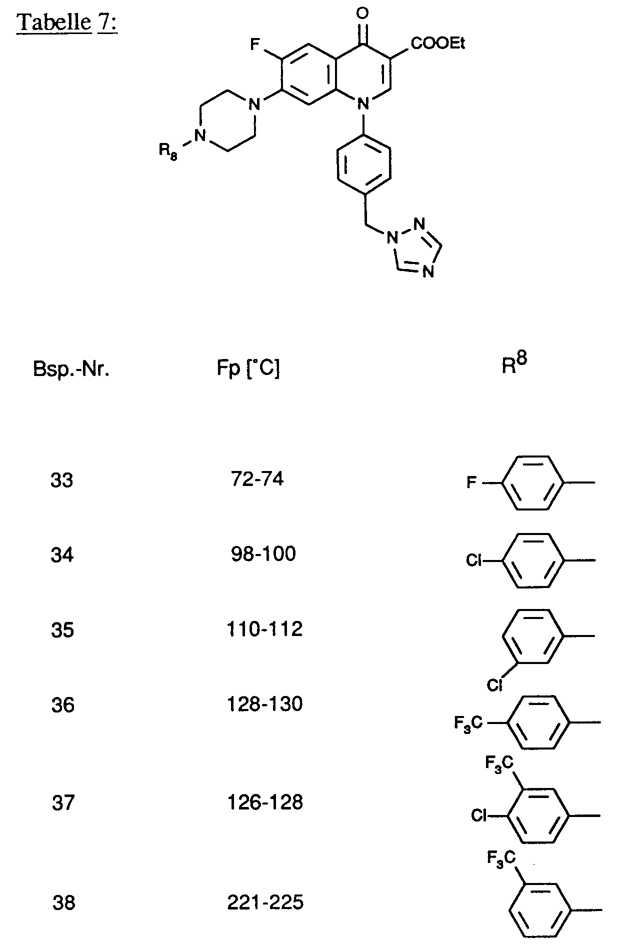

### Beispiel 39

### 6-Fluor-1,4-dihydro-4-oxo-7-(4-phenylpiperazin-1-yl)-1-[4-(1H-1,2,4-triazol-1-yl-methyl)phenyl]-3-chinolin-carbonsäure

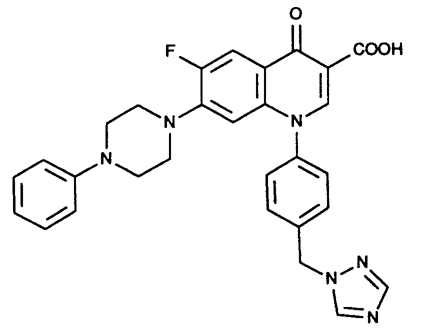

0,5 g (0,9 mmol) des im Beispiel 32 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,46 g (82% der Theorie)
Schmelzpunkt: 234-236°C

In Analogie zur Vorschrift des Beispiels 39 werden die in Tabelle 8 aufgeführten Verbindungen hergestellt:

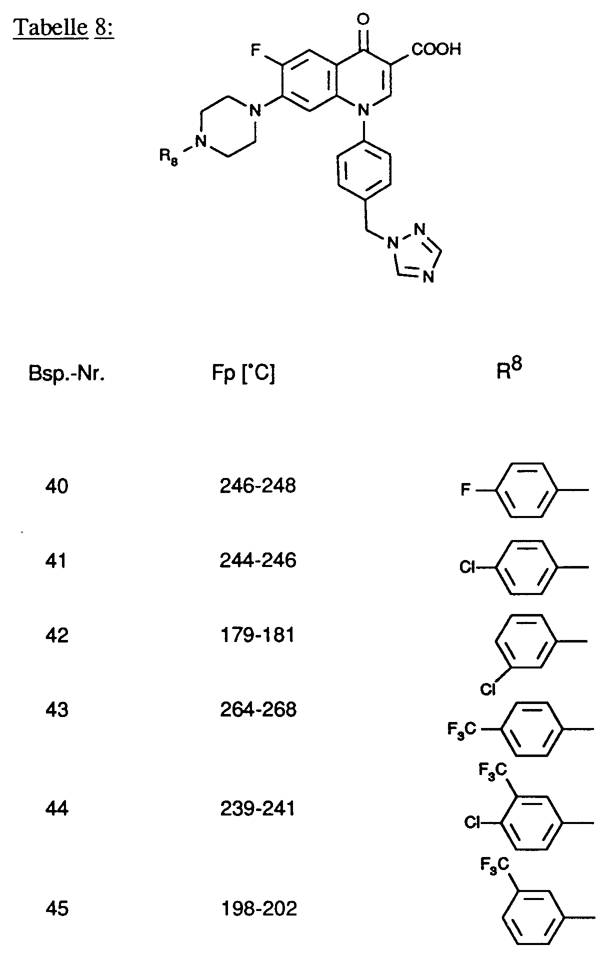

### Beispiel 46

### 6-Fluor-1,4-dihydro-4-oxo-7-(4-phenylpiperazin-1-yl)-1-[4-(1H-1,2,4-triazol-1-yl-methyl)phenyl]-1,8-naphthyridin-3-carbonsäureethylester

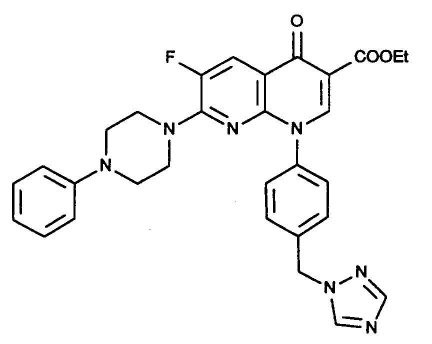

0,5 g (1,2 mmol) 7-Chlor-6-fluor-1,4-dihydro-4-oxo-1-[4-(1H-1,2,4-triazol-1-yl-methyl)-phenyl]-1,8-naphthyridin-3-carbonsäureethylester werden mit 190 mg (1,2 mmol) N-Phenylpiperazin und 460 mg (4,1 mmol) Diazabicyclo[2.2.2]octan in 12 ml Dimethylsulfoxid sechs Stunden auf 120°C erwärmt. Alle flüchtigen Komponenten werden im Hochvakuum entfernt. Der Rückstand wird mit Wasser verrührt und durch Säulenchromatographie (Kieselgel, Methylenchlorid/Methanol 96/4) gereinigt.
Ausbeute: 500 mg (78 % der Theorie)
Schmelzpunkt: 179-180°C

In Analogie zur Vorschrift des Beispiels 46 werden die in Tabelle 9 aufgeführten Verbindungen hergestellt:

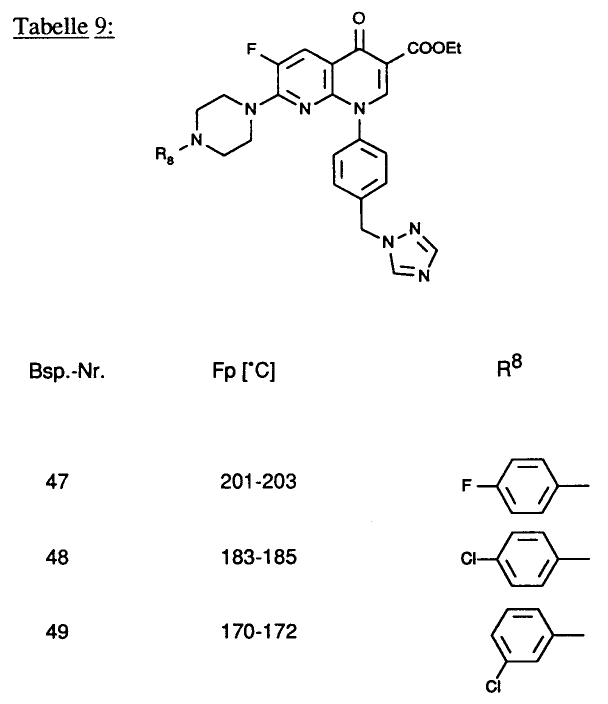

### Beispiel 50

### 6-Fluor-1,4-dihydro-4-oxo-7-(4-phenylpiperazin-1-yl)-1-[4-(1H-1,2,4-triazol-1-yl-methyl)phenyl]-1,8-naphthyridin-3-carbonsäure

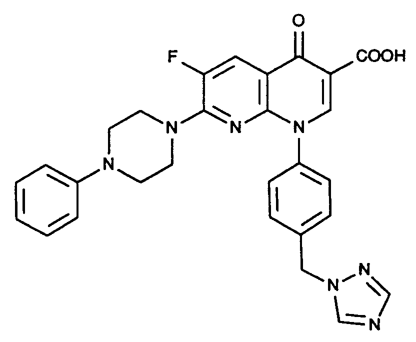

0,45 g (0,8 mmol) des Esters aus Beispiel 46 werden mit 1,6 ml 1 N Natronlauge in einem Gemisch aus 8 ml Ethanol und 8 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,39 mg (90% der Theorie)
Schmelzpunkt: 234-238°C (unter Zersetzung)

In Analogie zur Vorschrift des Beispiels 50 werden die in Tabelle 10 aufgeführten Verbindungen hergestellt:

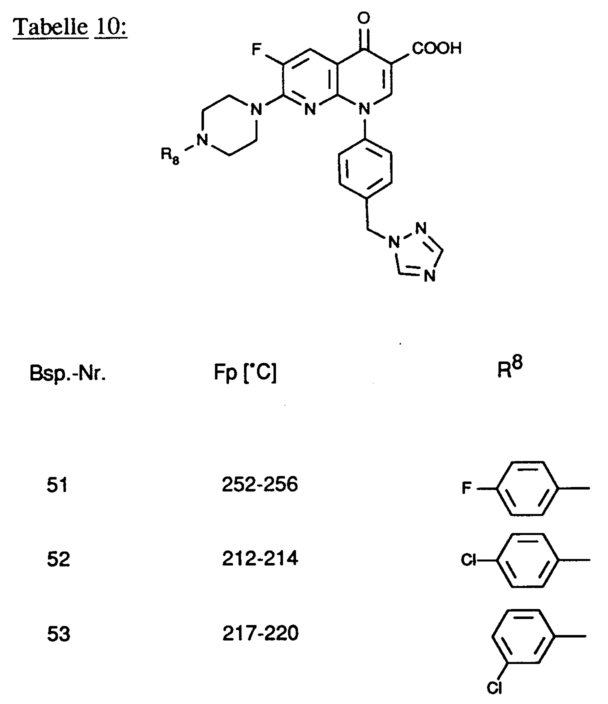

### Beispiel 54

### 6-Fluor-1,4-dihydro-1-[4-(N-imidazolyl-methyl)phenyl]-4-oxo-7-(4-phenylpiperazin-1-yl)-3-chinolin-carbonsäureethylester

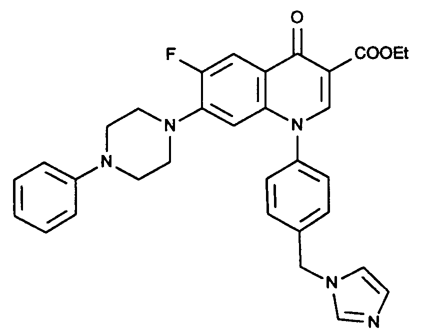

0,5 g (1,2 mmol) 6,7-Difluor-1,4-dihydro-1-[4-(N-imidazolyl-methyl)-phenyl]-4-oxo-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid fünf Stunden auf 120°C erwärmt. Alle flüchtigen Komponenten werden im Hochvakuum entfernt, der Rückstand mit Wasser verrührt und bei ca 100°C getrocknet.
Ausbeute: 580 mg (86 % der Theorie)
Schmelzpunkt: 130-133°C

In Analogie zur Vorschrift des Beispiels 54 werden die in Tabelle 11 aufgeführten Verbindungen hergestellt:

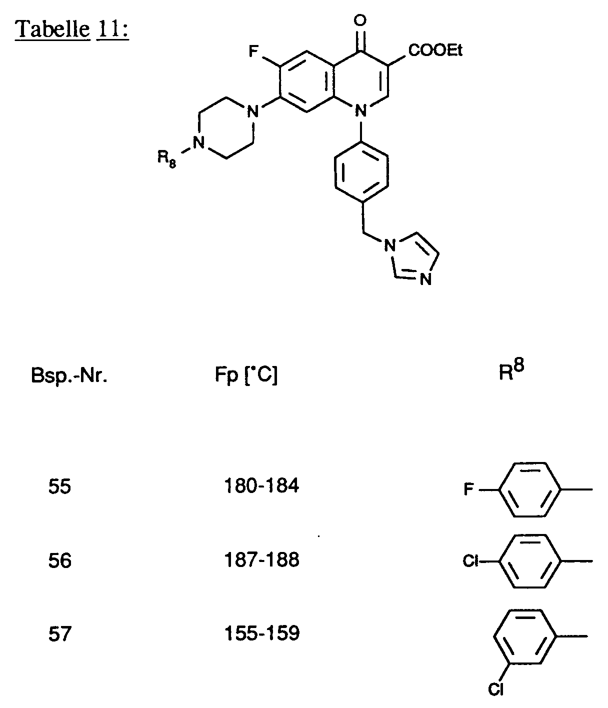

### Beispiel 58

### 6-Fluor-1,4-dihydro-1-[4-(N-imidazolyl-methyl)-phenyl]-4-oxo-7-(4-phenylpiperazin-1-yl)-3-chinolin-carbonsäure

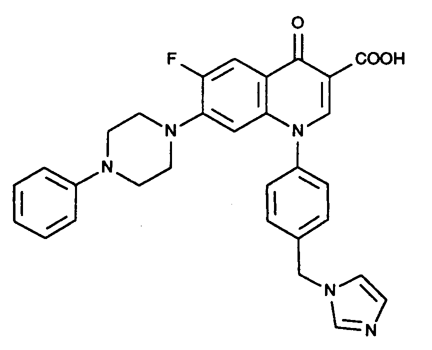

0,5 g (0,9 mmol) des im Beispiel 55 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,42 g (89% der Theorie)
Schmelzpunkt: 248-251°C

In Analogie zur Vorschrift des Beispiels 58 werden die in Tabelle 12 aufgeführten Verbindungen hergestellt:

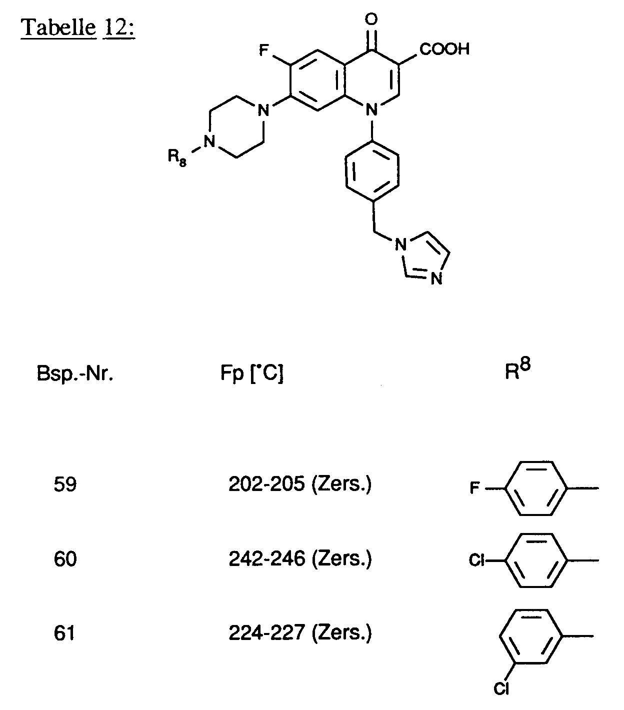

### Beispiel 62

### 6-Fluor-1,4-dihydro-4-oxo-7-(4-phenylpiperazin-1-yl)-1-[3-(1H-1,2,3-triazol-1-yl-methyl)phenyl]-3-chinolin-carbonsäureethylester

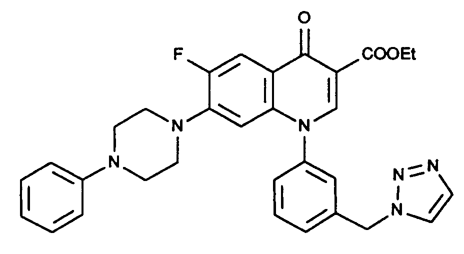

0,5 g (1,2 mmol) 6,7-Difluor-1,4-dihydro-4-oxo-1-[3-(1H-1,2,3-triazol-1-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid drei Stunden auf 120°C erwärmt. Alle flüchtigen Komponenten werden im Hochvakuum entfernt, der Rückstand mit Wasser verrührt und bei ca 100°C getrocknet.
Ausbeute: 650 mg (97 % der Theorie)
Schmelzpunkt: 170-172°C

In Analogie zur Vorschrift des Beispiels 62 werden die in Tabelle 13 aufgeführten Verbindungen hergestellt:

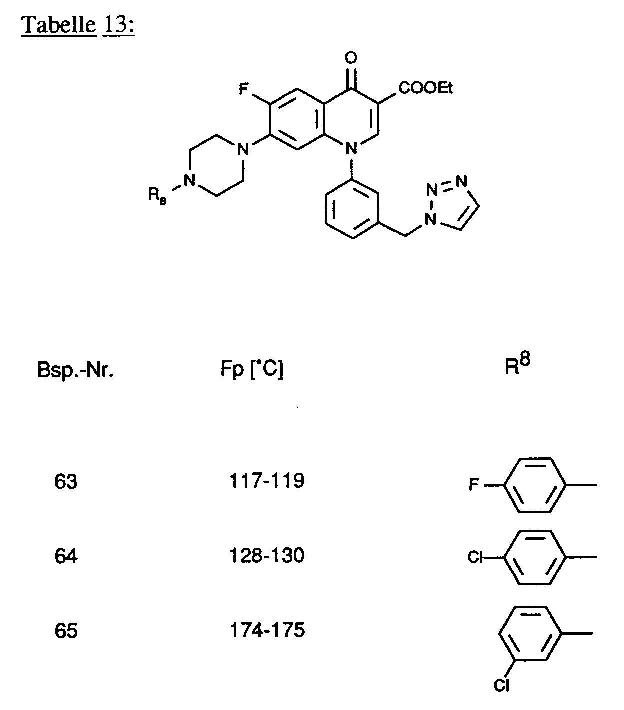

### Beispiel 66

### 6-Fluor-1,4-dihydro-4-oxo-7-(4-phenylpiperazin-1-yl)-1-[3-(1H-1,2,3-triazol-1-yl-methyl)phenyl]-3-chinolin-carbonsäure

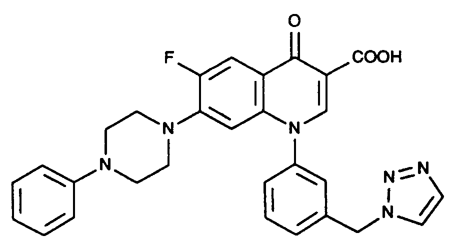

0,55 g (0,9 mmol) des im Beispiel 62 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,31 g (64% der Theorie)
Schmelzpunkt: 243-245°C

In Analogie zur Vorschrift des Beispiels 66 werden die in Tabelle 14 aufgeführten Verbindungen hergestellt:

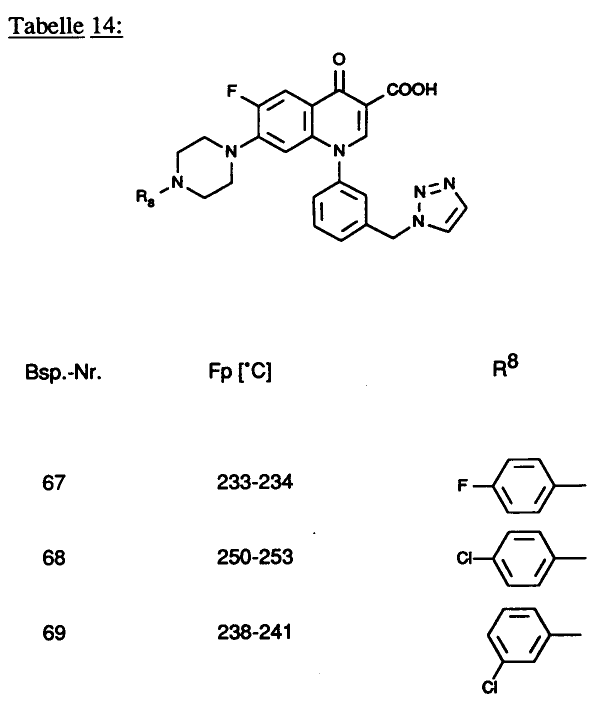

### Beispiel 70

### 6-Fluor-1,4-dihydro-4-oxo-7-(4-phenylpiperazin-1-yl)-1-[3-(2H-1,2,3-triazol-2-yl-methyl)phenyl]-3-chinolin-carbonsäure-ethylester

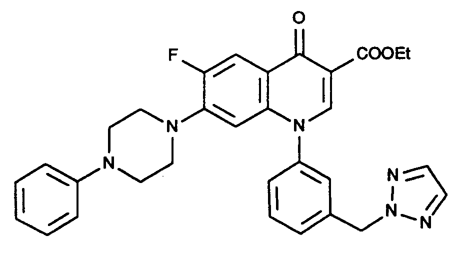

0,5 g (1,2 mmol) 6,7-Difluor-1,4-dihydro-4-oxo-1-[3-(2H-1,2,3-triazol-2-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid drei Stunden auf 120°C erwärmt. Alle flüchtigen Komponenten werden im Hochvakuum entfernt, der Rückstand mit Wasser verrührt und bei ca 100°C getrocknet.
Ausbeute: 590 mg (89 % der Theorie)
Schmelzpunkt: 112-115°C

In Analogie zur Vorschrift des Beispiels 70 werden die in Tabelle 15 aufgeführten Verbindungen hergestellt:

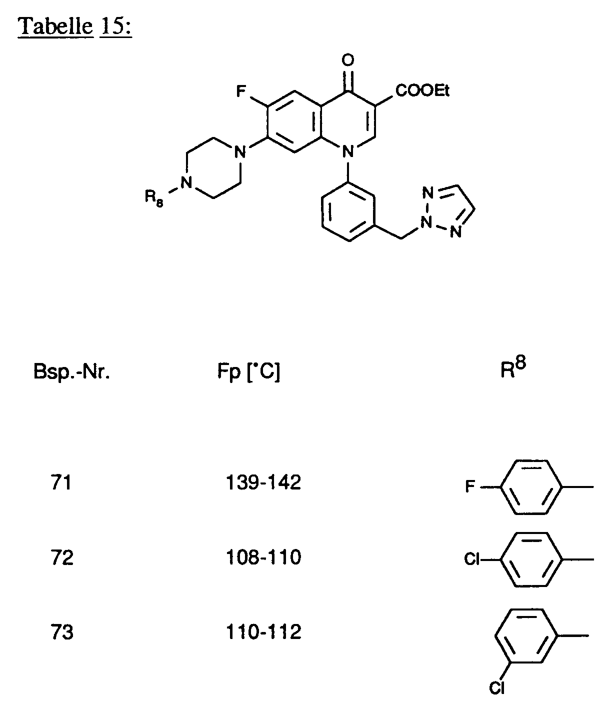

### Beispiel 74

### 6-Fluor-1,4-dihydro-4-oxo-7-(4-phenylpiperazin-1-yl)-1-[3-(2H-1,2,3-triazol-2-yl-methyl)phenyl]-3-chinolin-carbonsäure

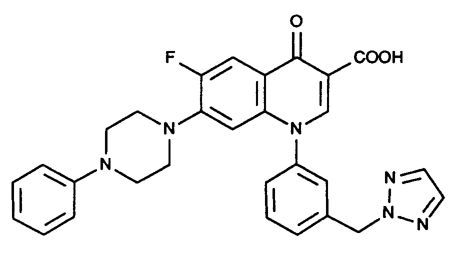

0,5 g (0,9 mmol) des im Beispiel 70 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,2 g (42% der Theorie)
Schmelzpunkt: 233-235°C

In Analogie zur Vorschrift des Beispiels 74 werden die in Tabelle 16 aufgeführten Verbindungen hergestellt:

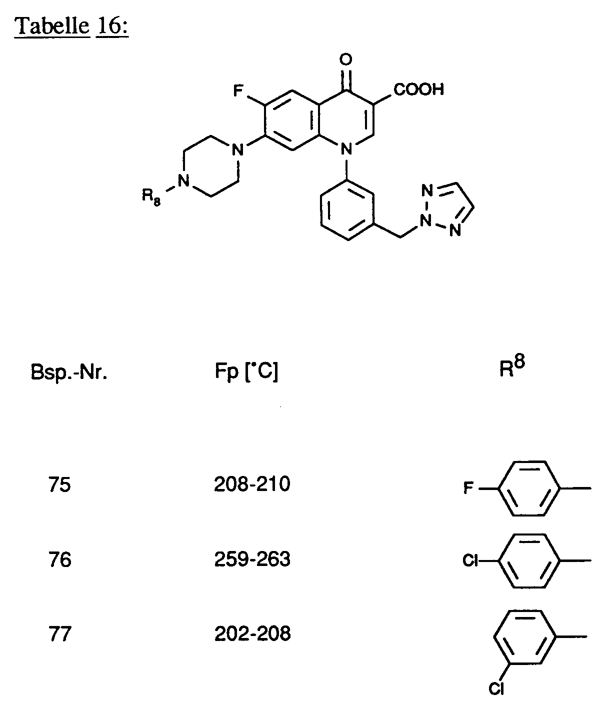

### Beispiel 78

### 6-Fluor-1,4-dihydro-4-oxo-7-(4-phenylpiperazin-1-yl)-1-[3-(1H-1,2,4-triazol-1-yl-methyl)phenyl]-3-chinolin-carbonsäure-ethylester

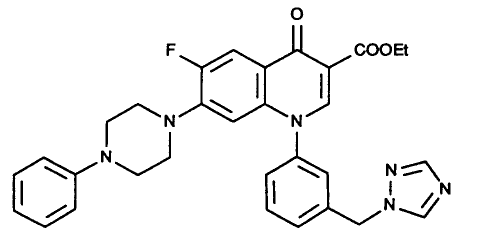

0,5 g (1,2 mmol) 6,7-Difluor-1,4-dihydro-4-oxo-1-[3-(1H-1,2,4-triazol-1-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid sechs Stunden auf 120°C erwärmt. Alle flüchtigen Komponenten werden im Hochvakuum entfernt, der Rückstand mit Wasser verrührt und bei ca 100°C getrocknet.
Ausbeute: 600 mg (89% der Theorie)
Schmelzpunkt: 160-162°C

In Analogie zur Vorschrift des Beispiels 78 werden die in Tabelle 17 aufgeführten Verbindungen hergestellt:

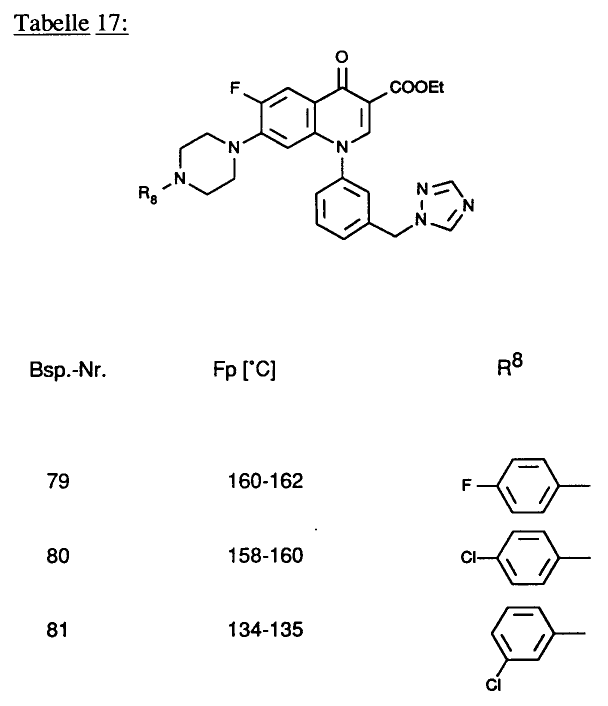

### Beispiel 82

### 6-Fluor-1,4-dihydro-4-oxo-7-(4-phenylpiperazin-1-yl)-1-[3-(1H-1,2,4-triazol-1-yl-methyl)phenyl]-3-chinolin-carbonsäure

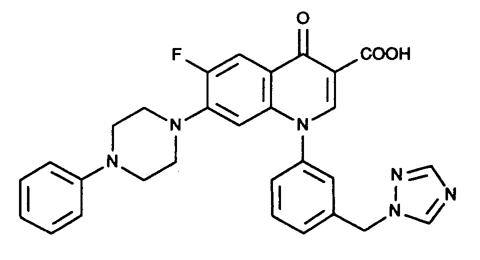

0,5 g (0,9 mmol) des im Beispiel 78 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,44 g (93% der Theorie)
Schmelzpunkt: 256-260°C

In Analogie zur Vorschrift des Beispiels 82 werden die in Tabelle 18 aufgeführten Verbindungen hergestellt:

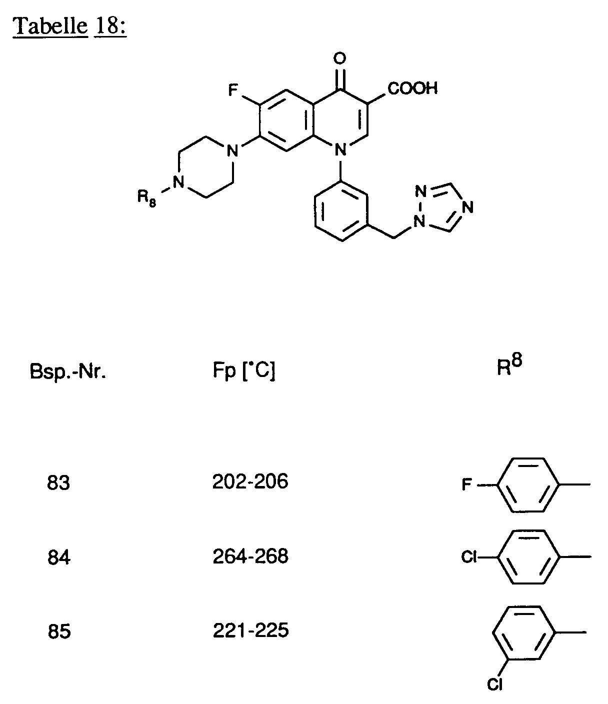

### Beispiel 86

### 6-Fluor-1,4-dihydro-4-oxo-7-(4-phenylpiperazin-1-yl)-1-[3-(1H-1,2,4-triazol-1-yl-methyl)phenyl]-1,8-naphthyridin-3-carbonsäureethylester

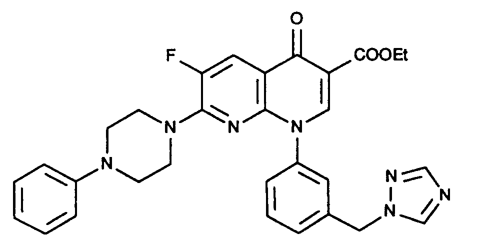

0,5 g (1,2 mmol) 7-Chlor-6-fluor-1,4-dihydro-4-oxo-1-[3-(1H-1,2,4-triazol-1-yl-methyl)-phenyl]-1,8-naphthyridin-3-carbon-säureethylester werden mit 190 mg (1,2 mmol) N-Phenylpiperazin und 460 mg (4,1 mmol) Diazabicyclo[2.2.2]octan in 12 ml Dimethylsulfoxid vier Stunden auf 120°C erwärmt. Alle flüchtigen Komponenten werden im Hochvakuum entfernt. Der Rückstand wird mit Wasser verrührt und durch Säulenchromatographie (Kieselgel, Methylenchlorid/Methanol 9/1) gereinigt.
Ausbeute: 280 mg (43 % der Theorie)
Schmelzpunkt: 109-111°C

In Analogie zur Vorschrift des Beispiels 86 werden die in Tabelle 19 aufgeführten Verbindungen hergestellt:

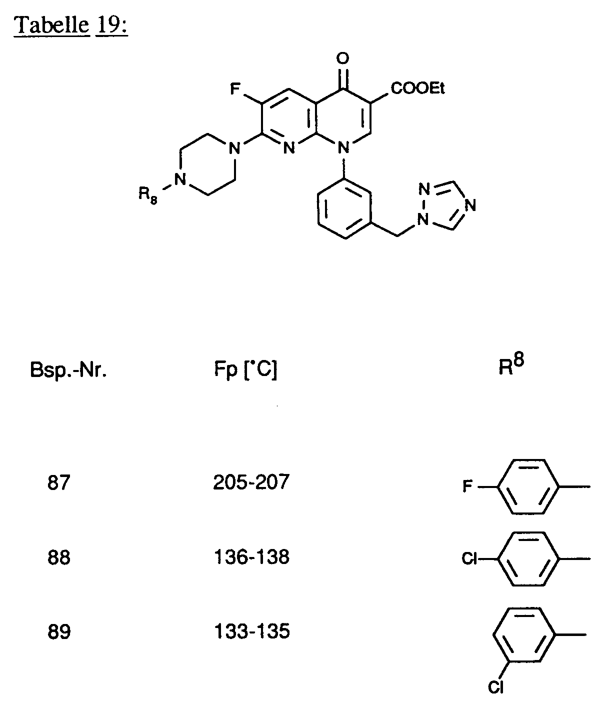

### Beispiel 90

### 6-Fluor-1,4-dihydro-4-oxo-7-(4-phenylpiperazin-1-yl)-1-[3-(1H-1,2,4-triazol-1-yl-methyl)phenyl]-1,8-naphthyridin-3-carbonsäure

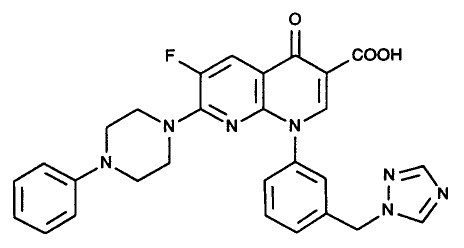

0,25 g (0,4 mmol) des Esters aus Beispiel 46 werden mit 0,8 ml 1 N Natronlauge in einem Gemisch aus 4 ml Ethanol und 4 ml Wasser 1,5 Stunden auf 50°C erwärmt. Es wird mit verdünnter Salzsäure neutralisiert, die Losungsmittel werden im Vakuum entfernt. Der Rückstand wird durch Säulenchromatographie (Kieselgel, Methylenchlorid/Methanol 9/1) gereinigt.
Ausbeute: 0,21 mg (90% der Theorie)
Schmelzpunkt: 264-268°C (unter Zersetzung)

In Analogie zur Vorschrift des Beispiels 90 werden die in Tabelle 20 aufgeführten Verbindungen hergestellt:

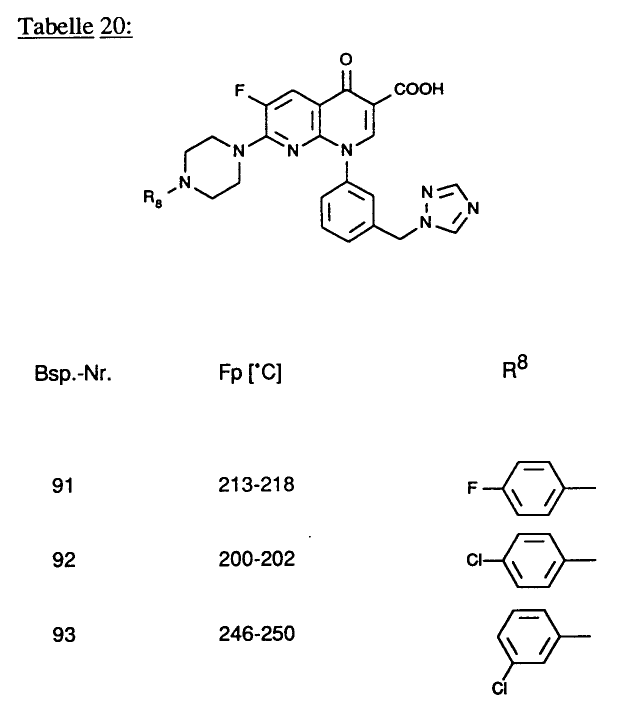

### Beispiel 94

### 6-Fluor-1,4-dihydro-1-[3-(N-imidazolyl-methyl)phenyl]-4-oxo-7-(4-phenylpiperazin-1-yl)-3-chinolin-carbonsäureethylester

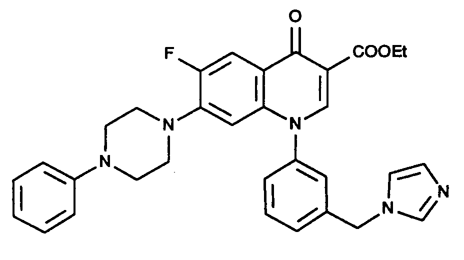

0,5 g (1,2 mmol) 6,7-Difluor-1,4-dihydro-1-[3-(N-imidazolyl-methyl)-phenyl]-4-oxo-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid vier Stunden auf 120°C erwärmt. Alle flüchtigen Komponenten werden im Hochvakuum entfernt, der Rückstand mit Wasser verrührt und bei ca 100°C getrocknet.
Ausbeute: 590 mg (88 % der Theorie)
Schmelzpunkt: 187-190°C

In Analogie zur Vorschrift des Beispiels 94 werden die in Tabelle 21 aufgeführten Verbindungen hergestellt:

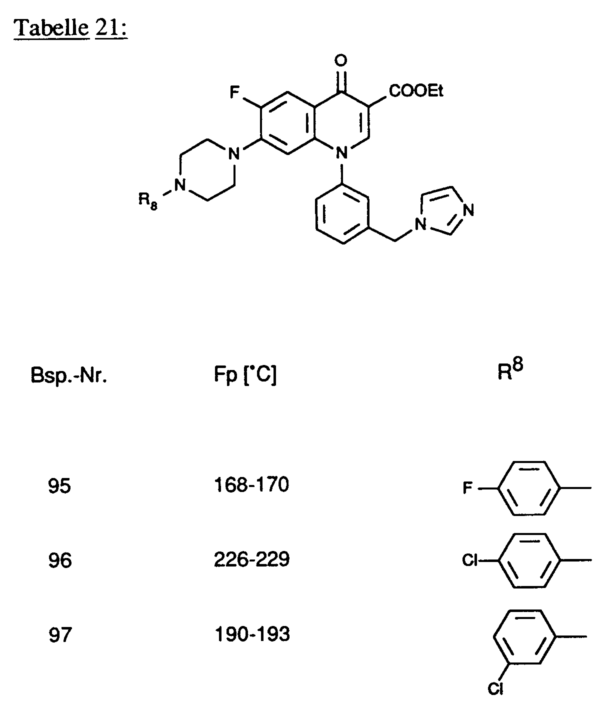

### Beispiel 98

### 6-Fluor-1,4-dihydro-1-[3-(N-imidazolyl-methyl)phenyl]-4-oxo-7-(4-phenylpiperazin-1-yl)-3-chinolin-carbonsäure

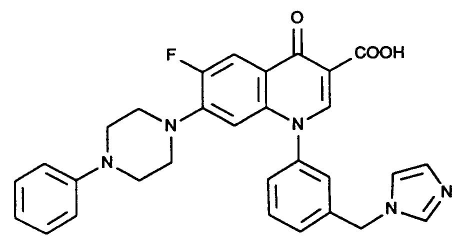

0,5 g (0,9 mmol) des im Beispiel 95 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,45 g (95% der Theorie)
Schmelzpunkt: 207-210°C

In Analogie zur Vorschrift des Beispiels 98 werden die in Tabelle 22 aufgeführten Verbindungen hergestellt:

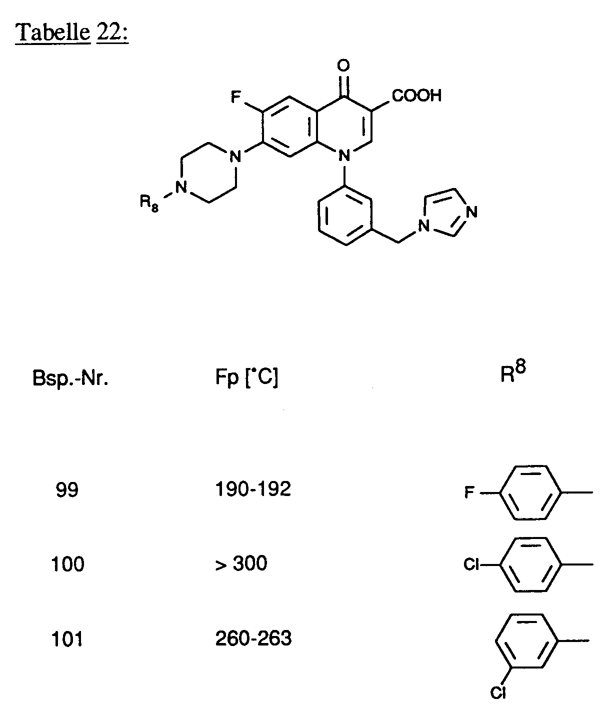

### Beispiel 102

### 6-Fluor-1,4-dihydro-4-oxo-7-(4-phenylpiperazin-1-yl)-1-[2-(1H-1,2,3-triazol-1-yl-methyl)phenyl]-3-chinolin-carbonsäure-ethylester

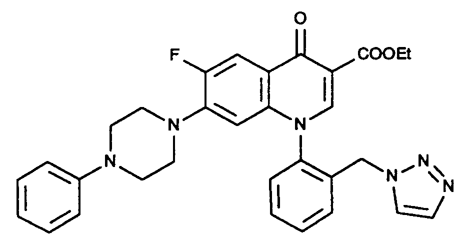

0,5 g (1,2 mmol) 6,7-Difluor-1,4-dihydro-4-oxo-1-[2-(1H-1,2,3-triazol-1-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid drei Stunden auf 120°C erwärmt. Alle flüchtigen Komponenten werden im Hochvakuum entfernt, der Rückstand mit Wasser verrührt und bei ca 100°C getrocknet.
Ausbeute: 580 mg (86 % der Theorie)
Schmelzpunkt: 138-141°C

In Analogie zur Vorschrift des Beispiels 102 werden die in Tabelle 23 aufgeführten Verbindungen hergestellt:

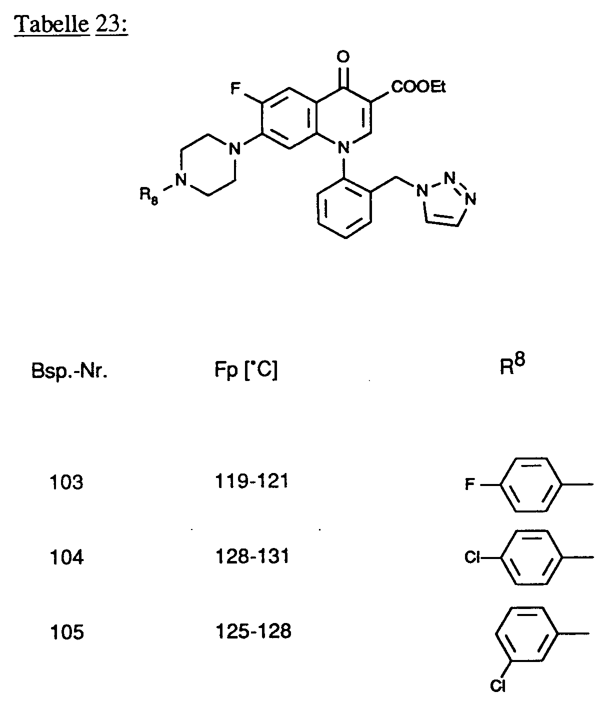

### Beispiel 106

### 6-Fluor-1,4-dihydro-4-oxo-7-(4-phenylpiperazin-1-yl)-1-[2-(1H-1,2,3-triazol-1-yl-methyl)phenyl]-3-chinolin-carbonsäure

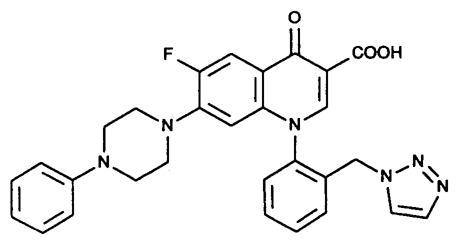

0,5 g (0,9 mmol) des im Beispiel 102 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 83 mg (18% der Theorie)
Schmelzpunkt: 235-238°C

In Analogie zur Vorschrift des Beispiels 106 werden die in Tabelle 24 aufgeführten Verbindungen hergestellt:

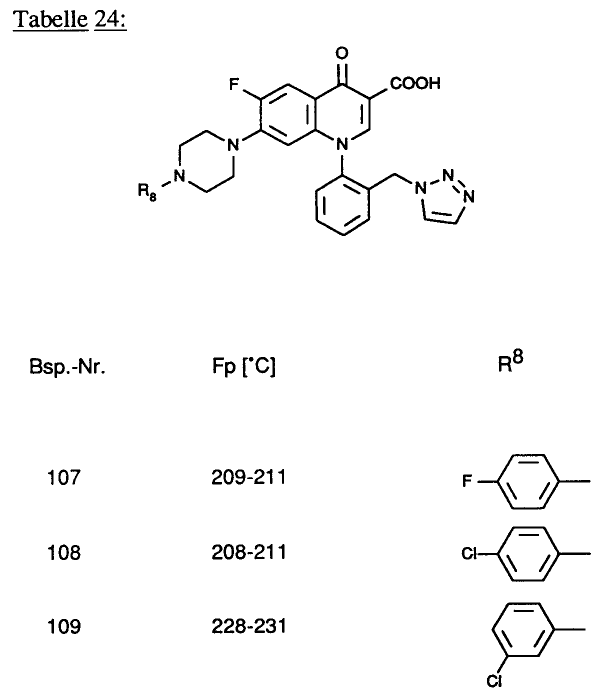

### Beispiel 110

### 6-Fluor-1,4-dihydro-4-oxo-7-(4-phenylpiperazin-1-yl)-1-[2-(2H-1,2,3-triazol-2-yl-methyl)phenyl]-3-chinolin-carbonsäure-ethylester

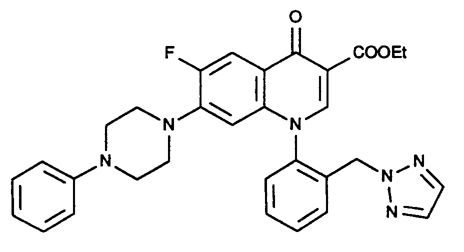

0,5 g (1,2 mmol) 6,7-Difluor-1,4-dihydro-4-oxo-1-[2-(2H-1,2,3-triazol-2-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid drei Stunden auf 120°C erwärmt. Alle flüchtigen Komponenten werden im Hochvakuum entfernt, der Rückstand mit Wasser verrührt und bei ca 100°C getrocknet.
Ausbeute: 590 mg (80 % der Theorie)
Schmelzpunkt: 198-200°C

In Analogie zur Vorschrift des Beispiels 110 werden die in Tabelle 25 aufgeführten Verbindungen hergestellt:

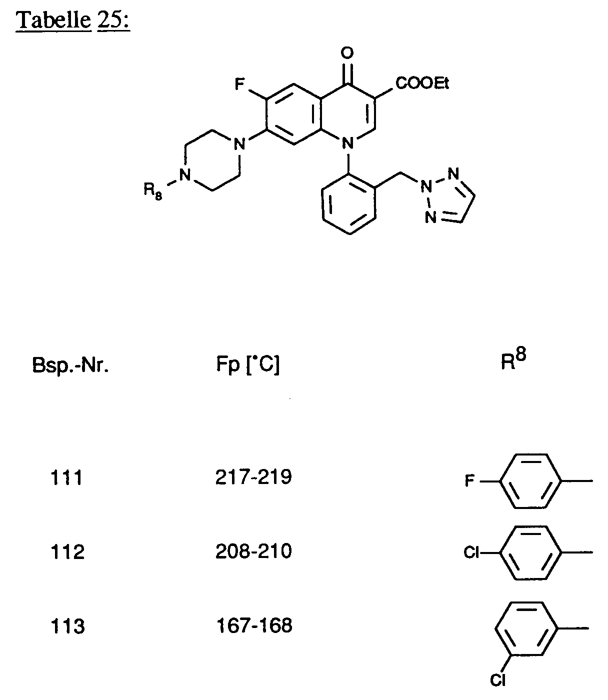

### Beispiel 114

### 6-Fluor-1,4-dihydro-4-oxo-7-(4-phenylpiperazin-1-yl)-1-[3-(2H-1,2,3-triazol-2-yl-methyl)phenyl]-3-chinolin-carbonsäure

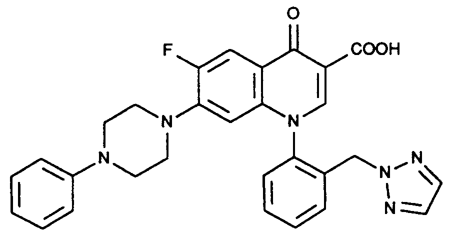

0,5 g (0,9 mmol) des im Beispiel 110 erhaltenen Esters werden mit 1,8 ml 1 N Natronlauge in einem Gemisch aus 9 ml Ethanol und 9 ml Wasser 1,5 Stunden auf 50°C erwärmt. Es wird mit verdünnter Salzsäure neutralisiert, die Losungsmittel werden im Vakuum entfernt. Der Rückstand wird durch Säulenchromatographie (Kieselgel, Methylenchlorid/Methanol 9/1) gereinigt.
Ausbeute: 280 mg (60% der Theorie)
Schmelzpunkt: 270-273°C

In Analogie zur Vorschrift des Beispiels 114 werden die in Tabelle 26 aufgeführten Verbindungen hergestellt:

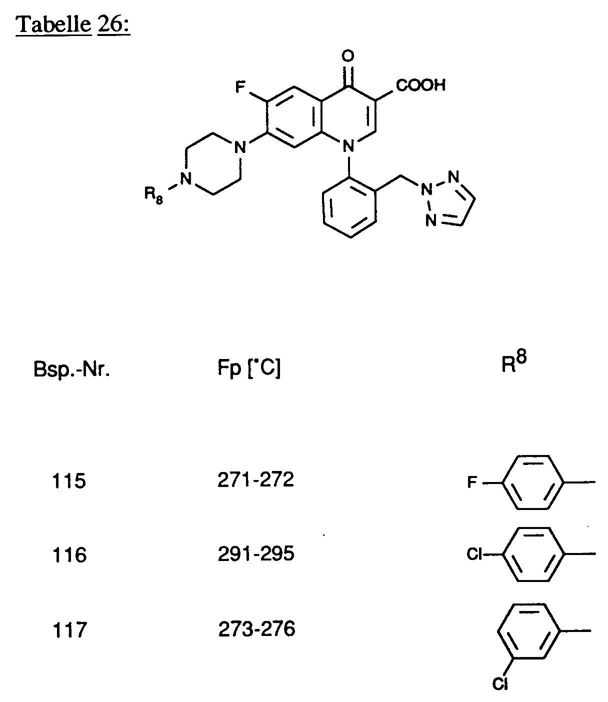

### Beispiel 118

### 6-Fluor-1,4-dihydro-4-oxo-7-(4-phenylpiperazin-1-yl)-1-[2-(1H-1,2,4-triazol-1-yl-methyl)phenyl]-3-chinolin-carbonsäure-ethylester

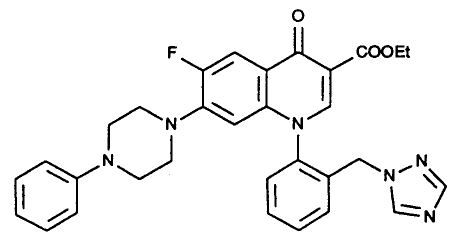

0,5 g (1,2 mmol) 6,7-Difluor-1,4-dihydro-4-oxo-1-[2-(1H-1,2,4-triazol-1-yl-methyl)-phenyl]-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid vier Stunden auf 120°C erwärmt. Alle flüchtigen Komponenten werden im Hochvakuum entfernt, der Rückstand mit Wasser verrührt und bei ca 100°C getrocknet.
Ausbeute: 420 mg (62 % der Theorie)
Schmelzpunkt: 214-217°C

In Analogie zur Vorschrift des Beispiels 118 werden die in Tabelle 27 aufgeführten Verbindungen hergestellt:

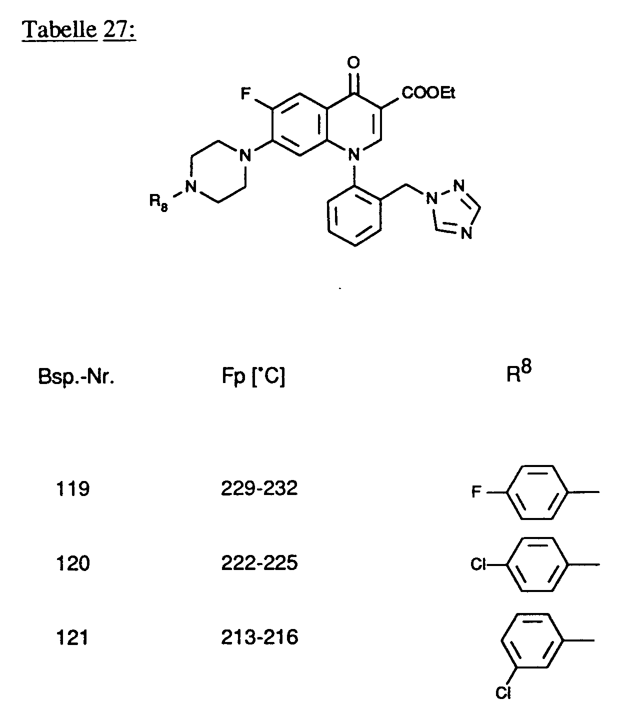

### Beispiel 122

### 6-Fluor-1,4-dihydro-4-oxo-7-(4-phenylpiperazin-1-yl)-1-[2-(1H-1,2,4-triazol-1-yl-methyl)phenyl]-3-chinolin-carbonsäure

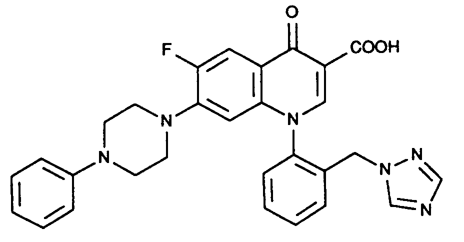

0,35 g (0,6 mmol) des im Beispiel 118 erhaltenen Esters werden mit 1,2 ml 1 N Natronlauge in einem Gemisch aus 6 ml Ethanol und 6 ml Wasser zwei Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 280 mg (89% der Theorie)
Schmelzpunkt: 270-274°C

In Analogie zur Vorschrift des Beispiels 122 werden die in Tabelle 28 aufgeführten Verbindungen hergestellt:

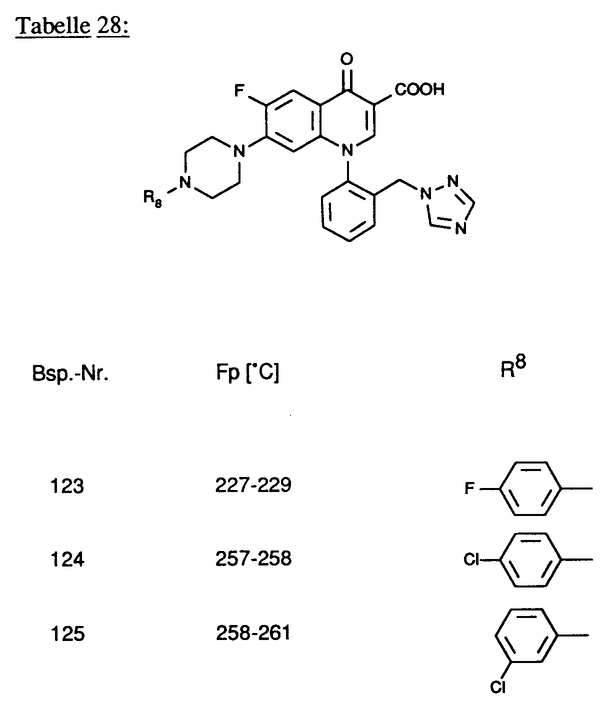

### Beispiel 126

### 6-Fluor-1,4-dihydro-1-[2-(N-imidazolyl-methyl)phenyl]-4-oxo-7-(4-phenylpiperazin-1-yl)-3-chinolin-carbonsäureethylester

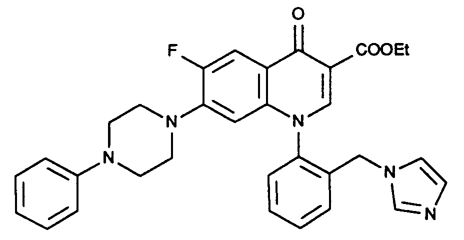

0,5 g (1,2 mmol) 6,7-Difluor-1,4-dihydro-1-[2-(N-imidazolyl-methyl)-phenyl]-4-oxo-3-chinolincarbonsäureethylester werden mit 190 mg (1,2 mmol) N-Phenylpiperazin und 460 mg (4,1 mmol) Diazabicyclo[2.2.2]octan in 12 ml Dimethylsulfoxid zwei Stunden auf 120°C erwärmt. Alle flüchtigen Komponenten werden im Hochvakuum entfernt, der Rückstand mit Wasser verrührt und bei ca 100°C getrocknet.
Ausbeute: 470 mg (71 % der Theorie)
Schmelzpunkt: 117-119°C

In Analogie zur Vorschrift des Beispiels 126 werden die in Tabelle 29 aufgeführten Verbindungen hergestellt:

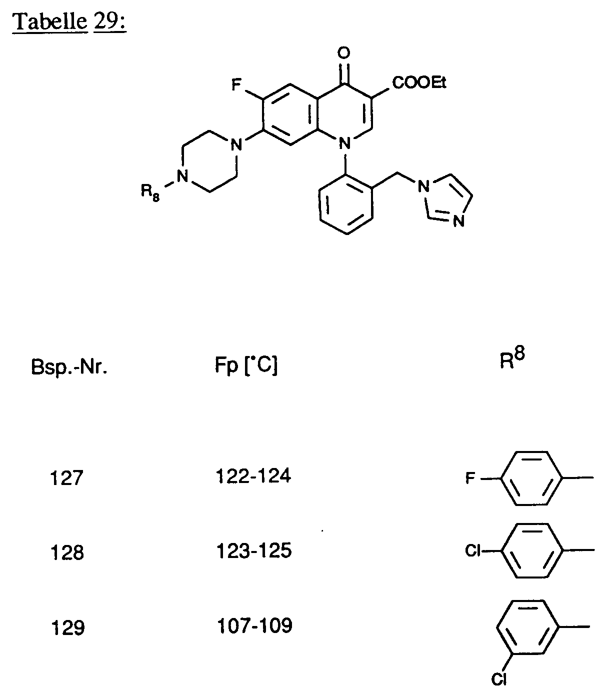

### Beispiel 130

### 6-Fluor-1,4-dihydro-1-[2-(N-imidazolyl-methyl)-phenyl]-4-oxo-7-(4-phenylpiperazin-1-yl)- 3-chinolin-carbonsäure

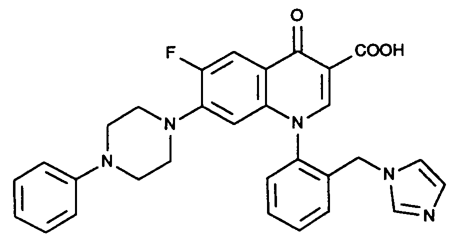

0,4 g (0,7 mmol) des im Beispiel 126 erhaltenen Esters werden mit 1,4 ml 1 N Natronlauge in einem Gemisch aus 7 ml Ethanol und 7 ml Wasser 1,5 Stunden auf 50°C erwärmt. Es wird mit verdünnter Salzsäure neutralisiert, die Lösungsmittel werden im Vakuum entfernt. Der Rückstand wird durch Säulenchromatographie (Kieselgel, Methylenchlorid/Methanol 9/1) gereinigt.
Ausbeute: 0,29 g (79% der Theorie)
Schmelzpunkt: 260-263°C

In Analogie zur Vorschrift des Beispiels 130 werden die in Tabelle 30 aufgeführten Verbindungen hergestellt:

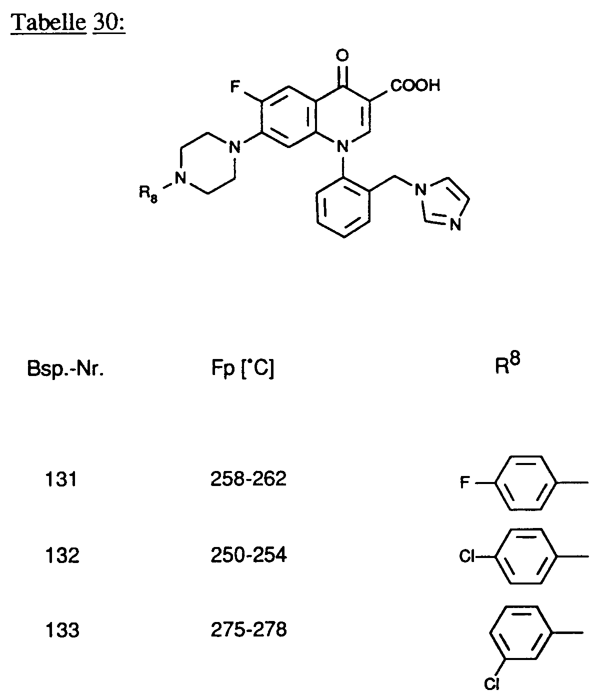

### Beispiel 134

### 6,8-Difluor-1,4-dihydro-4-oxo-7-(4-phenylpiperazin-1-yl)-1-[4-(1H-1,2,3-triazol-1-yl-methyl)phenyl]-3-chinolin-carbonsäure

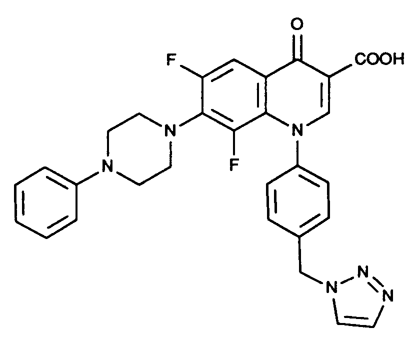

300 mg (0,75 mmol) 6,7,8-Trifluor-1,4-dihydro-4-oxo-1-[4-(1H-1,2,3-triazol-1-yl-methyl)-phenyl]-3-chinolincarbonsäure werden mit 410 mg (2,25 mmol) N-(4-Fluorphenyl)-piperazin in 8 ml DMSO auf 100°C erwärmt. Alle flüchtigen Komponenten werden im Hochvakuum entfernt, der Rückstand mit iso-Propanol verrührt und der anfallende Feststoff isoliert. Es wird nochmals mit iso-Propanol und Diethylether gewaschen und bei ca 100°C getrocknet.
Ausbeute: 387 mg (92% der Theorie)
Schmelzpunkt: 222-224°C

In Analogie zur Vorschrift des Beispiels 134 werden die in Tabelle 31 aufgeführten Verbindungen hergestellt:

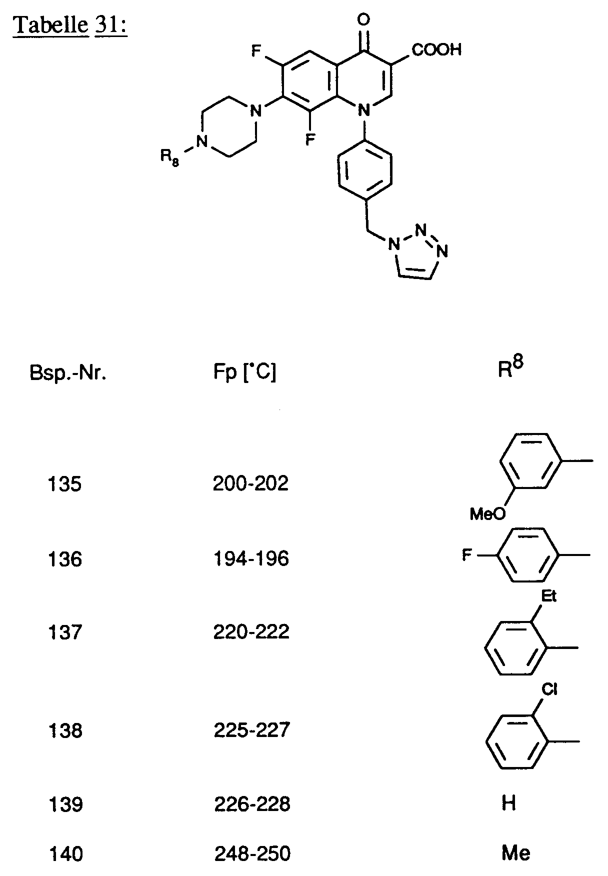

### Beispiel 141

### 8-Chlor-6-fluor-1,4-dihydro-4-oxo-7-[4-(4-fluorphenyl)-piperazin-1-yl]-1-[4-(1H-1,2,3-triazol-1-yl-methyl)-phenyl]-3-chinolin-carbonsäure

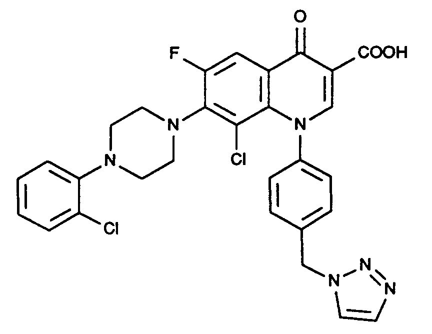

Analog zum Beispiel 134 wird bei der Umsetzung von 8-Chlor-6,7-difluor-1,4-dihydro-4-oxo-1-[4-(1H-1,2,3-triazol-1-yl-methyl)-phenyl]-3-chinolincarbonsäure mit N-(4-Fluorphenyl)-piperazin die Titelverbindung erhalten.
Schmelzpunkt: 128-130°C

### Beispiel 142

### 8-Chlor-6-fluor-1,4-dihydro-4-oxo-7-[4-(2-chlorphenyl)-piperazin-1-yl]-1-[4-(1H-1,2,3-triazol-1-yl-methyl)phenyl]-3-chinolin-carbonsäure

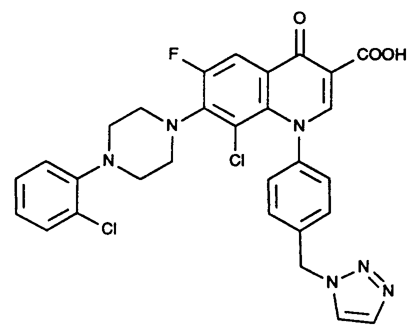

Analog zum Beispiel 134 wird bei der Umsetzung von 8-Chlor-6,7-difluor-1,4-dihydro-4-oxo-1-[4-(1H-1,2,3-triazol-1-yl-methyl)-phenyl]-3-chinolincarbonsäure mit N-(2-Chlorphenyl)-piperazin die Titelverbindung erhalten.
Schmelzpunkt: 158-160°C

### Beispiel 143

### 8-Chlor-6-fluor-1,4-dihydro-4-oxo-7-[4-(4-fluorphenyl)-piperazin-1-yl]-1-[4-(2H-1,2,3-triazol-2-yl-methyl)phenyl]-3-chinolin-carbonsäure

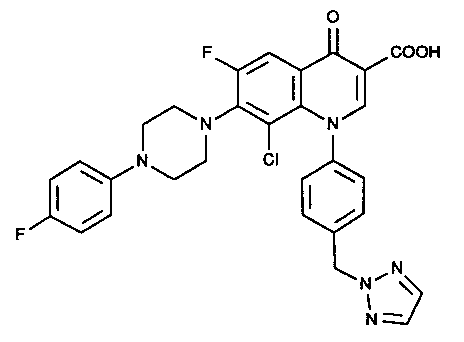

Analog zum Beispiel 134 wird bei der Umsetzung von 8-Chlor-6,7-difluor-1,4-dihydro-4-oxo-1-[4-(2H-1,2,3-triazol-2-yl-methyl)-phenyl]-3-chinolincarbonsäure mit N-(4-Fluorphenyl)-piperazin die Titelverbindung erhalten.
Schmelzpunkt: 213-215°C

### Beispiel 144

### 8-Chlor-6-fluor-1,4-dihydro-4-oxo-7-[4-(2-chlorphenyl)-piperazin-1-yl]-1-[4-(2H-1,2,3-triazol-2-yl-methyl)phenyl]-3-chinolin-carbonsäure

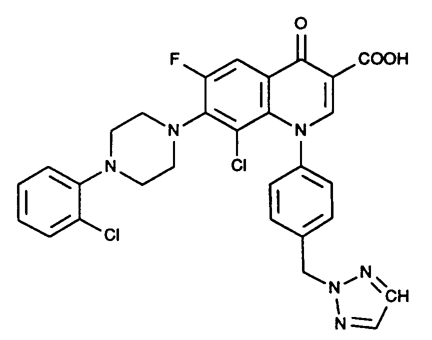

Analog zum Beispiel 134 wird bei der Umsetzung von 8-Chlor-6,7-difluor-1,4-dihydro-4-oxo-1-[4-(2H-1,2,3-triazol-2-yl-methyl)-phenyl]-3-chinolincarbonsäure mit N-(2-Chlorphenyl)-piperazin die Titelverbindung erhalten.
Schmelzpunkt: 228-230°C (unter Zersetzung)

In Analogie zur Vorschrift des Beispiels 144 werden bei der Umsetzung von 6,7,8-Trifluor-1,4-dihydro-4-oxo-1-[4-(1H-1,2,4-triazol-1-yl-methyl)-phenyl]-3-chinolincarbonsäure die folgenden Produkte erhalten:

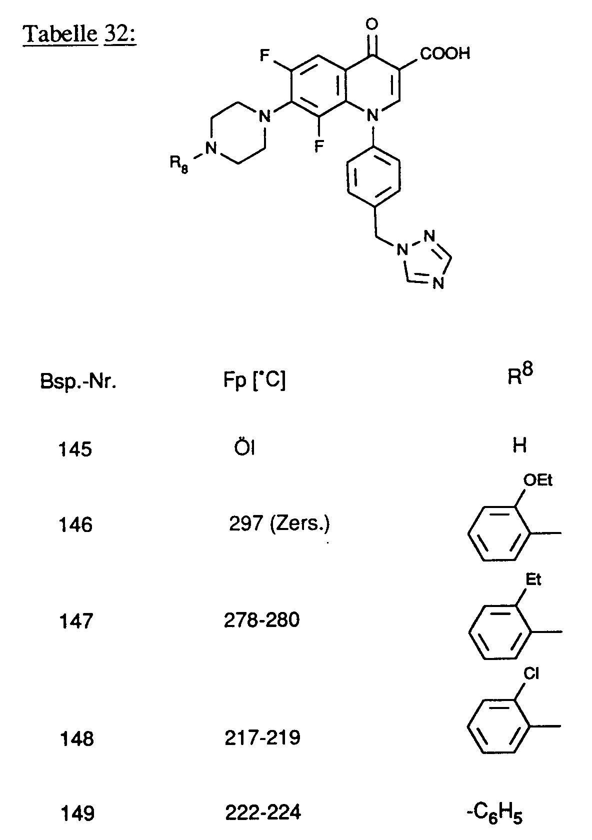

### Beispiel 150

### 8-Chlor-6-fluor-1,4-dihydro-4-oxo-7-[4-(4-fluorphenyl)-piperazin-1-yl]-1-[4-(1H-1,2,4-triazol-1-yl-methyl)phenyl]-3-chinolin-carbonsäure

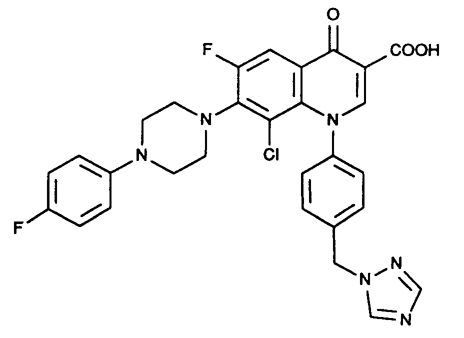

Analog zum Beispiel 134 wird bei der Umsetzung von 8-Chlor-6,7-difluor-1,4-dihydro-4- oxo-1-[4-(1H-1,2,4-triazol-1-yl-methyl)-phenyl]-3-chinolincarbonsäure mit N-(4-Fluorphenyl)-piperazin die Titelverbindung erhalten.
Schmelzpunkt: 248-250°C

### Beispiel 151

### 8-Chlor-6-fluor-1,4-dihydro-4-oxo-7-[4-(2-chlorphenyl)-piperazin-1-yl]-1-[4-(1H-1,2,4-triazol-1-yl-methyl)phenyl]-3-chinolin-carbonsäure

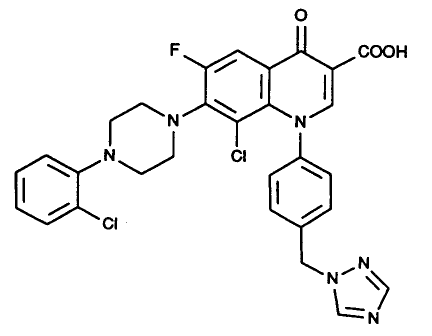

Analog zum Beispiel 134 wird bei der Umsetzung von 8-Chlor-6,7-difluor-1,4-dihydro-4-oxo-1-[4-(1H-1,2,4-triazol-1-yl-methyl)-phenyl]-3-chinolincarbonsäure mit N-(2-Chlorphenyl)-piperazin die Titelverbindung erhalten.
Schmelzpunkt: 248-250°C

### Beispiel 152

### 6-Fluor-1,4-dihydro-4-oxo-7-[4-(4-fluorphenyl)-piperazin-1-yl]-1-[4-(1H-1,2,3-triazol-1-yl-methyl)phenyl]-3-chinolincarbonsäure-propylamid

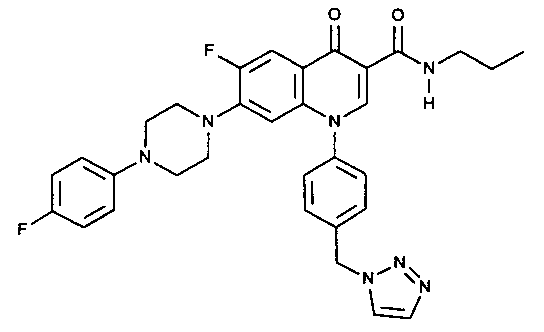

1,32 g (3 mmol) 6-Fluor-1,4-dihydro-4-oxo-7-[4-(4-fluorphenyl)-piperazin-1-yl]-1-[4-(1H-1,2,3-triazol-1-yl-methyl)-phenyl]-3-chinolincarbonsäure werden in 10 ml Aceton bei 0°C mit 0,46 ml (3,3 mmol) Triethylamin versetzt. Anschließend tropft man bei dieser Temperatur innerhalb von 30 Minuten 0,34 ml Chlorameisensäureethylester zu. Nach Zugabe von 0,30 ml (3,6 mmol) Propylamin wird 1,5 Stunden unter Rückfluß gekocht.

Zur Aufarbeitung gibt man auf gesättigte Natriumhydrogencarbonatlösung, extrahiert mit Methylenchlorid und trocknet über Natriumsulfat. Das eingedampfte Rohprodukt wird an Kieselgel(Methylenchlorid/Methanol 96/4) chromatographiert
Ausbeute: 0,8 g (57% der Theorie)
Schmelzpunkt: 202-204°C

In Analogie zur Vorschrift des Beispiels 152 werden die in Tabelle 33 aufgeführten Verbindungen hergestellt:

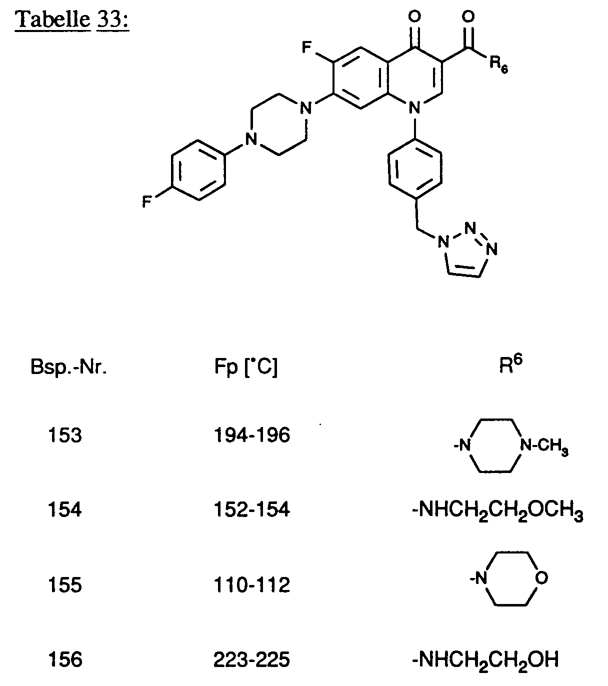

### Beispiel 157

### 6-Fluor-1,4-dihydro-4-oxo-7-[4-(4-fluorphenyl)-piperazin-1-yl]-1-[4-(1H-1,2,3-triazol-1-yl-methyl)phenyl]-3-chinolincarbonsäure-benzylester

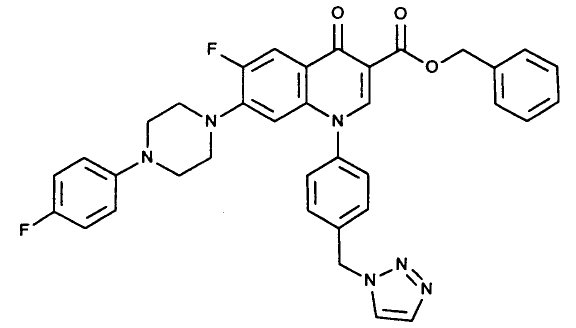

0,44 g (1,0 mmol) 6-Fluor-1,4-dihydro-4-oxo-7-[4-(4-fluorphenyl)-piperazin-1-yl]-1-[4-(1H-1,2,3-triazol-1-yl-methyl)-phenyl]-3-chinolincarbonsäure werden in 10 ml Aceton bei 0°C mit 0,14 ml (1,2 mmol) Triethylamin versetzt. Anschließend tropft man bei dieser Temperatur innerhalb von 30 Minuten 0,14 ml (1,2 mmol) Chlorameisensäureethylester zu. Nach Zugabe von 0,30 ml (3,6 mmol) Benzylalkohol wird 2 Stunden unter Rückfluß gekocht. Zur Aufarbeitung gibt man auf gesättigte Natriumhydrogencarbonatlösung und extrahiert mit Essigester. Die vereinigten organischen Phasen werden mit gesättigter Natriumchloridlösung gewaschen und über Natriumsulfat getrocknet. Das eingedampfte Rohprodukt wird an Kieselgel 60 (Laufmittel: Methylenchlorid/Methanol = 9/1) chromatographiert.
Ausbeute: 0,35 g (55% der Theorie)
Schmelzpunkt: 226-228°C

In Analogie zur Vorschrift des Beispiels 157 werden die in Tabelle 34 aufgeführten Verbindungen hergestellt:

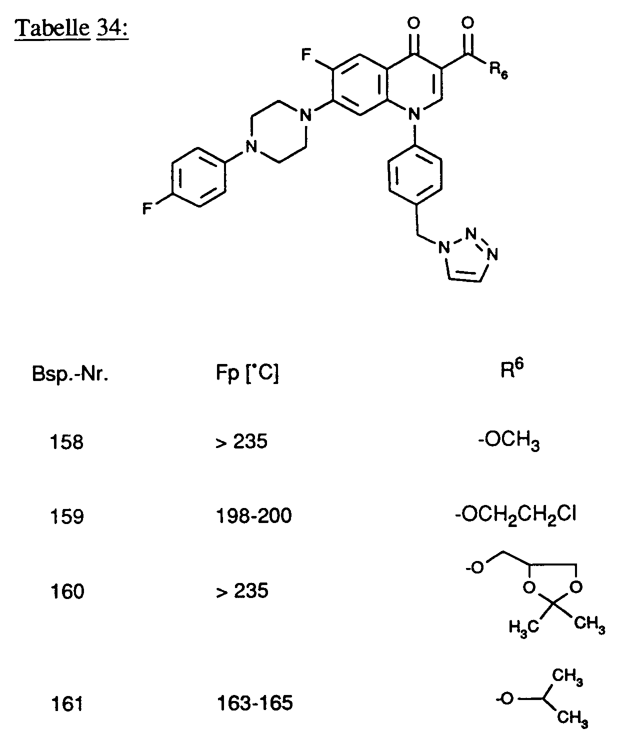

### Beispiel 162

### 1,4-Dihydro-4-oxo-7-(4-phenylpiperazin-1-yl)-1-[4-(1H-1,2,4-triazol-1-yl-methyl)phenyl]-1,8-naphthyridincarbonsäureethylester

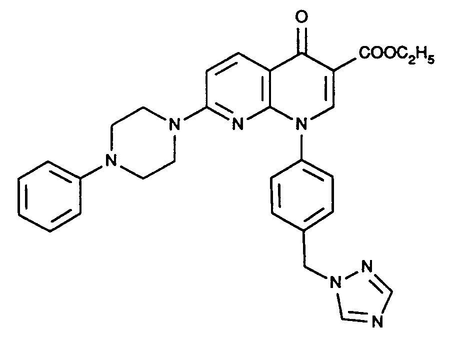

0,5 g (1,2 mmol) 7-Chlor-1,4-dihydro-4-oxo-1-[4-(1H-1,2,4-triazol-1-yl-methyl)-phenyl]-1,8-naphthyridin-3-carbonsäureethylester werden mit 0,58 g (3,6 mmol) N-Methylpiperazin in 12 ml Dimethylsulfoxid über Nacht bei Raumtemperatur gerührt. Alle flüchtigen Komponenten werden im Hochvakuum entfernt. Der Rückstand wird auf Wasser gegeben und mit Dichlormethan extrahiert. Das nach dem Einrotieren erhaltene Rohprodukt wird an Kieselgel (Laufmittel Dichlormethan/Methanol 96/4) gereinigt.
Ausbeute: 475 mg (73% d.Th.)
Schmp.: 170-171°C

### Beispiel 163

### 7-[4-(4-Fluorphenyl)piperazin-1-yl]-1,4-dihydro-4-oxo-1-[4-(1H-1,2,4-triazol-1-yl-methyl)phenyl]-1,8-naphthyridin-3-carbonsäureethylester

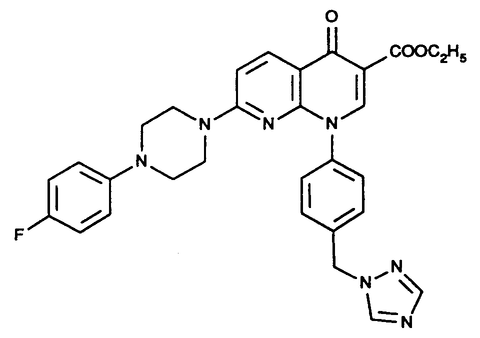

Analog zum Beispiel 162 wird bei der Umsetzung mit 1-(4-Fluorphenyl)piperazin die Titelverbindung erhalten.
Schmp.: 176-178°C

### Beispiel 164

### 1,4-Dihydro-4-oxo-7-(4-phenylpiperazin-1-yl)-1-[4-(1H-1,2,4-triazol-1-yl-methyl)phenyl]-1,8-naphthyridin-3-carbonsäure

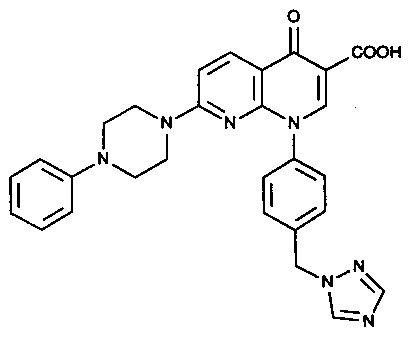

0,45 g (0,84 mmol) des im Beispiel 162 erhaltenen Esters werden mit 1,68 ml 1 N Natronlauge in einem Gemisch aus 8 ml Ethanol und 8 ml Wasser eine Stunde bei 50°C gerührt. Es wird mit verdünnter Salzsäure neutralisiert und die Lösemittel im Vakuum entfernt. Der Rückstand wird an Kieselgel (Laufmittel Dichlormethan / Methanol 96/4) gereinigt.
Ausbeute: 158 mg (37% d.Th.)
Schmp.: 286-287°C

### Beispiel 165

### 7-[4-(4-Fluorphenyl)-piperazin-1-yl]-1,4-dihydro-4-oxo-1-[4-(1H-1,2,4-triazol-1-yl-methyl)phenyl]-1,8-naphthyridin-3-carbonsäure

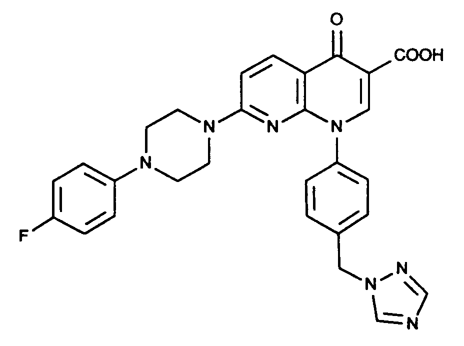

Analog zum Beispiel 164 wird bei der Umsetzung des Esters aus Beispiel 163 die Titelverbindung erhalten.
Schmp.: 284-285°C

## Patentansprüche

1. Chinolon- und Naphthyridoncarbonsäurederivate der allgemeinen Formel (I) 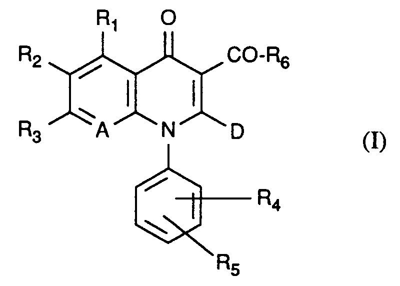 in welcher
R¹ für Wasserstoff, Hydroxy, Mercapto, Halogen, geradkettiges oder verzweigtes Alkyl mit bis zu 3 Kohlenstoffatomen, Perfluoralkyl mit bis zu 3 Kohlenstoffatomen, geradkettiges oder verzweigtes Alkoxy oder Alkylthio mit jeweils bis zu 6 Kohlenstoffatomen, Arylthio mit 6 bis 10 Kohlenstoffatomen oder
für die Gruppe der Formel -NR⁷R⁸ steht,
worin
R⁷ und R⁸ gleich oder verschieden sind und Wasserstoff oder geradkettiges oder verzweigtes Alkyl mit bis zu 6 Kohlenstoffatomen bedeuten,
R² für Wasserstoff, Nitro oder Halogen steht,
R³ für einen Rest der Formel 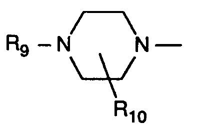 steht,
worin
R⁹ und R¹⁰ gleich oder verschieden sind und Wasserstoff, geradkettiges oder verzweigtes Alkyl mit bis zu 6 Kohlenstoffatomen oder
einen Rest der Formel 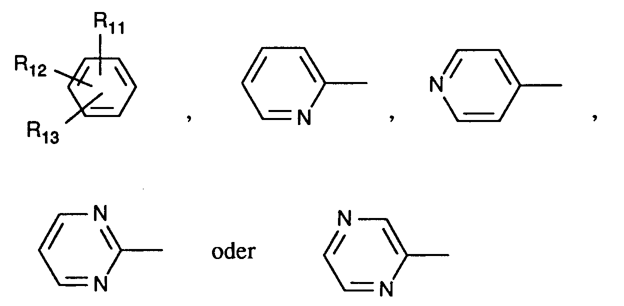 bedeuten,
worin
R¹¹, R¹² und R¹³ gleich oder verschieden sind und Wasserstoff, Halogen, Nitro, Hydroxy, Cyano, Mercapto, Arylthlo mit 6 bis 10 Kohlenstoffatomen, geradkettiges oder verzweigtes Alkyl, Alkoxy, Acyl oder Alkylthio mit jeweils bis zu 6 Kohlenstoffatomen oder Perfluoralkyl mit bis zu 4 Kohlenstoffatomen bedeuten,
A für ein Stickstoffatom oder für die Gruppe der Formel - CR¹⁴ steht,
worin
R¹⁴ Wasserstoff, Halogen, Methyl, Hydroxy, Methoxy, Vinyl oder Alkinyl bedeutet,
R⁴ für einen Rest der Formel 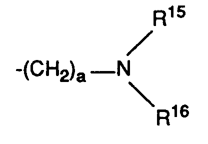 steht,
worin
a eine Zahl 1, 2, 3 oder 4 bedeutet,
R¹⁵ und R¹⁶ gemeinsam mit dem Stickstoffatom einen Imidazolyl-, Pyrrolyl-, 1,2,3-Triazol-1-yl, 1,2,3-Triazol-2-yl, 1,2,4-Triazol-1-yl- bzw. 1,2,4-Triazol-4-yl- oder Tetrazolylring bilden,
R⁵ für Wasserstoff, Hydroxy, Halogen oder für geradkettiges oder verzweigtes Alkyl oder Alkoxy mit jeweils bis zu 6 Kohlenstoffatomen steht,
R⁶ für Hydroxy, Benzyloxy, Morpholino oder geradketttiges oder verzweigtes Alkoxy mit bis zu 6 Kohlenstoffatomen steht, wobei letzteres bis zu 3-fach gleich oder verschieden durch Halogen, Hydroxy oder durch einen heterocyclischen Rest der Formel 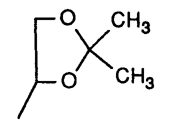 substituiert sein kann, oder
für eine Gruppe der Formel -NR¹⁷R¹⁸ steht,
worin
R¹⁷ und R¹⁸ gleich oder verschieden sind und Wasserstoff oder geradkettiges oder verzweigtes Alkyl mit bis zu 8 Kohlenstoffatomen bedeuten, das gegebenenfalls bis zu 3-fach gleich oder verschieden durch Hydroxy oder durch geradkettiges oder verzweigtes Alkoxy mit bis zu 6 Kohlenstoffatomen substituiert ist,
oder
R⁶ für einen Rest der Formel 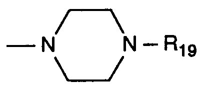 steht,
worin
R¹⁹ Wasserstoff oder geradkettiges oder verzweigtes Alkyl mit bis zu 4 Kohlenstoffatomen bedeutet,
D für Wasserstoff, Amino, Phenyl, Cyano, Hydroxy oder für geradkettiges oder verzweigtes Alkenyl, Alkylthio oder Alkoxycarbonyl mit jeweils bis zu 6 Kohlenstoffatomen steht, oder für geradkettiges oder verzweigtes Alkyl mit bis zu 6 Kohlenstoffatomen steht, das gegebenenfalls durch geradkettiges oder verzweigtes Alkoxycarbonyl mit bis zu 4 Kohlenstoffatomen substituiert ist
und deren Hydrate und Salze, gegebenenfalls in einer isomeren Form.

2. Verbindungen der allgemeinen Formel (I) gemäß Anspruch 1, in welcher
R¹ für Wasserstoff, Hydroxy, Mercapto, Fluor, Chlor, Brom, geradkettiges oder verzweigtes Alkyl mit bis zu 3 Kohlenstoffatomen, Trifluormethyl, geradkettiges oder verzweigtes Alkoxy oder Alkylthio mit jeweils bis zu 4 Kohlenstoffatomen, Phenylthio oder für die Gruppe der Formel -NR⁷R⁸ steht,
worin
R⁷ und R⁸ gleich oder verschieden sind und Wasserstoff oder geradkettiges oder verzweigtes Alkyl mit bis zu 4 Kohlenstoffatomen bedeuten,
R² für Wasserstoff, Nitro, Fluor, Chlor oder Brom steht,
R³ für einen Rest der Formel 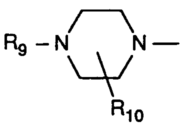 steht,
worin
R⁹ und R¹⁰ gleich oder verschieden sind und Wasserstoff, geradkettiges oder verzweigtes Alkyl mit bis zu 4 Kohlenstoffatomen bedeuten, oder
einen Rest der Formel 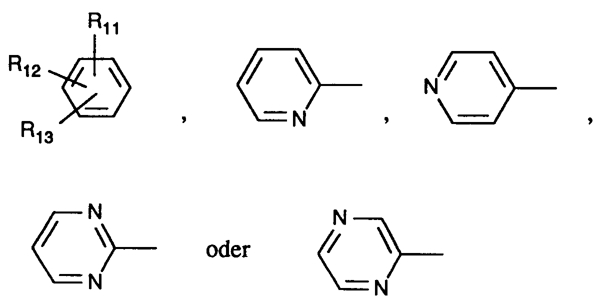 bedeuten,
worin
R¹¹, R¹² und R¹³ gleich oder verschieden sind und Wasserstoff, Fluor, Chlor, Brom, Nitro, Hydroxy, Cyano, Phenylthio, geradkettiges oder verzweigtes Alkyl, Alkoxy, Acyl oder Alkylthio mit jeweils bis zu 4 Kohlenstoffatomen oder Trifluormethyl bedeuten,
A für ein Stickstoffatom oder für die Gruppe der Formel - CR¹⁴ steht,
worin
R¹⁴ Wasserstoff, Fluor, Chlor, Brom, Methyl, Hydroxy, Methoxy, Vinyl oder Alkinyl bedeutet,
R⁴ für einen Rest der Formel 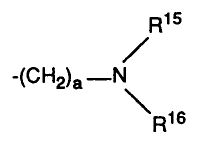 steht,
worin
a eine Zahl 1, 2 oder 3 bedeutet,
R¹⁵ und R¹⁶ gemeinsam mit dem Stickstoffatom einen Imidazolyl-, Pyrrolyl-, 1,2,3-Triazol-1-yl-, 1,2,3-Triazol-2-yl, 1,2,4-Triazol-1-yl- bzw. 1,2,4-Triazol-4-yl- oder Tetrazolylring bilden,
R⁵ für Wasserstoff, Hydroxy, Fluor, Chlor, Brom oder
für geradkettiges oder verzweigtes Alkyl oder Alkoxy mit jeweils bis zu 4 Kohlenstoffatomen steht,
R⁶ für Hydroxy, Benzyloxy, Morpholino oder geradketttiges oder verzweigtes Alkoxy mit bis zu 5 Kohlenstoffatomen steht, wobei letzteres bis zu 2-fach gleich oder verschieden durch Halogen, Hydroxy oder durch einen heterocyclischen Rest der Formel 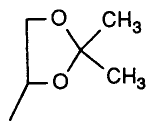 substituiert sein kann, oder
für eine Gruppe der Formel -NR¹⁷R¹⁸ steht,
worin
R¹⁷ und R¹⁸ gleich oder verschieden sind und Wasserstoff oder geradkettiges oder verzweigtes Alkyl mit bis zu 6 Kohlenstoffatomen bedeuten, das gegebenenfalls bis zu 2-fach gleich oder verschieden durch Hydroxy oder durch geradkettiges oder verzweigtes Alkoxy mit bis zu 6 Kohlenstoffatomen substituiert ist,
oder
R⁶ für einen Rest der Formel 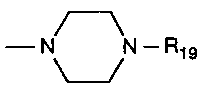 steht,
worin
R¹⁹ Wasserstoff oder geradkettiges oder verzweigtes Alkyl mit bis zu 4 Kohlenstoffatomen bedeutet,
und
D für Methyl, Vinyl, Phenyl oder Wasserstoff steht,
und deren Hydrate und Salze, gegebenenfalls in einer isomeren Form.

3. Verbindungen der allgemeinen Formel (I) gemäß Anspruch 1, in welcher
R¹ für Wasserstoff, Fluor, Chlor, Brom oder Methyl steht,
R² für Wasserstoff, Fluor oder Chlor steht,
R³ für einen Rest der Formel 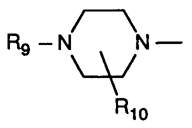 steht,
worin
R⁹ und R¹⁰ gleich oder verschieden sind und Wasserstoff, geradkettiges oder verzweigtes Alkyl mit bis zu 4 Kohlenstoffatomen bedeuten, oder
einen Rest der Formel 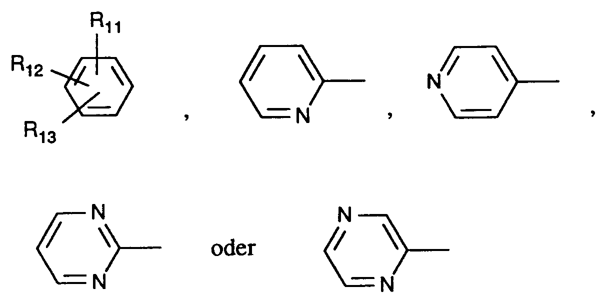 bedeuten,
worin
R¹¹, R¹² und R¹³ gleich oder verschieden sind und Wasserstoff, Fluor, Chlor, Brom, Nitro, Hydroxy, Cyano, Phenylthio, geradkettiges oder verzweigtes Alkyl, Alkoxy, Acyl oder Alkylthio mit jeweils bis zu 3 Kohlenstoffatomen oder Trifluormethyl bedeuten,
A für ein Stickstoffatom oder für die Gruppe der Formel - CR¹⁴ steht,
worin
R¹⁴ Wasserstoff, Fluor oder Chlor bedeutet,
R⁴ für einen Rest der Formel 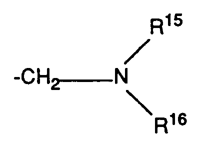 steht,
worin
R¹⁵ und R¹⁶ gemeinsam mit dem Stickstoffatom einen Imidazolyl-, Pyrrolyl-, 1,2,3-Triazol-1-yl-, 1,2,3-Triazol-2-yl, 1,2,4-Triazol-1-yl- bzw. 1,2,4-Triazol-4-yl-Ring bilden,
R⁵ für Wasserstoff steht,
R⁶ für Hydroxy, Benzyloxy, Morpholino oder geradkettiges oder verzweigtes Alkoxy mit bis zu 4 Kohlenstoffatomen steht, wobei letzteres gegebenenfalls durch Fluor, Chlor, Brom, Hydroxy oder durch einen Rest der Formel 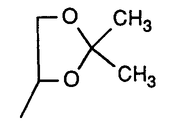 substituiert sein kann, oder
für eine Gruppe der Formel -NR¹⁷R¹⁸ steht,
worin
R¹⁷ und R¹⁸ gleich oder verschieden sind und Wasserstoff oder geradkettiges oder verzweigtes Alkyl mit bis zu 4 Kohlenstoffatomen bedeuten, das gegebenenfalls bis zu 2-fach gleich oder verschieden durch Hydroxy oder durch geradkettiges oder verzweigtes Alkoxy mit bis zu 6 Kohlenstoffatomen substituiert ist,
oder
R⁶ für einen Rest der Formel 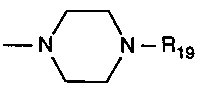 steht,
worin
R¹⁹ Wasserstoff oder geradkettiges oder verzweigtes Alkyl mit bis zu 3 Kohlenstoffatomen bedeutet,
und
D für Wasserstoff steht,
und deren Hydrate und Salze, gegebenenfalls in einer isomeren Form.

4. Verfahren zur Herstellung der Verbindungen der allgemeinen Formel (I) gemäß Anspruch 1, dadurch gekennzeichnet, daß man
Verbindungen der allgemeinen Formel (II) 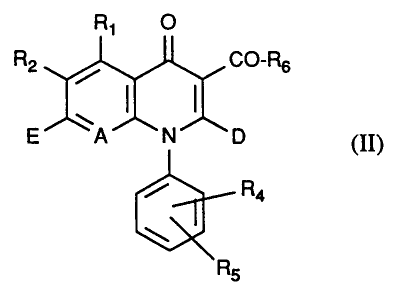 in welcher
R¹, R², R⁴, R⁵, R⁶, A und D die oben angegebene Bedeutung haben
und
E für Halogen, vorzugsweise für Chlor und Fluor steht,
mit Verbindungen der allgemeinen Formel (III)
R³-H (III)
in welcher
R³ die oben angegebene Bedeutung hat,
in inerten Lösemitteln, gegebenenfalls in Anwesenheit von Säurefängern umsetzt,
und im Fall der Säuren die Ester verseift,
und im Fall der Ester die Säuren nach üblichen Methoden mit den entsprechenden Alkoholen umsetzt,
und im Fall der Amide die Säuren, gegebenenfalls nach vorgeschalteter Aktivierung und in Anwesenheit einer Base und/oder Hilfsstoffes, amidiert.

5. Arzneimittel enthaltend eine oder mehrere Verbindungen aus den Ansprüchen 1 bis 3.

6. Verwendung der Verbindungen aus den Ansprüchen 1 bis 3 zur Herstellung von Arzneimitteln.

7. Verbindungen der allgemeinen Formel (II) 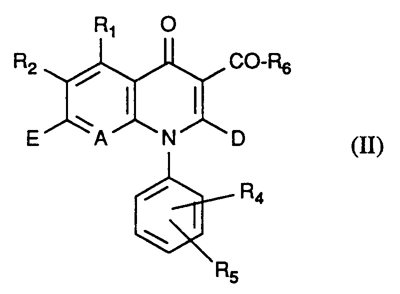 in welcher
R¹, R², R⁴, R⁵, R⁶, A und D die in den Ansprüchen 1 bis 3 angegebene Bedeutung haben
und
E für Halogen, vorzugsweise für Chlor und Fluor steht.

8. Verfahren zur Herstellung der Verbindungen der allgemeinen Formel (II), dadurch gekennzeichnet, daß man zunächst Verbindungen der allgemeinen Formel (IV) 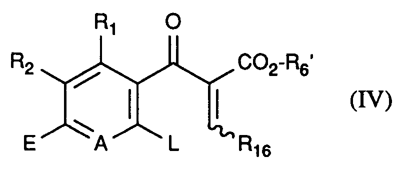 in welcher
R¹, R², A und E die in Anspruch 7 angegebene Bedeutung haben,
L für Halogen, vorzugsweise für Chlor oder Fluor steht,
R^{6'} für C₁-C₄-Alkyl steht
und
R¹⁶ für C₁-C₄-Alkoxy oder C₁-C₄-Dialkylamino steht,
durch Umsetzung mit Aminen der allgemeinen Formel (V) 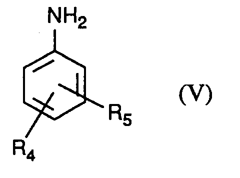 in welcher
R⁴ und R⁵ die oben angegebene Bedeutung haben,
in einem der oben aufgeführten Lösemittel, vorzugsweise Ethanol,
in die Verbindungen der allgemeinen Formel (VI) 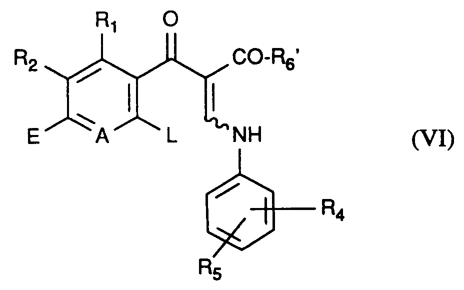 in welcher
R¹, R², R⁴, R⁵, A, E, L und R^{6'} die oben angegebene Bedeutung haben,
überführt, und in einem letzten Schritt in einem der oben aufgeführten Lösemittel und einer dort genannten Base, vorzugsweise DMF und K₂CO₃/KF cyclisiert,
und im Fall D ≠ H, diese Substituenten nach üblichen Methoden variiert.

9. Verbindungen der allgemeinen Formel (VI) 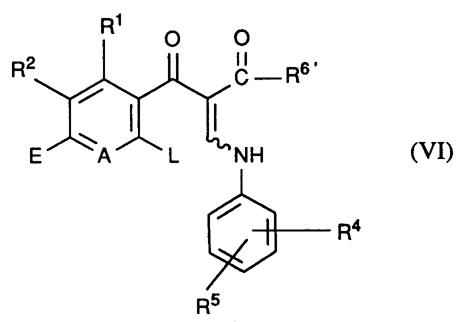 in welcher
R¹, R², R⁴, R⁵, A, E, L und R^{6'} die oben angegebene Bedeutung haben.

## Claims

1. Derivatives of quinolonecarboxylic acid and naphthyridonecarboxylic acid of the general formula (I) 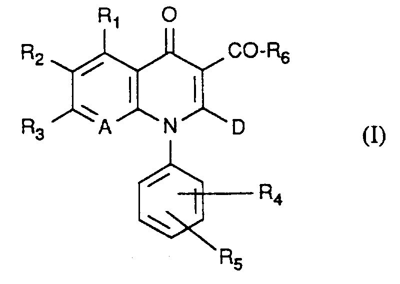 in which
R¹ represents hydrogen, hydroxyl, mercapto, halogen, straight-chain or branched alkyl having up to 3 carbon atoms, perfluoroalkyl having up to 3 carbon atoms, straight-chain or branched alkoxy or alkylthio having in each case up to 6 carbon atoms, arylthio having 6 to 10 carbon atoms, or
represents the group of the formula -NR⁷R⁸,
in which
R⁷ and R⁸ are identical or different and denote hydrogen or straight-chain or branched alkyl having up to 6 carbon atoms,
R² represents hydrogen, nitro or halogen,
R³ represents a residue of the formula 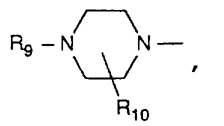 in which
R⁹ and R¹⁰ are identical or different and denote hydrogen, straight-chain or branched alkyl having up to 6 carbon atoms, or a residue of the formula 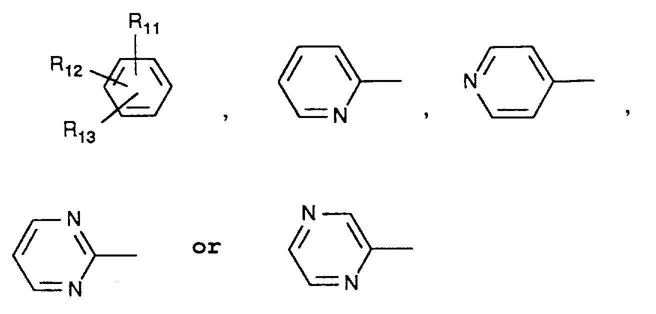 in which
R¹¹, R¹² and R¹³ are identical or different and denote hydrogen, halogen, nitro, hydroxyl, cyano, mercapto, arylthio having 6 to 10 carbon atoms, straight-chain or branched alkyl, alkoxy, acyl or alkylthio having in each case up to 6 carbon atoms, or perfluoroalkyl having up to 4 carbon atoms,
A represents a nitrogen atom or represents the group of the formula -CR¹⁴,
in which
R¹⁴ denotes hydrogen, halogen, methyl, hydroxyl, methoxy, vinyl or alkinyl,
R⁴ represents a residue of the formula 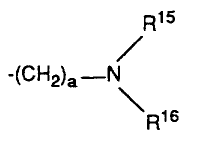 in which
a denotes a number 1, 2, 3 or 4,
R¹⁵ and R¹⁶, together with the nitrogen atom, form an imidazolyl, pyrrolyl, 1,2,3-triazol-1-yl, 1,2,3-triazol-2-yl, 1,2,4-triazol-1-yl, 1,2,4-triazol-4-yl or tetrazolyl ring,
R⁵ represents hydrogen, hydroxyl, halogen, or represents straight-chain or branched alkyl or alkoxy having in each case up to 6 carbon atoms,
R⁶ represents hydroxyl, benzyloxy, morpholino, or straight-chain or branched alkoxy having up to 6 carbon atoms, where the latter can be substituted identically or differently up to 3 times by halogen, hydroxyl, or by a heterocyclic residue of the formula 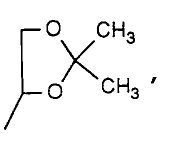 or
represents a group of the formula -NR¹⁷R¹⁸,
in which
R¹⁷ and R¹⁸ are identical or different and denote hydrogen or straight-chain or branched alkyl having up to 8 carbon atoms, which is optionally substituted identically or differently up to 3 times by hydroxyl or by straight-chain or branched alkoxy having up to 6 carbon atoms,
or
R⁶ represents a residue of the formula 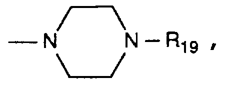 in which
R¹⁹ denotes hydrogen or straight-chain or branched alkyl having up to 4 carbon atoms,
D represents hydrogen, amino, phenyl, cyano, hydroxyl, or represents straight-chain or branched alkenyl, alkylthio or alkoxycarbonyl having in each case up to 6 carbon atoms, or represents straight-chain or branched alkyl having up to 6 carbon atoms, which is optionally substituted by straight-chain or branched alkoxycarbonyl having up to 4 carbon atoms,
and hydrates and salts thereof, optionally in an isomeric form.

2. Compounds of the general formula (I) according to Claim 1,
in which
R¹ represents hydrogen, hydroxyl, mercapto, fluorine, chlorine, bromine, straight-chain or branched alkyl having up to 3 carbon atoms, trifluoromethyl, straight-chain or branched alkoxy or alkylthio having in each case up to 4 carbon atoms, phenylthio, or represents the group of the formula -NR⁷R⁸,
in which
R⁷ and R⁸ are identical or different and denote hydrogen or straight-chain or branched alkyl having up to 4 carbon atoms,
R² represents hydrogen, nitro, fluorine, chlorine or bromine,
R³ represents a residue of the formula 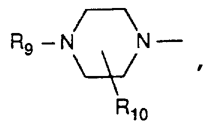 in which
R⁹ and R¹⁰ are identical or different and denote hydrogen, straight-chain or branched alkyl having up to 4 carbon atoms, or
denote a residue of the formula 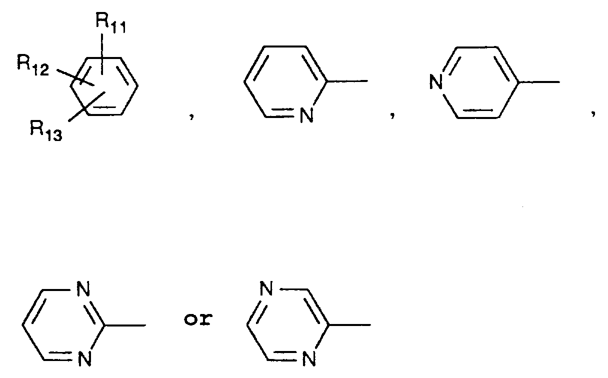 in which
R¹¹ and R¹² and R¹³ are identical or different and denote hydrogen, fluorine, chlorine, bromine, nitro, hydroxyl, cyano, phenylthio, straight-chain or branched alkyl, alkoxy, acyl or alkylthio having in each case up to 4 carbon atoms, or trifluoromethyl,
A represents a nitrogen atom or represents the group of the formula -CR¹⁴,
in which
R¹⁴ denotes hydrogen, fluorine, chlorine, bromine, methyl, hydroxyl, methoxy, vinyl or alkinyl,
R⁴ represents a residue of the formula 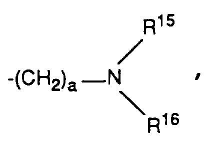 in which
a denotes a number 1, 2 or 3,
R¹⁵ and R¹⁶, together with the nitrogen atom, form an imidazolyl, pyrrolyl, 1,2,3-triazol-1-yl, 1,2,3-triazol-2-yl, 1,2,4-triazol-1-yl, 1,2,4-triazol-4-yl or tetrazolyl ring,
R⁵ represents hydrogen, hydroxyl, fluorine, chlorine, bromine, or represents straight-chain or branched alkyl or alkoxy having in each case up to 4 carbon atoms,
R⁶ represents hydroxyl, benzyloxy, morpholino, or straight-chain or branched alkoxy having up to 5 carbon atoms, where the latter can be substituted identically or differently up to 2 times by halogen, hydroxyl, or by a heterocyclic residue of the formula 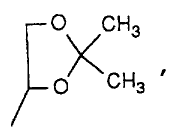 or
represents a group of the formula -NR¹⁷R¹⁸,
in which
R¹⁷ and R¹⁸ are identical or different and denote hydrogen or straight-chain or branched alkyl having up to 6 carbon atoms, which is optionally substituted identically or differently up to 2 times by hydroxyl or by straight-chain or branched alkoxy having up to 6 carbon atoms,
or
R⁶ represents a residue of the formula 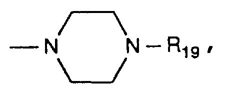 in which
R¹⁹ denotes hydrogen or straight-chain or branched alkyl having up to 4 carbon atoms,
and
D represents methyl, vinyl, phenyl or hydrogen,
and hydrates and salts thereof, optionally in an isomeric form.

3. Compounds of the general formula (I) according to Claim 1,
in which
R¹ represents hydrogen, fluorine, chlorine, bromine or methyl,
R² represents hydrogen, fluorine or chlorine,
R³ represents a residue of the formula 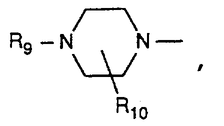 in which
R⁹ and R¹⁰ are identical or different and denote hydrogen, straight-chain or branched alkyl having up to 4 carbon atoms, or denote a residue of the formula 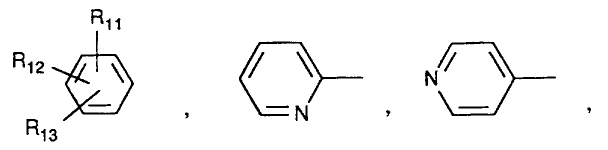 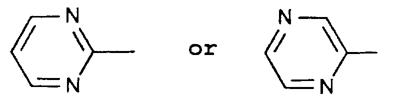 in which
R¹¹, R¹² and R¹³ are identical or different and denote hydrogen, fluorine, chlorine, bromine, nitro, hydroxyl, cyano, phenylthio, straight-chain or branched alkyl, alkoxy, acyl or alkylthio having in each case up to 3 carbon atoms, or trifluoromethyl,
A represents a nitrogen atom or represents the group of the formula -CR¹⁴,
in which
R¹⁴ denotes hydrogen, fluorine or chlorine,
R⁴ represents a residue of the formula 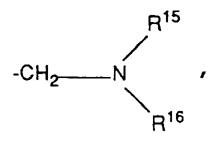 in which
R¹⁵ and R¹⁶, together with the nitrogen atom, form an imidazolyl, pyrrolyl, 1,2,3-triazol-1-yl, 1,2,3-triazol-2-yl, 1,2,4-triazol-1-yl or 1,2,4-triazol-4-yl ring,
R⁵ represents hydrogen,
R⁶ represents hydroxyl, benzyloxy, morpholino or straight-chain or branched alkoxy having up to 4 carbon atoms, where the latter can optionally be substituted by fluorine, chlorine, bromine, hydroxyl, or by a residue of the formula 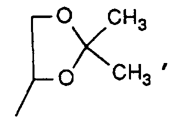 or represents a group of the formula -NR¹⁷R¹⁸,
in which
R¹⁷ and R¹⁸ are identical or different and denote hydrogen, or straight-chain or branched alkyl having up to 4 carbon atoms, which is optionally substituted identically or differently up to 2 times by hydroxyl or by straight-chain or branched alkoxy having up to 6 carbon atoms,
or
R⁶ represents a residue of the formula 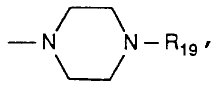 in which
R¹⁹ denotes hydrogen or straight-chain or branched alkyl having up to 3 carbon atoms,
and
D represents hydrogen,
and hydrates and salts thereof, optionally in an isomeric form.

4. Process for preparing the compounds of the general formula (I) according to Claim 1, characterized in that
compounds of the general formula (II) 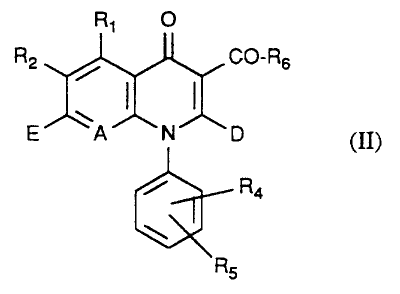 in which
R¹, R², R⁴, R⁵, R⁶, A and D have the abovementioned meaning
and
E represents halogen, preferably chlorine and fluorine, are reacted with compounds of the general formula (III)
R³-H (III)
in which
R³ has the abovementioned meaning,
in inert solvents, optionally in the presence of acid-capturing agents,
and, in the case of the acids, the esters are hydrolyzed,
and, in the case of the esters, the acids are reacted with the corresponding alcohols according to customary methods,
and, in the case of the amides, the acids are amidated, optionally after prior activation and in the presence of a base and/or auxiliary substance.

5. Medicament containing one or more compounds from Claims 1 to 3.

6. Use of the compounds from Claims 1 to 3 for preparing medicaments.

7. Compounds of the general formula (II) 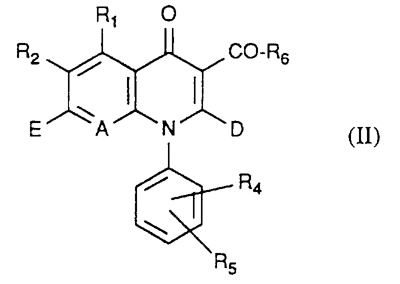 in which
R¹, R², R⁴, R⁵, R⁶, A and D have the meaning given in Claims 1 to 3,
and
E represents halogen, preferably chlorine and fluorine.

8. Process for preparing the compounds of the general formula (II), characterized in that compounds of the general formula (IV) 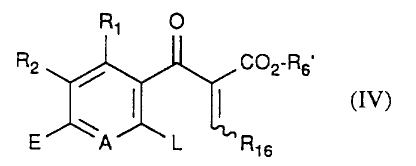 in which
R¹, R², A and E have the meaning given in Claim 7,
L represents halogen, preferably chlorine or fluorine,
R^{6'} represents C₁-C₄-alkyl, and
R¹⁶ represents C₁-C₄-alkoxy or C₁-C₄-dialkylamino,
are initially converted, by reaction with amines of the general formula (V) 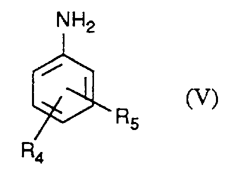 in which
R⁴ and R⁵ have the abovementioned meaning,
in one of the above listed solvents, preferably ethanol,
into the compounds of the general formula (VI) 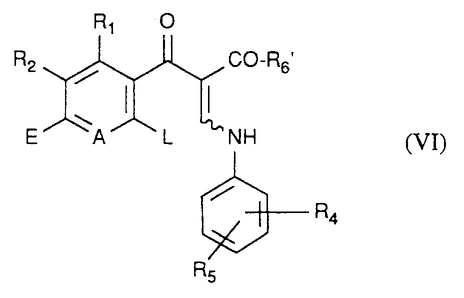 in which
R¹, R², R⁴, R⁵, A, E, L and R^{6'} have the above-mentioned meaning,
and, in a final step, are cyclized in one of the above listed solvents and one of the above listed bases, preferably DMF and K₂CO₃/KF,
and, in the case where D ≠ H, these substituents are varied in accordance with customary methods.

9. Compounds of the general formula (VI) 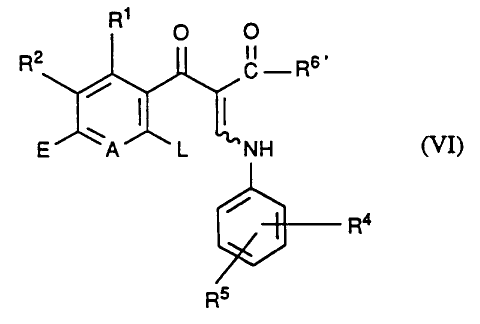 in which
R¹, R², R⁴, R⁵, A, E, L and R^{6'} have the above-mentioned meaning.

## Revendications

1. Dérivés d'acides quinolone- et naphtyridonecarboxyliques répondant à la formule générale (I) 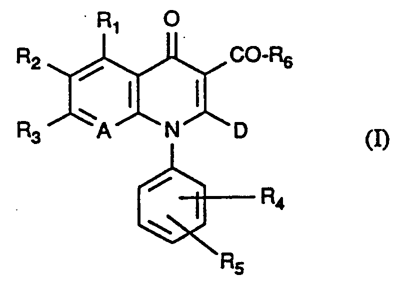 dans laquelle
R¹ représente un atome d'hydrogène, un groupe hydroxyle, un groupe mercapto, un atome d'halogène, un groupe alkyle à chaîne droite ou ramifiée contenant jusqu'à 3 atomes de carbone, un groupe perfluoroalkyle contenant jusqu'à 3 atomes de carbone, un groupe alcoxy ou un groupe alkylthio à chaîne droite ou ramifiée contenant respectivement jusqu'à 6 atomes de carbone, un groupe arylthio contenant de 6 à 10 atomes de carbone, ou encore le groupe répondant à la formule -NR⁷R⁸,
dans laquelle
R⁷ et R⁸ sont identiques ou différents et représentent un atome d'hydrogène ou un groupe alkyle à chaîne droite ou ramifiée contenant jusqu'à 6 atomes de carbone,
R² représente un atome d'hydrogène, un groupe nitro ou un atome d'halogène,
R³ représente un radical de formule 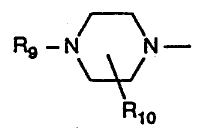 dans laquelle
R⁹ et R¹⁰ sont identiques ou différents et représentent un atome d'hydrogène, un groupe alkyle à chaîne droite ou ramifiée contenant jusqu'à 6 atomes de carbone, ou encore un radical répondant aux formules 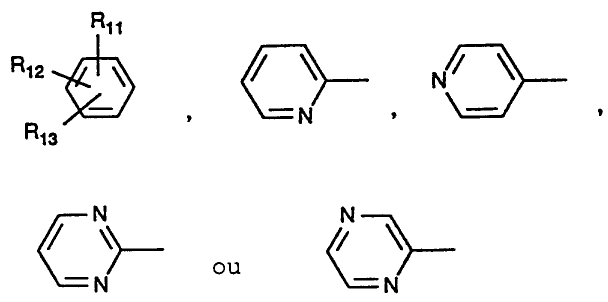 dans lesquelles
R¹¹, R¹² et R¹³ sont identiques ou différents et représentent un atome d'hydrogène, un atome d'halogène, un groupe nitro, un groupe hydroxyle, un groupe cyano, un groupe mercapto, un groupe arylthio contenant de 6 à 10 atomes de carbone; un groupe alkyle, un groupe alcoxy, un groupe acyle ou un groupe alkylthio à chaîne droite ou ramifiée contenant respectivement jusqu'à 6 atomes de carbone, ou encore un groupe perfluoroalkyle contenant jusqu'à 4 atomes de carbone,
A représente un atome d'azote ou le groupe répondant à la formule -CR¹⁴,
dans laquelle
R¹⁴ représente un atome d'hydrogène, un atome d'halogène, un groupe méthyle, un groupe hydroxyle, un groupe méthoxy, un groupe vinyle ou un groupe alcynyle,
R⁴ représente un radical de formule 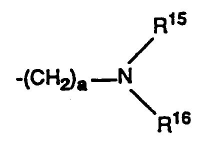 dans laquelle
a représente le nombre 1, 2, 3 ou 4,
R¹⁵ et R¹⁶ forment ensemble avec l'atome d'azote un noyau imidazolyle, un noyau pyrrolyle, un noyau 1,2,3-triazol-1-yle, un noyau 1,2,3-triazol-2-yle, un noyau 1,2,4-triazol-1-yle, respectivement un noyau 1,2,4-triazol-4-yle, ou encore un noyau tétrazolyle,
R⁵ représente un atome d'hydrogène, un groupe hydroxyle, un atome d'halogène ou encore un groupe alkyle ou un groupe alcoxy à chaîne droite ou ramifiée contenant respectivement jusqu'à 6 atomes de carbone,
R⁶ représente un groupe hydroxyle, un groupe benzyloxy, un groupe morpholino ou un groupe alcoxy à chaîne droite ou ramifiée contenant jusqu'à 6 atomes de carbone, ce dernier groupe pouvant porter jusqu'à 3 substituants identiques ou différents halogéno, hydroxyle ou pouvant porter, à titre de substituant, un radical hétérocyclique répondant à la formule 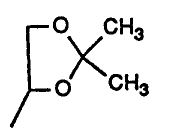 ou représente un groupe répondant à la formule -NR¹⁷R¹⁸,
dans laquelle
R¹⁷ et R¹⁸ sont identiques ou différents et représentent un atome d'hydrogène ou un groupe alkyle à chaîne droite ou ramifiée contenant jusqu'à 8 atomes de carbone, qui porte le cas échéant jusqu'à 3 substituants identiques ou différents hydroxyle ou alcoxy à chaîne droite ou ramifiée contenant jusqu'à 6 atomes de carbone,
ou
R⁶ représente un radical de formule 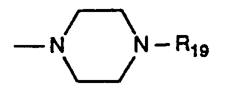 dans laquelle
R¹⁹ représente un atome d'hydrogène ou un groupe alkyle à chaîne droite ou ramifiée contenant jusqu'à 4 atomes de carbone,
D représente un atome d'hydrogène, un groupe amino, un groupe phényle, un groupe cyano, un groupe hydroxyle; ou encore un groupe alcényle, un groupe alkylthio ou un groupe alcoxycarbonyle à chaîne droite ou ramifiée contenant respectivement jusqu'à 6 atomes de carbone; ou représente un groupe alkyle à chaîne droite ou ramifiée contenant jusqu'à 6 atomes de carbone, qui porte le cas échéant un substituant alcoxycarbonyle à chaîne droite ou ramifiée contenant jusqu'à 4 atomes de carbone,
ainsi que leurs hydrates et leurs sels, éventuellement sous une forme isomère.

2. Composés répondant à la formule générale (I) selon la revendication 1,
dans laquelle
R¹ représente un atome d'hydrogène, un groupe hydroxyle, un groupe mercapto, un atome de fluor, un atome de chlore, un atome de brome, un groupe alkyle à chaîne droite ou ramifiée contenant jusqu'à 3 atomes de carbone, un groupe trifluorométhyle; un groupe alcoxy ou un groupe alkylthio à chaîne droite ou ramifiée contenant respectivement jusqu'à 4 atomes de carbone; un groupe phénylthio ou encore le groupe répondant à la formule -NR⁷R⁸,
dans laquelle
R⁷ et R⁸ sont identiques ou différents et représentent un atome d'hydrogène ou un groupe alkyle à chaîne droite ou ramifiée contenant jusqu'à 4 atomes de carbone,
R² représente un atome d'hydrogène, un groupe nitro, un atome de fluor, un atome de chlore ou un atome de brome,
R³ représente un radical de formule 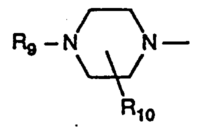 dans laquelle
R⁹ et R¹⁰ sont identiques ou différents et représentent un atome d'hydrogène, un groupe alkyle à chaîne droite ou ramifiée contenant jusqu'à 4 atomes de carbone, ou encore un radical répondant aux formules 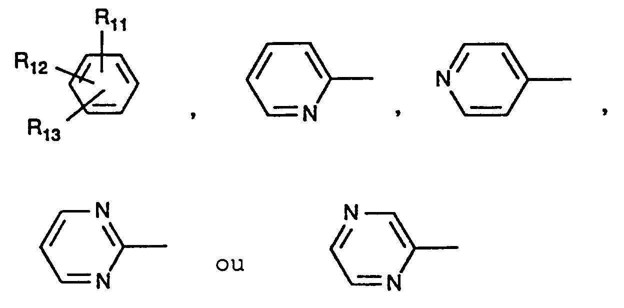 dans lesquelles
R¹¹, R¹² et R¹³ sont identiques ou différents et représentent un atome d'hydrogène, un atome de fluor, un atome de chlore, un atome de brome, un groupe nitro, un groupe hydroxyle, un groupe cyano, un groupe phénylthio; un groupe alkyle, un groupe alcoxy, un groupe acyle ou un groupe alkylthio à chaîne droite ou ramifiée contenant respectivement jusqu'à 4 atomes de carbone; ou encore un groupe trifluorométhyle,
A représente un atome d'azote ou encore le groupe répondant à la formule -CR¹⁴,
dans laquelle
R¹⁴ représente un atome d'hydrogène, un atome de fluor, un atome de chlore, un atome de brome, un groupe méthyle, un groupe hydroxyle, un groupe méthoxy, un groupe vinyle ou un groupe alcynyle,
R⁴ représente un radical de formule 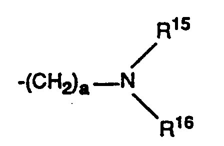 dans laquelle
a représente le nombre 1, 2 ou 3,
R¹⁵ et R¹⁶ forment ensemble avec l'atome d'azote un noyau imidazolyle, un noyau pyrrolyle, un noyau 1,2,3-triazol-1-yle, un noyau 1,2,3-triazol-2-yle, un noyau 1,2,4-triazol-1-yle, respectivement un noyau 1,2,4-triazol-4-yle, ou encore un noyau tétrazolyle,
R⁵ représente un atome d'hydrogène, un groupe hydroxyle, un atome de fluor, un atome de chlore, un atome de brome; ou encore un groupe alkyle ou un groupe alcoxy à chaîne droite ou ramifiée contenant respectivement jusqu'à 4 atomes de carbone,
R⁶ représente un groupe hydroxyle, un groupe benzyloxy, un groupe morpholino ou encore un groupe alcoxy à chaîne droite ou ramifiée contenant jusqu'à 5 atomes de carbone, ce dernier groupe pouvant porter jusqu'à 2 substituants identiques ou différents halogéno, hydroxyle, ou pouvant porter, à titre de substituant, un radical hétérocyclique répondant à la formule 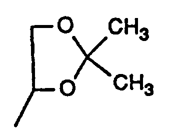 ou représente un groupe répondant à la formule -NR¹⁷R¹⁸,
dans laquelle
R¹⁷ et R¹⁸ sont identiques ou différents et représentent un atome d'hydrogène ou un groupe alkyle à chaîne droite ou ramifiée contenant jusqu'à 6 atomes de carbone, qui porte le cas échéant jusqu'à 2 substituants identiques ou différents hydroxyle ou alcoxy à chaîne droite ou ramifiée contenant jusqu'à 6 atomes de carbone,
ou
R⁶ représente un radical de formule 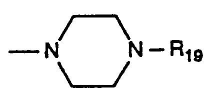 dans laquelle
R¹⁹ représente un atome d'hydrogène ou un groupe alkyle à chaîne droite ou ramifiée contenant jusqu'à 4 atomes de carbone,
et
D représente un groupe méthyle, un groupe vinyle, un groupe phényle ou un atome d'hydrogène,
ainsi que leurs hydrates et leurs sels, éventuellement sous une forme isomère.

3. Composés répondant à la formule générale (I) selon la revendication 1,
dans laquelle
R¹ représente un atome d'hydrogène, un atome de fluor, un atome de chlore, un atome de brome ou un groupe méthyle,
R² représente un atome d'hydrogène, un atome de fluor ou un atome de chlore,
R³ représente un radical répondant à la formule 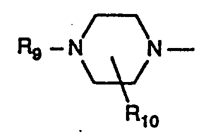 dans laquelle
R⁹ et R¹⁰ sont identiques ou différents et représentent un atome d'hydrogène, un groupe alkyle à chaîne droite ou ramifiée contenant jusqu'à 4 atomes de carbone, ou encore un radical répondant aux formules 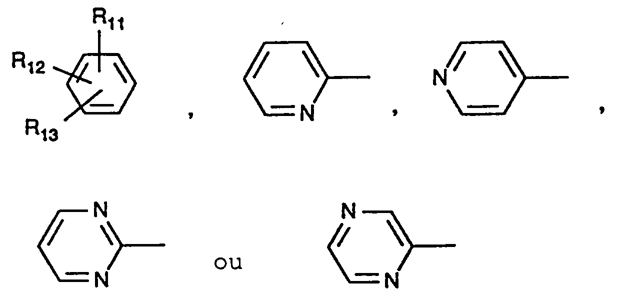 dans lesquelles
R¹¹, R¹² et R¹³ sont identiques ou différents et représentent un atome d'hydrogène, un atome de fluor, un atome de chlore, un atome de brome, un groupe nitro, un groupe hydroxyle, un groupe cyano, un groupe phénylthio; un groupe alkyle, un groupe alcoxy, un groupe acyle ou un groupe alkylthio à chaîne droite ou ramifiée contenant respectivement jusqu'à 3 atomes de carbone; ou encore un groupe trifluorométhyle,
A représente un atome d'azote ou encore le groupe répondant à la formule -CR¹⁴,
dans laquelle
R¹⁴ représente un atome d'hydrogène, un atome de fluor ou un atome de chlore,
R⁴ représente un radical de formule 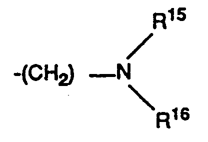 dans laquelle
R¹⁵ et R¹⁶ forment ensemble avec l'atome d'azote un noyau imidazolyle, un noyau pyrrolyle, un noyau 1,2,3-triazol-1-yle, un noyau 1,2,3-triazol-2-yle, un noyau 1,2,4-triazol-1-yle, respectivement un noyau 1,2,4-triazol-4-yle,
R⁵ représente un atome d'hydrogène,
R⁶ représente un groupe hydroxyle, un groupe benzyloxy, un groupe morpholino ou encore un groupe alcoxy à chaîne droite ou ramifiée contenant jusqu'à 4 atomes de carbone, ce dernier groupe pouvant porter le cas échéant un atome de fluor, un atome de chlore, un atome de brome, un groupe hydroxyle ou pouvant porter, à titre de substituant, un radical répondant à la formule 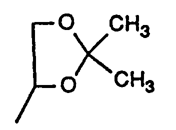 ou représente un groupe répondant à la formule -NR¹⁷R¹⁸,
dans laquelle
R¹⁷ et R¹⁸ sont identiques ou différents et représentent un atome d'hydrogène ou un groupe alkyle à chaîne droite ou ramifiée contenant jusqu'à 4 atomes de carbone, qui porte le cas échéant jusqu'à 2 substituants identiques ou différents hydroxyle ou alcoxy à chaîne droite ou ramifiée contenant jusqu'à 6 atomes de carbone,
ou
R⁶ représente un radical de formule 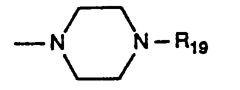 dans laquelle
R¹⁹ représente un atome d'hydrogène ou un groupe alkyle à chaîne droite ou ramifiée contenant jusqu'à 3 atomes de carbone,
et
D représente un atome d'hydrogène,
ainsi que leurs hydrates et leurs sels, éventuellement sous une forme isomère.

4. Procédé pour la préparation des composés répondant à la formule générale (I) selon la revendication 1, caractérisé en ce qu'on fait réagir des composés répondant à la formule générale (II) 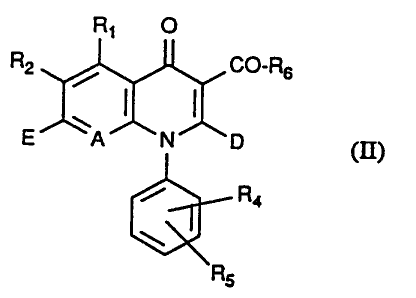 dans laquelle
R¹, R², R⁴, R⁵, R⁶, A et D ont la signification indiquée ci-dessus,
et
E représente un atome d'halogène, de préférence un atome de chlore et un atome de fluor,
avec des composés répondant à la formule générale (III)
R³-H (III)
dans laquelle
R³ a la signification indiquée ci-dessus,
dans des solvants inertes, éventuellement en présence d'agents neutralisant les acides,
et, dans le cas des acides, on saponifie les esters,
et, dans le cas des esters, on fait réagir les acides conformément à des procédés habituels avec les alcools correspondants,
et, dans le cas des amides, on soumet les acides à une amidation éventuellement après activation montée en amont et en présence d'une base et/ou d'un adjuvant.

5. Médicament contenant un ou plusieurs composés selon les revendications 1 à 3.

6. Utilisation des composés selon les revendications 1 à 3, pour la préparation de médicaments.

7. Composés répondant à la formule générale (II) 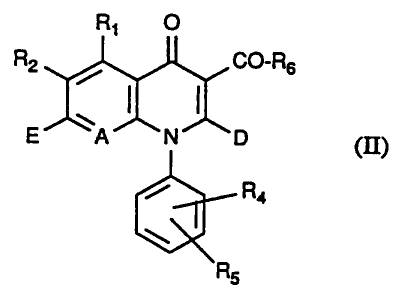 dans laquelle
R¹, R², R⁴, R⁵, R⁶, A et D ont la signification indiquée dans les revendications 1 à 3
et
E représente un atome d'halogène, de préférence un atome de chlore et un atome de fluor.

8. Procédé pour la préparation des composés répondant à la formule générale (II), caractérisé en ce qu'on transforme d'abord des composés répondant à la formule générale (IV) 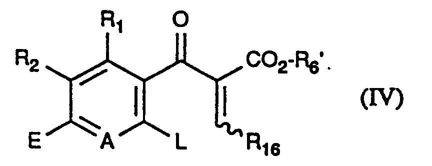 dans laquelle
R¹, R², A et E ont la signification indiquée à la revendication 7,
L représente un atome d'halogène, de préférence un atome de chlore ou un atome de fluor,
R^{6'} représente un groupe alkyle en C₁-C₄,
et
R¹⁶ représente un groupe alcoxy en C₁-C₄ ou un groupe di(alkyl en C₁-C₄)amino,
par mise en réaction avec des amines répondant à la formule générale (V) 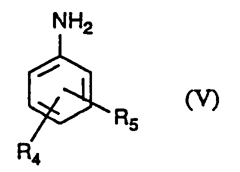 dans laquelle
R⁴ et R⁵ ont la signification indiquée ci-dessus, dans un des solvants mentionnés ci-dessus, de préférence dans de l'éthanol, en composés répondant à la formule générale (VI) 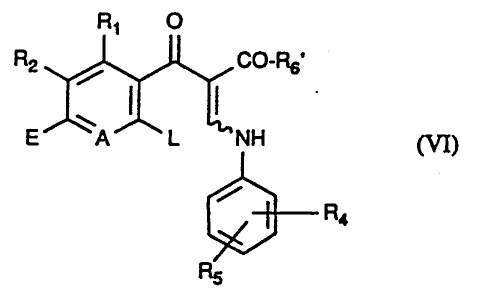
dans laquelle
R¹, R², R⁴, R⁵, A, E, L et R^{6'} ont la signification indiquée ci-dessus,
et, dans une dernière étape, on les soumet à une cyclisation dans un des solvants mentionnés ci-dessus et dans une base qui y est mentionnée, de préférence le DMF et K₂CO₃/KF,
et, au cas où D ≠ H, on fait varier ces substituants conformément à des procédés habituels.

9. Composés répondant à la formule générale (VI) 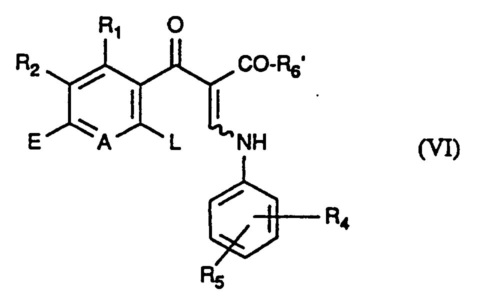 dans laquelle
R¹, R², R⁴, R⁵, A, E, L et R^{6'} ont la signification indiquée ci-dessus.
